# EUROPEAN PATENT APPLICATION

(11) **EP 4 176 895 A1**
(43) Date of publication of application: **10.05.2023**
(21) Application number: 21206918.1
(22) Date of filing: 08.11.2021
(51) Int. Cl.: A61K 39/00, C07K 14/54, C07K 14/725, C07K 16/28, C07K 16/32, A61P 31/00, A61P 35/00, A61P 37/02

(54) **TARGETING MODULES AGAINST IL13RA2 OR HER2 FOR USE IN COMBINATION WITH A CHIMERIC ANTIGEN RECEPTOR**

(71) Applicant: AvenCell Europe GmbH, 01307 Dresden (DE)
(72) Inventor: JIMÉNEZ, Gabriel Jurado, 01307 Dresden (DE); SPEHR, Johannes, 01307 Dresden (DE); CARTELLIERI, Marc, 01307 Dresden (DE); EHNINGER, Armin, 01307 Dresden (DE)
(74) Representative: Kailuweit & Uhlemann Patentanwälte Partnerschaft mbB

(57) **Abstract**

The present invention relates to a targeting module comprising at least one tumor-binding domain, in particular at least one IL13Rα2-binding domain and/or at least one HER2-binding domain, and a tag-binding domain or a tag for use in a method for stimulating a chimeric antigen receptor-mediated immune response in a mammal, a nucleic acid, a vector or a cell comprising a nucleotide sequence encoding the targeting module, a pharmaceutical composition and a kit comprising the targeting module and a vector or a cell comprising a nucleotide sequence encoding a switchable chimeric antigen receptor.

## Description

The present invention relates to a targeting module comprising at least one tumor-binding domain, in particular at least one IL13Rα2-binding domain and/or at least one HER2-binding domain, and a tag-binding domain or a tag for use in a method for stimulating a chimeric antigen receptor-mediated immune response in a mammal, a nucleic acid, a vector or a cell comprising a nucleotide sequence encoding the targeting module, a pharmaceutical composition and a kit comprising the targeting module and a vector or a cell comprising a nucleotide sequence encoding a switchable chimeric antigen receptor.

Chimeric antigen receptors (CARs) are artificial receptors consisting of a binding moiety, which provides the antigen-specificity and one or several signaling chains derived from immune receptors (Cartellieri *et al.* 2010). These two principal CAR domains are connected by a linking peptide chain including a transmembrane domain, which anchors the CAR in the cellular plasma membrane. Immune cells, in particular T and NK cells, can be genetically modified to express CARs inserted into their plasma membrane. If such a CAR-modified immune cell encounters other cells or tissue structures expressing or being decorated with the appropriate target of the CAR binding moiety, upon binding of the CAR binding moiety to the target antigen, the CAR-modified immune cell is cross-linked to the target. Cross-linking leads to an induction of signal pathways via the CAR signaling chains, which will change the biologic properties of the CAR-engrafted immune cell. For example, CAR triggering in effector CD4+ and CD8+ T cells will activate typical effector functions like secretion of lytic compounds and cytokines, which will eventually lead to the killing of the respective target cell. In contrast, CAR activation in gene-modified regulatory T cells (Tregs) leads to an activation of Treg-specific immunomodulatory and suppressive mechanisms like interleukin (IL)-10 or tumor growth factor-beta (TGF-β) secretion. The adoptive transfer of immune cells engineered with chimeric antigen receptors (CARs) is currently considered a highly promising therapeutic option for the treatment of otherwise incurable malignant, infectious or autoimmune diseases. Until today, five CAR-T cell therapeutics have gained market approval for the treatment of B cell-derived malignancies, proving the clinical feasibility of this approach.

Brown *et al.* disclose CAR T cells targeting IL-13 receptor a2 (IL13Ra2) for the treatment of patients with glioblastoma (Brown *et al.* 2018). They describe improved anti-tumor activity and T cell persistence using a 4-1BB (CD137) co-stimulatory CAR (IL13BBξ) as compared to first-generation IL13ξ-CAR CD8+ T cells that had shown evidence for bioactivity in patients.

However, the conventional CAR technology comes along with a number of critical issues, which need to be solved before this treatment modality can be widely applied for clinical treatments. First of all, several safety issues have to be addressed. So far, immune responses of T cells engineered with conventional CARs are difficult to control after infusion into the patient. Serious adverse event rates are high (Titov *et al.* 2018). Especially unexpected target gene expression on normal tissue may provoke a rapid and rigorous immune reaction of engineered T cells against normal cells, which can cause severe side effects (Morgan *et al.* 2010). Moreover, as CAR-T cells are a new class of self-amplifying cell drugs, infused T cells can undergo a vigorous expansion in the presence of heavy tumor burden leading to tumor lysis syndrome, cytokine release syndrome and macrophage activation syndrome (Brudno and Kochenderfer 2016). Another drawback of conventional CAR technology is the restriction of engineered T cell retargeting to a single antigen. Such a monotherapeutic approach implies the risk for the development of tumor escape variants, which have lost the target antigen during treatment. The emergence of tumor escape variants under conventional CAR T cell therapy after several months was already observed in clinical trials (Sotillo *et al.* 2015). Taken together, these obstacles restrict the application of CART cells to very few indications. In fact, examples of clinical effectiveness have been restricted to CD19- and BCMA-targeting CAR T cells until now.

Modular "universal" CAR T (UniCAR) approaches can overcome these limitations by separating antigen recognition and activating domain of a CAR into two separate operational units. T cells are engineered to express a CAR with a universal binding domain recognizing a tag (Cartellieri *et al.* 2016). Antigen-specificity is provided by soluble adapter proteins, which consist of an antigen-binding domain fused to the tag recognized by the universal CAR. Cartellieri *et al.* describe the treatment of CD33- and/or CD123-positive acute myeloid leukemia cells *in vitro* and *in vivo.*

WO 2012/082841 A2 discloses universal anti-tag chimeric antigen receptor-expressing T cells and methods of treating cell-related disorders, e.g. cancer. In addition, WO 2013/044225 A1 discloses a universal immune receptor expressed by T cells for the targeting of diverse and multiple antigens. Both methods describe the use of modified T cells expressing universal anti-tag immune receptors. These T cells can be redirected to disease-related cell surface antigens by additionally applying soluble modules binding these surface antigens and carrying the respective tag. WO 2016/030414 A1 provides a genetically modified immune cell that allows a redirection against diverse disorders in a safe and efficient manner using endogenous tags based on nuclear proteins.

WO 2018/224660 A1 describes target modules for universal anti-tag chimeric antigen receptor-expressing T cells comprising chemically synthesized peptide-binding units specific for a human surface protein or protein complex and their advantages, in particular the fast and easy preparation, the improved stability and good pharmaceutical properties. WO 2016/168773 A2 discloses a peptide neo-epitope (PNE)-based switch for CAR-T cells comprising a receptor-binding partner, in particular a peptide antigen, a target-binding moiety, in particular a soluble T cell receptor or a part thereof or an antibody fragment, and a linker.

WO 2015/057834 A1 discloses switchable CAR-T cells and a switch for CAR-T cells comprising a receptor-binding partner, in particular a peptide antigen. The peptide antigen may be a natural, a non-natural or an artificial peptide, in particular yeast transcription factor GCN4 or a homologue thereof. Furthermore, the switch comprises a target-binding moiety, in particular an antibody or antibody fragment, preferably selected from an anti-Her2 antibody, an anti-BCMA antibody, an anti-CD19 antibody, an anti-CEA antibody and fragments thereof, and a linker.

Yu *et al.* describes different generations of CAR-T cells and strategies to reduce toxicity, including the so-called cytokine release syndrome and on-target off-tumour toxicity (Yu *et al.* 2019), in particular switchable CAR-T cells, in particular by suicide genes, for example, herpes simplex virus thymidine kinase (HSV-tk) or inducible caspase 9 (iCasp9) or a universal CAR system using FITC-binding CAR-T cells and small molecules comprising FITC and an antibody for a tumor target, in particular cetuximab, trastuzumab or rituximab. Further, Yu *et al.* points out the disadvantages of iCAR-T cells, in particular that iCAR-T cells recognize only tissue-specific antigens that are absent or down-regulated on tumors and expressed by the off-target tissue, and that no temporal and spatial control of iCAR-T cells is possible.

WO 99/51643 A1 describes chimeric molecules comprising mutagenized IL13 having one or more mutations in the domain that interacts with the hlL4 receptor subunit designated the 140 kDa hIL4R_{β} subunit with increased specificity for cancer cells as compared to normal cells for specifically delivering effector molecules to neoplasias. Preferably, the mutagenized IL13 has a mutation of amino acid residue selected from the group consisting of residue 13, residue 66, residue 69, residue 109, and residue 112, and is a basic amino acid, in particular lysine, or aspartic acid.

The object of the present invention is to provide an alternative approach for targeting IL13RA2- and/or HER2-expressing tumors, preferably glioblastoma, breast cancer and melanoma; with high specificity.

The object has been solved by a targeting module comprising
i) at least one IL13Ra2-binding domain,
ii) a tag-binding domain or a tag,
for use in a method for stimulating a chimeric antigen receptor-mediated immune response in a mammal, preferably for use in the treatment of cancer, infectious disease or autoimmune disease; wherein the targeting module is administered in combination with a vector or a cell comprising a nucleotide sequence encoding a switchable chimeric antigen receptor,
wherein the switchable chimeric antigen receptor comprises three domains, wherein
- the first domain is a tag-binding domain or a tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

Advantageously, the targeting module according to the invention used in combination with a switchable chimeric antigen receptor actively targets IL13Rα2 expressed by tumor cells and is capable of inducing a significant anti-tumor response, wherein the anti-tumor response of the switchable chimeric antigen receptor is only been induced in the presence of the targeting module. The effect can be interrupted immediately by withholding the administration of the targeting module. Further advantageously, the pharmacokinetic and pharmacodynamic half-life of the targeting module according to the invention is short, preferably in the range of 10 min to 5 h, providing a rapid and reversible switch-off mechanism of the mediated immune response.

As used herein, the term "targeting module" refers to an polypeptide or protein with at least two different domains, wherein each domain is specific for a target or a uniform group of targets, respectively, wherein at least one domain is specific for a target cell, in particular the IL13Rα2- and/or HER2-binding domain; and one domain is specific for a switchable chimeric antigen receptor, in particular the tag-binding domain or the tag. In embodiments, the targeting module is isolated. Preferably, the targeting module according to the invention is expressed as a recombinant protein. In further embodiments, the targeting module is chemically synthesized.

According to the invention, the targeting module comprises at least one IL13Rα2-binding domain.

As used herein "IL13Ra2" (Interleukin-13 receptor subunit alpha-2) refers to a membrane-bound protein that in humans is encoded by the IL13RA2 gene and binds IL13 with high affinity.

The term "autoimmune disorder" refers to an abnormal immune response of the body against substances and tissues normally present in the body (autoimmunity).

As used herein, the term "domain" refers to a part of a protein sequence, which can exist and function independently from the rest of the protein.

As used herein, the term "specific" refers to the ability of an antibody or antibody fragment or a protein, peptide or low molecular weight organic ligand to recognize and bind with a binding partner (e.g. a tumor antigen) protein present in a sample, but not substantially recognize or bind other molecules in the sample.

As used herein, the term "binds" or "binding" refers to a non-covalent binding, in particular ionic bonds, hydrogen bonds, Van der Waals forces and/or hydrophobic interactions.

As used herein, the term "administered in combination" refers to a treatment, wherein the targeting module is administered prior to, simultaneously with and/or after the administration of the vector or cell comprising a nucleotide sequence encoding a switchable chimeric antigen receptor.

In embodiments, the targeting module is administered on its own, preferably one hour to 2 days, more preferably 4 to 24 hours, prior to the administration of the vector or cell comprising a nucleotide sequence encoding a switchable CAR. Advantageously, the administration of the targeting module prior to the administration of the vector or cell comprising a nucleotide sequence encoding a switchable chimeric antigen receptor stimulates the switchable chimeric antigen receptors and increases the expansion of the switchable chimeric antigen receptor carrying effector cells and their accumulation at the target site.

In further embodiments, the targeting module is administered simultaneously with the vector or cell comprising a nucleotide sequence encoding a switchable CAR.

In further embodiments, the targeting module is administered until, preferably in the range of 3 days to 30 days, after the administration of the vector or cell comprising a nucleotide sequence encoding a switchable chimeric antigen receptor. Furthermore, additional such doses of the targeting module may be administered following resting periods to reactivate the switchableCAR-carrying effector cells.

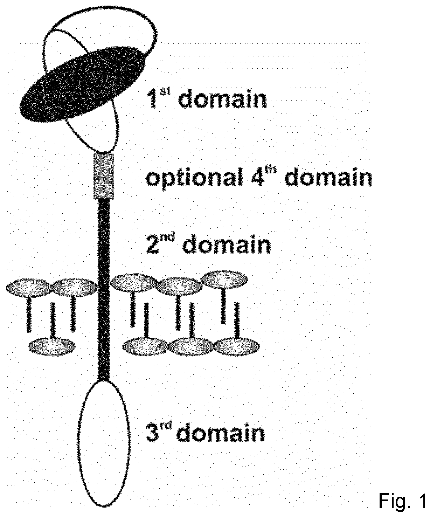

As used herein, the term "switchable chimeric antigen receptor" refers to an artificial chimeric fusion protein, in particular a receptor comprising a tag-binding domain or a tag, an extracellular hinge and a transmembrane domain and a signal transduction domain (Fig. 1). The domains can be derived from different sources and therefore, the receptor is called chimeric. Advantageously, the receptor can bind with the tag-binding domain or tag to different targeting modules.

Advantageously, the cell comprising a nucleotide sequence encoding a switchable CAR expresses the switchable CAR, which has binding specificity for the tag-binding domain or tag of the targeting module, which in turn binds to IL13Ra2 and/or HER2 on a target cell.

In embodiments, the targeting module is in monomeric, dimeric or polymeric form, preferably in monomeric form.

In further embodiments, the targeting module is monovalent, bivalent or multivalent.

In some embodiments, the targeting module according to the invention is bivalent or multivalent and comprises at least one IL13Ra2-binding domain and/or at least one HER2-binding domain.

Preferably, the IL13Ra2-binding domain is an IL13 mutein or an antibody or antibody fragment. Preferably, the HER2-binding domain is an antibody or antibody fragment.

As used herein, the term "antibody" refers to a protein, which binds antigens via the antigen-binding fragment variable region (Fab). This is composed of one constant and one variable domain of each of the heavy (V_{H}) and the light chain (V_{L}). As used herein, the term "antibody fragment or antigen-binding fragment" refers to a protein comprising at least the V_{L} or V_{H} of an antibody. In an embodiment, antibody fragments are selected from single-chain variable fragments (scFv), single-chain antibodies, F(ab')2 fragments, Fab fragments, and fragments produced by a Fab expression library or single-domain antibodies (nanobodies).

As used herein, the term "single-chain variable fragment (scFv)" refers to an artificial antibody fragment comprising a variable domain of a light chain and a variable domain of a heavy chain of an antibody covalently linked. In embodiments, the V_{L} and V_{H} of an antibody are covalently linked by a short peptide of 10 to 25 amino acids. In further embodiments, the short peptide links the N-terminus of the V_{H} with the C-terminus of the V_{L}, or vice versa.

Preferably, V_{H} and V_{L} are connected via a glycine-serine linker with the structure (GₓS_{y}) with x and y selected from 1 to 10, preferably 1 to 5. Mostly preferred are 1 to 10 repeats of the sequence G₄S₁ (SEQ ID No. 31). Moreover, linkers are preferred that are constituted of a peptide sequence that can increase the protease resistance of the antibody derivatives.

In embodiments, the linker is SEQ ID No. 32 or SEQ ID No. 33.

In embodiments, the antibody is obtained from an animal species, preferably from a mammal such as human, simian, mouse, rat, rabbit, guinea pig, horse, cow, sheep, goat, pig, dog or cat. Preferably, the antibody or antibody fragment is a human, humanized or deimmunized antibody. Humanized antibodies can be prepared in various ways, for example, by resurfacing and CDR grafting. In case of resurfacing, a combination of molecular modeling, statistical analyses, and mutagenesis is used to modify all non-CDR regions on the surface of the antibody to become similar to the surface of antibodies of the target organism. In CDR grafting, the CDR regions according to the invention are introduced into known human framework regions, which are similar in sequence to the original ones. Deimmunized antibodies can be obtained by specifically mutating residues that confer immunogenicity hotspots as predicted based on *in silico* peptide-MHC affinity prediction.

In embodiments, the antibody or antibody fragment is a polyclonal, a monoclonal or a chimeric antibody, wherein an antigen-binding region of a non-human antibody is transferred into the framework of a human antibody by recombinant DNA techniques including *in silico* design.

In embodiments, antibodies to a selected tag or antigen may be produced by immunization of various hosts including, but not limited to, goats, rabbits, rats, mice, humans, through injection with cells expressing a particular protein, DNA or RNA encoding for the protein, the protein itself or any portion, fragment or oligopeptide that retain immunogenic properties of the protein.

In embodiments, the IL13Ra2-binding domain comprises a human IL13 according to SEQ ID No. 1 or an IL13 mutein with a sequence identity of at least 95 % with SEQ ID No. 1, preferably human IL13 E11Y-G30Y according to SEQ ID No. 2 or human IL13 E11Y-M32A according to SEQ ID No. 3, or an antibody or antibody fragment, wherein the V_{L} comprises the amino acid complementarity determining region (CDR) sequences SEQ ID No. 11, SEQ ID No. 12 and SEQ ID No. 13, and the V_{H} comprises the amino acid sequences SEQ ID No. 14, SEQ ID No. 15 and SEQ ID No. 16.

Advantageously, the at least one IL13Ra2-binding domain does not bind to IL13Ra1, which is expressed ubiquitously in normal tissues.

As used herein, the term "IL13" refers to a protein that in humans is encoded by the IL13 gene, a cytokine secreted by T helper type 2 cells, CD4 cells, natural killer T cell, mast cells, basophils, eosinophils and nuocytes and is a central regulator in IgE synthesis, goblet cell hyperplasia, mucus hypersecretion, airway hyper responsiveness, fibrosis and chitinase up-regulation.

As used herein, the term "mutein" refers to a protein with an altered amino acid sequence, in particular a protein that results from the translation of a mutation.

In embodiments, the IL13Ra2-binding domain comprises an IL13 mutein with a sequence identity of at least 98% sequence identity with SEQ ID No. 1, preferably at least 99% sequence identity with SEQ ID No. 1. In embodiments, the IL13Ra2-binding domain comprises an IL13 mutein with an amino acid sequence according to SEQ ID No. 4, wherein X₁₁, X₃₀ and X₃₂ are independently from each other selected from a proteinogenic alpha-amino acid residue.

In embodiments, X₁₁ is E or Y, X₃₀ is G or Y and/or X₃₂ is M or A.

As used herein, the term "CDR (Complementarity-determining regions)" refers to parts of the variable chains in antibodies or antibody fragments, where the antibodies or antibody fragments bind to their specific antigen. An antibody comprises three CDRs (CDR1, CDR2 and CDR3), arranged non-consecutively, on the amino acid sequence of each variable domain and thus, six CDRs on the two variable domains (V_{H} and V_{L}), which can come into contact with the antigen.

In embodiments, the IL13Ra2-binding domain is a human IL13 according to SEQ ID No. 1 or an IL13 mutein with a sequence identity of at least 95 % with SEQ ID No. 1, preferably at least 98 % with SEQ ID No. 1, more preferably at least 99 % with SEQ ID No. 1, most preferably human IL13 E11Y-G30Y according to SEQ ID No. 2 or human IL13 E11Y-M32A according to SEQ ID No. 3, or an antibody or antibody fragment, wherein the V_{L} comprises the amino acid complementarity determining region (CDR) sequences SEQ ID No. 11, SEQ ID No. 12 and SEQ ID No. 13, and the V_{H} comprises the amino acid sequences SEQ ID No. 14, SEQ ID No. 15 and SEQ ID No. 16.

In embodiments, the IL13Ra2-binding domain comprises an antibody or antibody fragment comprising a V_{L} according to SEQ ID No. 17, wherein X₁, X₂, X₄, X₁₀, X₁₃, X₁₄, X₁₈, X₁₉, X₂₁, X₂₂, X₂₄, X₄₁, X₆₀, X₇₃, X₇₇, X₇₈, X₈₀, X₈₃, X₈₄, X₈₅ and X₈₇ are independently from each other selected from a proteinogenic alpha-amino acid residue, and/or a V_{H} according to SEQ ID No. 18, wherein X₁₂, X₂₀, X₃₈, X₄₈, X₆₁, X₆₂, X₆₅, X₆₆, X₆₈, X₇₀, X₇₂, X₇₄, X₇₅, X₇₆, X₇₉, X₈₃, X₈₅, X₈₉, X₁₁₀ and X₁₁₄ are independently from each other selected from a proteinogenic alpha-amino acid residue, preferably the V_{L} according to one of the sequence SEQ ID No. 19 to 24 and/or the V_{H} according to one of the sequence SEQ ID No. 25 to 30.

In embodiments, the IL13Ra2-binding domain is an antibody or antibody fragment comprising a V_{L} according to SEQ ID No. 17, wherein X₁, X₂, X₄, X₁₀, X₁₃, X₁₄, X₁₈, X₁₉, X₂₁, X₂₂, X₂₄, X₄₁, X₆₀, X₇₃, X₇₇, X₇₈, X₈₀, X₈₃, X₈₄, X₈₅ and X₈₇ are independently from each other selected from a proteinogenic alpha-amino acid residue, and/or a V_{H} according to SEQ ID No. 18, wherein X₁₂, X₂₀, X₃₈, X₄₈, X₆₁, X₆₂, X₆₅, X₆₆, X₆₈, X₇₀, X₇₂, X₇₄, X₇₅, X₇₆, X₇₉, X₈₃, X₈₅, X₈₉, X₁₁₀ and X₁₁₄ are independently from each other selected from a proteinogenic alpha-amino acid residue, preferably the V_{L} according to one of the sequence SEQ ID No. 19 to 24 and/or the V_{H} according to one of the sequence SEQ ID No. 25 to 30.

In embodiments, X₁ is D or Q, X₂ is I or V, X₄ is L or M, X₁₀ is F or T, X₁₃ is V or L, X₁₄ is T or S, X₁₈ is K or R, X₁₉ is V or A, X₂₁ is L or I, X₂₂ is T or S, X₂₄ is R or Q, X₄₁ is D or G, X₆₀ is S or D, X₇₃ is L or F, X₇₇ is S or R, X₇₈ is V or L, X₆₀ is A or P, X₈₃ is F or A, X₈₄ is G or A, X₈₅ is T or V and/or X₈₇ is Y or F.

In embodiments, X₁₂ is V or K, X₂₀ is L or V, X₃₈ is K or R, X₄₈ is I or M, X₅₁ is A or N, X₅₂ is Q or E, X₆₅ is Q or K, X₅₆ is G or T, X₆₈ is A or V, X₇₀ is L or M, X₇₂ is V or R, X₇₄ is T or K, X₇₅ is S or P, X₇₆ is I or S or T, X₇₉ is A or V, X₈₃ is I or R, X₈₅ is R or S, X₈₉ is D or E, X₁₁₀ is Q or A, X₁₁₄ is L or V.

In more preferred embodiments, the IL13Ra2-binding domain comprises an antibody or antigen-binding fragment thereof comprising one of the sequences according to SEQ ID No. 5 to SEQ ID No. 10. Preferably, the IL13Rα2-binding domain is an antibody or antigen-binding fragment thereof comprising one of the sequences according to SEQ ID No. 5 to SEQ ID No. 10.

As used herein, the term "tag" refers to a marker, in particular a peptide sequence or an organic molecule, attached to peptides or proteins to enable them to bind to specific atoms, ions or molecules, in particular the tag-binding domain.

In embodiments, the tag is selected from organic molecules including fluorescence labels, e.g. FITC (Fluorescein isothiocyanate), and biotin.

In embodiments, the tag is a peptide epitope tag. In further embodiments, the tag comprises 10 to 20 amino acids.

In embodiments, the peptide epitope tag is a myc-tag, a His-tag, a short linear peptide sequence from yeast transcription factor GCN4, preferably according to SEQ ID No. 39, SEQ ID No. 40 or mutants thereof; a leucine zipper sequence, preferably SYNZIP 1 to SYNZIP 48, BATF, FOS, ATF4, ATF3, BACH1, JUND, NFE2L3, HEPTAD (Reinke *et al.* 2010), a sequence according to SEQ ID No. 41 or SEQ ID No. 42 or mutants thereof; or a short linear peptide sequence from a human nuclear protein, preferably from the human La protein.

In preferred embodiments, the peptide epitope tag is a myc-tag, a His-tag, a short linear peptide sequence from yeast transcription factor GCN4, preferably according to SEQ ID No. 39, SEQ ID No. 40; a leucine zipper sequence, preferably SYNZIP 1 to SYNZIP 48, BATF, FOS, ATF4, ATF3, BACH1, JUND, NFE2L3, HEPTAD (Reinke *etal.* 2010), a sequence according to SEQ ID No. 41 or SEQ ID No. 42; or a short linear peptide sequence from a human nuclear protein, preferably from the human La protein.

Preferably, the peptide epitope tag from the human La protein is the human La epitope E5B9 according to SEQ ID No. 43 or E7B6 according to SEQ ID No. 44 or SEQ ID No. 45, most preferably the human La epitope E5B9 according to SEQ ID No. 43 or E7B6 according to SEQ ID No. 45.

As used herein, the term "mutants" refers to peptides or proteins having at least 85 % sequence identity to the named peptides or proteins, preferably at least 95 % sequence identity. Advantageously, the mutants are capable of having one or more activities of the named peptides or proteins, in particular bind the identical tag-binding domains as the peptide epitope tag.

In embodiments, mutants are truncated versions of peptides or proteins. As used herein, the term "truncated versions" refers to shortened peptides or proteins having at least 85 % sequence identity to the named peptides or proteins, preferably at least 95 % sequence identity, more preferably having a chain length of at least 85 % and a sequence identity of 100 %, most preferably a chain length of at least 95 % and a sequence identity of 100 %. Advantageously, the truncated version has at least 80 %, preferably of at least 90 %, more preferably of at least 95 %; of the activity of the named peptide or protein.

As used herein, the term "nuclear protein" refers to a protein found in the cell nucleus.

In embodiments, the His-tag is an amino acid sequence consisting of histidine residues, preferably in the range of six to fourteen histidine residues.

Advantageously, tags, which are peptide sequences from nuclear antigens, cannot be accessed and bound by the corresponding tag-binding domain in the context of the native protein under physiological conditions. Further advantageously, the tag is not immunogenic. This leads to minimization of the risk of uncontrolled on-target off-site toxicities by CAR-expressing immune cells like the release of toxic levels of cytokines, referred to variously as cytokine storms or cytokine release syndrome (CRS).

In embodiments, the tag-binding domain is an antibody or an antibody fragment.

In embodiments, the tag-binding domain is an antibody or an antibody fragment binding to a myc-tag, a His-tag, a short linear peptide sequence from yeast transcription factor GCN4, a leucine zipper sequence or a short linear peptide sequence from a human nuclear protein.

In embodiments, the tag-binding domain binds to a tag from the human nuclear La protein; preferably, the tag-binding domain is an antibody or an antigen-binding fragment, comprising a V_{L} according to the following sequence:
DIVMTQSPDSLAVSLGERATINCX₂₄SSQSLLNSRTX₃₅KNYLAWYQQKPGQPPKLLIYWASTR X₆₁SGVPDRFSGSGSGTDFTLTISSLQAEDVAVYYCKQSYNLX1₀₁TFGGGTKVEIK (SEQ ID No. 46), wherein X₂₄, X₃₅, X₆₁ and X₁₀₁ are independently from each other selected from a proteinogenic alpha-amino acid residue;
or a sequence having at least 85 % sequence identity, preferably at least 95 % sequence identity; to one of the sequences SEQ ID No. 48 or SEQ ID No. 50.

In embodiments, the tag-binding domain constitutes an anti-La epitope scFv.

In some embodiments, X₂₄ to X₁₀₁ are selected as follows:
X₂₄ is selected from polar and/or positive charged residues, such as Serine, Threonine, Asparagine, Glutamine, Histidine, Lysine and Arginine; preferably Lysine or Arginine;
X₃₅ is preferably selected from Lysine and Proline;
X₆₁ is selected from polar and charged residues, such as Asparagine, Aspartic Acid, Glutamine, Glutamic acid, Histidine, Lysine and Arginine, preferably Glutamic acid and Lysine;
X₁₀₁ is selected from hydrophobic residues, such as Isoleucine, Leucine, Valine, Alanine, Methionine, Phenylalanine, Proline and Tryptophan; preferably Leucine or Proline.

In further embodiments, the tag-binding domain is an antibody or an antigen-binding fragment comprising a sequence having at least 85 % sequence identity, preferably at least 95 % sequence identity; to one of the sequences SEQ ID No. 47 or SEQ ID No. 49.

Preferably, the tag-binding domain comprises a sequence having each at least 85 % sequence identity, preferably at least 95 % sequence identity; to the sequences according to SEQ ID NO. 47 (V_{H}) and SEQ ID NO. 48 (V_{L}) or the sequences according to SEQ ID NO. 49 (V_{H}) and SEQ ID NO. 50 (V_{L}).

Most preferably, the tag-binding domain constitutes the anti-La 5B9 scFv according to SEQ ID NO. 47 (V_{H}) and SEQ ID NO. 48 (V_{L}) or the anti-La 7B6 scFv according to SEQ ID NO. 49 (V_{H}) and SEQ ID NO. 50 (V_{L}).

In embodiments, the targeting module for use in a method for stimulating a chimeric antigen receptor-mediated immune response in a mammal further comprises an additional effector cell-binding domain, wherein the effector cell-binding domain specifically binds an epitope on a human CD3 chain, a human T cell receptor (TCR) or human CD16.

As used herein, the term "effector cell-binding domain" refers to a peptide or protein, which specifically binds a protein or protein complex (antigen) on an effector cell. As used herein, the term "protein complex" refers to a group of two or more associated protein chains.

As used herein, the term "effector cell" refers to an immune cell, which executes immune reactions, in particular an immune cell with cytolytic, phagocytic and/or immunosuppressive activity. Preferably, the effector cell is a T cell, a natural killer (NK) cell or a macrophage. Advantageously, the targeting module activates the effector cell by binding to the domain and exceeding a certain threshold regarding required number of such interactbns.

Preferably, the effector cell-binding domain is an antibody or antibody fragment.

In embodiments, the effector cell-binding domain specifically binds an epitope on human CD3 with a dissociation constant K_{d} up to 10⁻⁸ M. Commonly, the affinity of the effector cell-binding domain, in particular the dissociation constant K_{d}, is determined with surface plasmon resonance measurements or flow-cytometry-based cellular binding assay.

Preferably, the effector cell-binding domain specifically binds an epitope on human CD3 with a low affinity, preferably with a dissociation constant K_{d} in the range of 10⁻⁴ M to 10⁻⁸ M.

In embodiments, the variable regions of the at least one IL13Ra2- and/or HER2-binding domain and/or the effector cell-binding domain comprise a humanized amino acid sequence.

In embodiments, the effector cell-binding domain comprises at least one of the following sequences:
i) a V_{L} according to SEQ ID No. 51 or 53 and/or
ii) a V_{H} according to SEQ ID No. 52 or 54.

In embodiments, the effector cell-binding domain comprises at least one sequence with a sequence identity of at least 90 %, preferably at least 95 %, more preferably at least 98 % to SEQ ID No. 51 or 53 and/or SEQ ID No. 52 or 54, wherein the at least one sequence binds the antigen CD3.

Preferably, the humanized anti-human CD3 effector cell-binding domain comprises a variable region of the heavy chain (V_{H}) according to SEQ ID No. 54 and a variable region of the light chain (V_{L}) according to SEQ ID No. 53, e.g. in the form of an scFv. Preferably, V_{H} and V_{L} are connected via glycine serine linker.

In some embodiments, the effector cell-binding domain is a humanized anti-TCR domain and preferably comprises at least one of the following sequences:
i) a V_{L} according to SEQ ID No. 55 and/or
ii) a V_{H} according to SEQ ID No. 56.

In embodiments, the effector cell-binding domain comprises at least one sequence with a sequence identity of at least 90 %, preferably at least 95 %, more preferably at least 98 % to the sequences according to SEQ ID No. 55 or SEQ ID No. 56, wherein the at least one sequence binds the antigen TCR.

In some embodiments, the effector cell-binding domain is a humanized anti-CD16A domain and preferably comprises at least one of the following sequences:
i) a V_{L} according to SEQ ID No. 57 and/or
ii) a V_{H} according to SEQ ID No. 58,
more preferably, a V_{L} according to SEQ ID No. 57 and a V_{H} according to SEQ ID No. 58.

In an embodiment, the effector cell-binding domain comprises at least one sequence with a sequence identity of at least 90 %, preferably at least 95 %, more preferably at least 98 % to the sequences according to SEQ ID No. 57 or SEQ ID No. 58, wherein the at least one sequence binds the antigen CD16A.

In embodiments, the different domains of the targeting module according to the invention are linked with each other by a linker. The linker comprises a short sequence of preferably 10 to 20 amino acid residues. In embodiments, the targeting module comprises a flexible peptide sequence that is selected such that the domains have a three-dimensional folding that allows them to exhibit the specificity for effector cell and target cell binding. Preferred linkers are glycine-serine linkers with the structure (GₓS_{y}) with x and y selected from 1 to 10, preferably 1 to 5. Mostly preferred are 1 to 10 repeats of the sequence G₄S₁ (SEQ ID No. 31). Moreover, linkers are preferred that are constituted of a peptide sequence that can increase the protease resistance of the antibody derivatives.

In embodiments, the linker is SEQ ID No. 32 or SEQ ID No. 33.

In embodiments, the targeting module for use in a method for stimulating a chimeric antigen receptor-mediated immune response in a mammal comprises one of the sequences according to SEQ ID No. 86 to SEQ ID No. 112.

In embodiments, the targeting module according to the invention comprises a further domain selected from the group comprising co-stimulatory ligands, radionuclides, cell death-inducing chemical compounds and half-life increasing domains, preferably IgG1 Fc, IgG2 Fc, IgG3 Fc, IgG4 Fc, HSA, FcRn-binding peptides or mutants thereof. As used herein, the term "mutants" refers to proteins having at least 85 % sequence identity to the half-life increasing domain, preferably at least 95 % sequence identity. Advantageously, the mutant is capable of having one or more activities of the named peptides or proteins; in particular, the mutant increases the half-life like the half-life increasing domain.

In preferred embodiments, the targeting module according to the invention comprises a further domain selected from the group comprising co-stimulatory ligands, radionuclides, cell death-inducing chemical compounds and half-life increasing domains, preferably IgG1 Fc, IgG2 Fc, IgG3 Fc, IgG4 Fc, HSA or FcRn-binding peptides.

In embodiments, the length of the targeting module is in the range of 20 to 1600 amino acids

In embodiments, the targeting module according to the invention is used in a method for stimulating a chimeric antigen receptor-mediated immune response in a mammal, wherein the targeting module is administered in combination with a vector or a cell comprising a nucleotide sequence encoding a switchable chimeric antigen receptor and at least one further targeting module, wherein the at least one further targeting module comprises at least one target cell-binding domain and a tag-binding domain or a tag, wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269 (BCMA), CD273, CD274, CD276, CD279, CD319, CD366 and CD371, interleukin receptors, especially preferred IL-8Ra (CXCR1), IL-8Rβ (CXCR2), IL-11Rα, IL-11Rβ, IL13Ra1, CXCR4, c-Met, mesothelin, members of the epidermal growth factor receptor family and mutants thereof, especially preferred ErbB1, ErbB2, ErbB3, ErbB4 or mutants thereof, members of the tumor necrosis factor receptor superfamily, ephrins, ephrin receptors, especially preferred EphA1-10, EphA5 or EphB1-6; prostate specific antigens, preferably prostate stem cell antigen (PSCA) and prostate specific membrane antigen (PSMA); embryonic antigen, preferably carcinoembryonic antigen (CEA) and fetal acethylcholine receptor, members of the vascular endothelia growth factor family, epithelia cell adhesion molecule (EpCAM), alphafetoprotein (AFP), members of the intercellular adhesion molecule family, members of the mucin protein family, follicle stimulating hormone receptor (FSHR), the human high molecular weight-melanoma-associated antigen (HMW-MAA), folate binding protein FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, melanoma-associated chondroitin sulfate proteoglycan (MCSP), glycoprotein A33, guanylate cyclase 2C and tumor-specific glycans, including mutants and analogues of the named antibodies, antibody fragments, proteins, peptides or low molecular weight organic ligands, wherein the targeting module according to the invention and the at least one further targeting module comprise identical tag-binding domains or tags.

As used herein, the term "target cell-binding domain" refers to a peptide, protein, or low molecular weight organic ligand, which specifically binds a protein or protein complex (antigen) on the surface of a target cell, preferably a cancer cell, T cell, infected cell, pathogens or parasites.

As used herein, the terms "peptide", "polypeptide" and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence.

As used herein, the term "low molecular weight organic ligand" refers to an organic molecule with a molecular weight of maximal 10 kilodaltons, preferably of maximal 3 kilodaltons, which specifically binds a protein or protein complex (antigen) on the surface of a target cell, preferably a cancer cell, T cell, infected cell or pathogens or parasites.

The term "target cell-binding domain" also comprises soluble T cell receptors, which are composed of the alpha and beta or the gamma and delta chains of a T cell receptor (TCR), fragments or mutants thereof. Such TCR-derived binding moieties recognize and bind to peptides presented by human leukocyte antigen class (HLA) I and II protein complexes. Examples are, but are not limited to, TCRs specific for peptides derived from proteins like EGFR family, survivin, sry-like high motility group box (SOX) protein family, melanoma-associated antigens (e.g. autoimmunogenic cancer/testis antigen NY-ESO-1, members of the melanoma antigen family A MAGEA, the preferentially expressed antigen in melanoma PRAME), and leukemia-associated antigens (e.g. Wilms tumor gene 1 WT1).

As used herein, the term "mutants" refers to peptides or proteins having at least 85 % sequence identity to the named antibodies, antibody fragments, proteins or peptides, preferably at least 95 % sequence identity. Advantageously, the mutants bind the identical antigens as the named antibodies, antibody fragments, proteins or peptides.

As used herein, the term "analogues" refers to molecules having a high degree of structural identity to the named antibodies, antibody fragments, proteins, peptides or low molecular weight organic ligands, preferably at least one atom, group of atoms, functional group or substructure is replaced with another group of atoms, e.g. a hydroxy group. In embodiments, an analogue of somatostatin (SRIF14) is octreotide or pasireotide. Advantageously, the analogues bind the identical antigens as the named antibodies, antibody fragments, proteins, peptides or low molecular weight organic ligands.

In embodiments, the analogues of the named antibodies, antibody fragments, proteins or peptides comprise modifications selected from the group comprising D amino acids, pseudo peptide bonds, aminoalcohols, non-proteinogenic amino acids, unnatural amino acids, amino acids with modified side chains and/or circular proteins. Advantageously, these analogues reveal increased stability.

In further embodiments, the target cell-binding domain is a soluble T cell receptor consisting of the alpha and beta or the gamma and delta chain of a T cell receptor (TCR).

In preferred embodiments, the at least one further targeting module comprises at least one target cell-binding domain and a tag-binding domain or a tag, wherein the at least one target cell-binding domain is an antibody or an antibody fragment that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269, CD273, CD274, CD276, CD279, CD319, CD366 and CD371, interleukin receptors, especially preferred IL-8Rα, IL-8Rβ, IL-11Rα, IL-11Rβ, IL13Ra1, CXCR4, c-Met, mesothelin, members of the epidermal growth factor receptor family, especially preferred ErbB1, ErbB2, ErbB3 or ErbB4; members of the tumor necrosis factor receptor superfamily, ephrins, ephrin receptors, especially preferred EphA1-10, EphA5 or EphB1-6, prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, folate binding protein FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, wherein the targeting module according to the invention and the at least one further targeting module comprise different target cell-binding domains, and identical tag-binding domains or a tags.

In preferred embodiments, the at least one further targeting module comprises at least one target cell-binding domain and a tag-binding domain or a tag, wherein the at least one target cell-binding domain is a protein, an antibody or an antibody fragment that binds to surface antigens selected from the group comprising HER2 (human epidermal growth factor receptor 2, ErbB2), PD-L1 (programmed cell death 1 ligand 1), NKG2D (natural killer group 2D) ligands, EGFR (epidermal growth factor receptor) and EGFRvIII (epidermal growth factor receptor variant III).

In embodiments, the at least one target cell-binding domain binding to HER2 is Trastuzumab, a Trastuzumab mutant or a fragment thereof, preferably comprising SEQ ID No. 37 and SEQ ID No. 38.

As used herein "PD-L1" (Programmed death ligand 1) refers to a transmembrane protein according to SEQ ID No. 176 that in humans is encoded by the CD274 or PDCD1LG1 gene and is a ligand of PD-1. In embodiments, the at least one target cell-binding domain binding to PD-L1 is selected from PD-1, BMS-936559, Atezolizumab, Durvalumab, Avelumab, 3F2.1 and fragments thereof. As used herein, the term "fragments" refers to proteins consisting of at least 90 %, preferably at least 95 %, more preferably at least 98 % of the amino acid sequence of the PD-L1 binding domains. Preferably, the PD-L1 binding domain is an scFv fragment of BMS-936559, Atezolizumab or Durvalumab.

As used herein, the term "PD-1" (Programmed cell death protein 1) refers to a protein on the surface of cells that has a role in regulating the immune system's response to the cells of the human body by down-regulating the immune system and promoting self-tolerance by suppressing T cell inflammatory activity. Preferably, the PD-L1 binding domain comprises at least one sequence with a sequence identity of at least 90 %, preferably at least 95 %, more preferably at least 98 % to SEQ ID No. 113 or a fragment thereof, wherein the at least one sequence binds the antigen PD-L1. In embodiments, the fragment of SEQ ID No. 113 comprises the extracellular domain of PD-1.

As used herein, the term "BMS-936559" refers to an antibody that specifically binds PD-L1. Preferably, the PD-L1 binding domain comprises at least one sequence each with a sequence identity of at least 90 %, preferably at least 95 %, more preferably at least 98 % to SEQ ID No. 114 and SEQ ID No. 115 or SEQ ID No. 116 and SEQ ID No. 117 or SEQ ID No. 118 and SEQ ID No. 119 or SEQ ID No. 120 and SEQ ID No. 121 or SEQ ID No. 122 and SEQ ID No. 123 or SEQ ID No. 124 and SEQ ID No. 125 or SEQ ID No. 126 and SEQ ID No. 127 or SEQ ID No. 128 and SEQ ID No. 129 or SEQ ID No. 130 and SEQ ID No. 131 or SEQ ID No. 132 and SEQ ID No. 133, wherein the at least one sequence binds the antigen PD-L1.

More preferably, the PD-L1 binding domain comprises at least one sequence each with a sequence identity of at least 90 %, preferably at least 95 %, more preferably at least 98 % to SEQ ID No. 114 and SEQ ID No. 115 or SEQ ID No. 126 and SEQ ID No. 127, wherein the at least one sequence binds the antigen PD-L1.

In embodiments, the PD-L1 binding domain comprises at least one sequence each with a sequence identity of at least 90 %, preferably at least 95 %, more preferably at least 98 % to one sequence of the group comprising SEQ ID No. 134, SEQ ID No. 135, SEQ ID No. 136, SEQ ID No. 137, SEQ ID No. 138, SEQ ID No. 139, SEQ ID No. 140, SEQ ID No. 141, SEQ ID No. 142, SEQ ID No. 143, SEQ ID No. 144, SEQ ID No. 145, SEQ ID No. 146, SEQ ID No. 147, SEQ ID No. 148, SEQ ID No. 149, SEQ ID No. 150, SEQ ID No. 151, SEQ ID No. 152 and SEQ ID No. 153.

Preferably, the PD-L1 binding domain comprises at least one sequence each with a sequence identity of at least 90 %, preferably at least 95 %, more preferably at least 98 % to SEQ ID No. 135 or SEQ ID No. 146.

As used herein, the term "Atezolizumab" refers to an antibody that specifically binds PD-L1. Preferably, the PD-L1 binding domain comprises at least one sequence each with a sequence identity of at least 90 %, preferably at least 95 %, more preferably at least 98 % to SEQ ID No. 154 and SEQ ID No. 155, wherein the at least one sequence binds the antigen PD-L1.

In embodiments, the PD-L1 binding domain comprises at least one sequence each with a sequence identity of at least 90 %, preferably at least 95 %, more preferably at least98 % to SEQ ID No. 156 or SEQ ID No. 157.

As used herein, the term "Durvalumab" refers to an antibody that specifically binds PD-L1. Preferably, the PD-L1 binding domain comprises at least one sequence each with a sequence identity of at least 90 %, preferably at least 95 %, more preferably at least 98 % to SEQ ID No. 158 and SEQ ID No. 159, wherein the at least one sequence binds the antigen PD-L1.

In embodiments, the PD-L1 binding domain comprises at least one sequence each with a sequence identity of at least 90 %, preferably at least 95 %, more preferably at least 98 % to SEQ ID No. 160 or SEQ ID No. 161.

As used herein, the term "Avelumab" refers to an antibody that specifically binds PD-L1. Preferably, the PD-L1 binding domain comprises at least one sequence each with a sequence identity of at least 90 %, preferably at least 95 %, more preferably at least 98 % to SEQ ID No. 162 and SEQ ID No. 163 or SEQ ID No. 164 and SEQ ID No. 165, wherein the at least one sequence binds the antigen PD-L1.

In embodiments, the PD-L1 binding domain comprises at least one sequence each with a sequence identity of at least 90 %, preferably at least 95 %, more preferably at least 98 % to SEQ ID No. 166 or SEQ ID No. 167.

As used herein, the term "3F2.1" refers to an antibody that specifically binds PD-L1. Preferably, the PD-L1 binding domain comprises at least one sequence each with a sequence identity of at least 90 %, preferably at least 95 %, more preferably at least 98 % to SEQ ID No. 168 and SEQ ID No. 169, wherein the at least one sequence binds the antigen PD-L1.

In embodiments, the at least one target cell-binding domain binding to EGFRvIII is derived from antibody clone MR1 (SEQ ID No. 170 and SEQ ID No. 171), 139 (SEQ ID No. 172 and SEQ ID No. 173), RM419, L8A4 (Wikstrand *et al.* 1995), Y10 (Wikstrand *et al.* 1995), H10 (Wikstrand *et* al. 1995) or D2C7 (SEQ ID No. 174 and SEQ ID No. 175).

More preferred, the at least one further targeting module comprises at least one target cell-binding domain and a tag-binding domain or a tag, wherein the at least one target cell-binding domain is an antibody or an antibody fragment that binds to HER2.

In further embodiments, the at least one further targeting module comprises two target cell-binding domains binding to surface antigens selected from PSCA and PSMA, ErbB-1 and ErbB-2, PSCA and ErbB-2, PSMA and CEA, IL13Ra2 and ErbB-2, CD38 and CD269, mesothelin and mucin 16, PD-L1 and ErbB-2.

In preferred embodiments, the targeting module according to the invention is used in a method for stimulating a chimeric antigen receptor-mediated immune response in a mammal, wherein the targeting module is administered until, preferably in the range of 3 days to 30 days, after the administration of the vector or cell comprising a nucleotide sequence encoding a switchable chimeric antigen receptor and the at least one further targeting module. Advantageously, by using the targeting module in combination with a further targeting module, wherein the targeting module is administered after the administration of the vector or cell comprising a nucleotide sequence encoding a switchable chimeric antigen receptor and the further targeting module, adverse systemic effects are reduced. Furthermore, additional such doses of the targeting modules may be administered following resting periods to reactivate the switchable chimeric antigen receptor-carrying effector cells.

More preferably, the targeting module according to the invention is used in a method for stimulating a chimeric antigen receptor-mediated immune response in a mammal, wherein the at least one further targeting module is administered on its own, preferably one hour to 2 days, more preferably 4 to 24 hours, prior to the administration of the vector or cell comprising a nucleotide sequence encoding a switchable chimeric antigen receptor and the targeting module according to the invention is administered until, preferably in the range of 3 days to 30 days, after the administration of the vector or cell comprising a nucleotide sequence encoding a switchable chimeric antigen receptor. Furthermore, additional such doses of the targeting module may be administered following resting periods to reactivate the switchable chimeric antigen receptor-carrying effector cells.

The present invention further comprises a nucleic acid, a vector or a cell comprising a nucleotide sequence encoding a targeting module according to the invention for use in a method for stimulating a chimeric antigen receptor-mediated immune response in a mammal, preferably for use in the treatment of cancer, infectious disease or autoimmune disease;
wherein the nucleic acid, vector or cell is administered in combination with a vector or a cell comprising a nucleotide sequence encoding a switchable chimeric antigen receptor, wherein the switchable chimeric antigen receptor comprises three domains, wherein
   - the first domain is a tag-binding domain or a tag,
   - the second domain is an extracellular hinge and a transmembrane domain and
   - the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

According to the invention, the nucleic acid, vector and/or cell are isolated. As used herein, the term "isolated" means altered or removed from the natural state.

In embodiments, the nucleic acid is a cDNA. As used herein, the term "cDNA" (complementary DNA) refers to double-stranded DNA synthesized from a single-stranded RNA, e.g. mRNA, in a reaction catalyzed by the enzyme reverse transcriptase. In embodiments, cDNA is of synthetic origin. In further embodiments, cDNA is derived from mRNA, therefore containing only exons but no introns, as opposed to genomic DNA.

The vector is preferably a plasmid, an artificial chromosome, linearized DNA or RNA, a virus particle or another vector that contains an expression cassette that is incorporated stably into the genome of a host cell or host organism.

In embodiments, the cell is selected from immune cells, preferably with cytolytic, phagocytic or immunosuppressive activity, such as T cells, Natural Killer (NK) cells and macrophages. In preferred embodiments, the cell is selected from T cells, including alpha/beta and gamma/delta T cells or subpopulations of T cells like stem-cell memory T cells or central memory T cells, cytotoxic T cells or NK cells.

In embodiments, the nucleic acid, vector or cell further comprisesan inducible expression system. In some embodiments, the inducible expression system is based on a prokaryotic operon, including, but not limited to, the lac operon, transposon Tn10 or tetracycline operon. In other embodiments, the inducible expression system is based on components of a eukaryotic signaling pathway, including, but not limited to, expression systems based on a steroid receptor, an estrogen receptor, progesterone or metallothionein.

In preferred embodiments, the inducible expression system is selected from Tet-On^{®} (also rtTA; TET Systems, Takara Bio), Tet-On^{®} Advanced (also rtTA2^{s}-M2 or rtTA2-syn1; TET Systems, Takara Bio), Tet-On^{®} 3G (also rtTA-V16; TET Systems, Takara Bio), T-REx^{®} (Life Technologies) or a promoter responsive to nuclear factor of activated T cells (NFAT).

As used herein, the term "Tet-On" refers to an inducible expression system comprising at least a tetracycline response element (TRE) and reverse tetracycline transactivator (rtTA) protein or variant thereof. As used herein, the term "reverse tetracycline transactivator (rtTA) protein" refers to a protein, which is capable of binding the operator (tetO) on the TRE, if bound by a tetracycline or a derivative of tetracycline, e.g. doxycycline. Thus, the introduction of tetracycline or a derivative of tetracycline to the system initiates the transcription of the genetic product. In embodiments, the inducible expression system induces the transcription of the nucleotide sequence encoding a switchable chimeric antigen receptor and/or a nucleotide sequence encoding a targeting module according to the invention, preferably the inducible expression system induces the transcription of the nucleotide sequence encoding a targeting module according to the invention.

As used herein, the term "variant" refers to a nucleotide sequence, peptide or protein, which is capable of performing one or more functions of the nucleotide or named amino acid sequence.

As used herein, the term "derivative" refers to a molecule having a high degree of structural identity to the molecule, preferably the same scaffold, wherein at least one atom, group of atoms, functional group or substructure is replaced with another atom, group of atoms functional group or substructure, e.g. a hydroxy group. Advantageously, the derivative is capable of having one or more activities of the named molecule.

Advantageously, the Tet-On system is preferred over Tet-Off for its faster responsiveness and its activation rather than repression by tetracycline or a derivative of tetracycline.

In embodiments, the rtTA protein is a fusion protein of rTetR (reverse tetracycline repressor), a mutated version of TetR (tetracycline repressor) from *Escherichia coli,* and the activation domain of virion protein 16 (VP16) from Herpes Simplex Virus. In preferred embodiments, rtTA comprises SEQ ID No. 59, SEQ ID No. 60 or SEQ ID No. 61.

As used herein, the term "tetracycline response element (TRE)" refers to at least one TetO sequence with a minimal promoter, responding to binding of the rtTA protein by increased expression of the gene or genes downstream of its promoter.

In embodiments, the TRE consists of 2 to 9 repeats, preferably 7 repeats, of the 19bp bacterial TetO sequence according to SEQ ID No. 62, preferably separated by spacer sequences, more preferably by spacer sequences comprising SEQ ID No. 63.

In preferred embodiments, the TRE is SEQ ID No. 64.

As used herein, the term "Tet-On Advanced" refers to a Tet-On system with reduced basal expression and increased sensitivity to doxycycline, which is human codon optimized and comprises three minimal transcriptional activation domains, corresponding to the rtTA of SEQ ID No. 60.

As used herein, the term "Tet-On 3G" refers to a Tet-On system with further increased sensitivity to doxycycline, corresponding to the rtTA of SEQ ID No. 61.

As used herein, the term "T-REx" refers to a system, where the gene of interest is flanked by an upstream CMV promoter and two TetO2 sites. Binding of TetR homodimers to each TetO2 site represses gene expression in the absence of tetracycline or a derivative of tetracycline. In the presence of tetracycline or a derivative of tetracycline to the TetR homodimers, they dissociate from TetO2 resulting in expression of the gene of interest.

As used herein, the term "promoter responsive to nuclear factor of activated T cells (NFAT)" refers to a promoter naturally occurring in human cell nucleus, which is bound by nuclear factors of activated T cells (NFAT) initiating the transcription of the genetic product. As used herein, the term "nuclear factors of activated T cells (NFAT)" refers to transcription factors with one DNA-binding domain for transcription and two calcineurin-binding domains for the activation by calcineurin. The activation by calcineurin takes place by the activation of the serine/threonine phosphatase calcineurin by calmodulin, a calcium sensor protein. Activated calcineurin dephosphorylates the serine-rich region (SRR) and SP-repeats in the amino termini of NFAT proteins, resulting in a conformational change that exposes a nuclear localization signal, resulting in NFAT nuclear import.

In embodiments, the promoter responsive to NFAT consists of 2 to 12 repeats, preferably 6 repeats, of the 30 bp human NFAT binding sequence according to SEQ ID No. 65, optionally separated by spacer sequences, followed by a minimal promoter.

In embodiments, the minimal promoter is derived from the interleukin-2 (IL-2) promoter according to SEQ ID No. 66 or SEQ ID No. 67.

In embodiments, the promoter responsive to NFAT has SEQ ID No. 68 or SEQ ID No. 69.

In further embodiments, the nucleic acid, vector or cell according to the invention further comprises a nucleotide sequence encoding a switchable chimeric antigen receptor,
wherein the switchable chimeric antigen receptor comprises three domains,
wherein
   - the first domain is a tag-binding domain or tag,
   - the second domain is an extracellular hinge and a transmembrane domain and
   - the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

Another aspect of the invention is a nucleic acid, a vector or a cell comprising
- an inducible expression system, preferably selected from Tet-On, Tet-On Advanced, Tet-On 3G, T-REx or a promoter responsive to NFAT, and
- a nucleotide sequence encoding a switchable chimeric antigen receptor, wherein the switchable chimeric antigen receptor comprises three domains, wherein
   - the first domain is a tag-binding domain or tag,
   - the second domain is an extracellular hinge and a transmembrane domain and
   - the third domain is a signal transduction domain, and
- a nucleotide sequence encoding a targeting module according to the invention, wherein the tag-binding domain of the targeting module binds to the tag of the switchable chimeric antigen receptor or the tag of the targeting module binds to the tag-binding domain of the switchable chimeric antigen receptor.

In embodiments, the inducible expression system induces the transcription of the nucleotide sequence encoding a switchable chimeric antigen receptor and/or a nucleotide sequence encoding a targeting module according to the invention, preferably the inducible expression system induces the transcription of the nucleotide sequence encoding a targeting module according to the invention.

In embodiments, the nucleic acid, vector or cell is administered in combination with a vector or a cell comprising a nucleotide sequence encoding a switchable chimeric antigen receptor and a nucleic acid, a vector or a cell comprising a nucleotide sequence encoding a further targeting module, wherein the at least one further targeting module comprises at least one target cell-binding domain and a tag-binding domain or a tag, wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269, CD273, CD274, CD276, CD279, CD319, CD366 and CD371, interleukin receptors, especially preferred IL-8Rα, IL-8Rβ, IL-11 Rα, IL-11Rβ, IL13Ra1, CXCR4, c-Met, mesothelin, members of the epidermal growth factor receptor family and mutants thereof, especially preferred ErbB1, ErbB2, ErbB3, ErbB4 or mutants thereof, members of the tumor necrosis factor receptor superfamily, ephrins, ephrin receptors, especially preferred EphA1-10, EphA5 or EphB1-6; prostate specific antigens, preferably PSCA and PSMA; embryonic antigens, preferably CEA and fetal acethylcholine receptor; members of the vascular endothelia growth factorfamily, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands, wherein the targeting module according to the invention and the at least one further targeting module comprise identical tag-binding domains or tags.

The invention further comprises a pharmaceutical composition comprising the targeting module according to the invention, the nucleic acid, vector or cell according to the invention and a pharmaceutically acceptable thinner or carrier for use in a method for stimulating a chimeric antigen receptor-mediated immune response in a mammal, preferably for use in the treatment of cancer, infectious disease or autoimmune disease; wherein the pharmaceutical composition is administered in combination with a vector or a cell comprising a nucleotide sequence encoding a switchable chimeric antigen receptor, wherein the switchable chimeric antigen receptor comprises three domains, wherein
- the first domain is a tag-binding domain or a tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

The pharmaceutical composition is preferably administered parenterally, particularly preferred intravenously. In embodiments, the pharmaceutical composition is present in a form suitable for intravenous administration. Preferably, the pharmaceutical composition is a solution, emulsion or suspension.

In embodiments, the pharmaceutical composition is an injectable buffered solution comprising a concentration in the range of 1 ng/ml to 500 mg/ml of the targeting module, the nucleic acid, vector and/or cell according to the invention, preferably in the range of 50 µg/ml to 5 mg/ml.

The pharmaceutical composition comprises a pharmaceutically acceptable thinner (dilution agent) or carrier. In embodiments, the carrier is selected from water, an aqueous buffer solution, 0.9 % saline solution, 5 % glucose, 5 % xylitol, 0.3 % glycine solution, ringer solutions or amino acid solutions. In further embodiments, the aqueous buffer solution is selected from an aqueous histidine, sodium succinate, sodium citrate, sodium phosphate or potassium phosphate-buffered solution with a pH value in the range of pH 5.0 to pH 7.0. In embodiments, the aqueous buffer solution has a buffer concentration in the range of 1 mmol/l (mM) to 500 mM, preferably in the range of 5 mM to 20 mM, especially preferred in the range of 5 mM to 10 mM.

In embodiments, the carrier comprises sodium chloride, preferably in a concentration in the range of 1 mM to 300 mM, especially preferred 150 mM.

In embodiments, the pharmaceutical composition further comprises a stabilizer, preferably with a concentration in the range of 1 mM to 900 mM, especially preferred in the range of 50 mM and 600 mM. In embodiments, the stabilizer is sucrose, trehalose or L-methionine.

In some embodiments, the pharmaceutical composition further comprises pharmaceutically acceptable excipients. The term "pharmaceutically acceptable excipients" refers to compounds, which provide approximately physiological conditions and/or increase the stability, such as agents for adjusting the pH value and buffering agents, agents for adjusting the toxicity and the like. In embodiments, pharmaceutically acceptable excipients are selected from sodium acetate, sodium chloride, potassium chloride, calcium chloride, sodium lactate and polysorbate-80, preferably polysorbate-80 in the range of 0.0001 % (w/v) to 1 % (w/v), especially preferred in the range of 0.001 % (w/v) to 0.1 % (w/v).

In preferred embodiments, the pharmaceutical composition comprises the targeting module in a dosage quantity in the range of 25 µg/day to 100 mg/day, preferably dosage quantities in the range of 0.1 mg/day to 20 mg/day.

In further embodiments, the pharmaceutical composition is sterile. The pharmaceutical composition is sterilized by conventional well-known techniques including, but not limited to, sterile filtration.

In embodiments, the pharmaceutical composition is used for administration to a subject.

In embodiments, the pharmaceutical composition is lyophilized prior to storage or stored as solution at ambient temperature or below, including, but not limited to, frozen storage.

In embodiments, the pharmaceutical composition is reconstituted and/or diluted in an infusion and stabilizer solution prior to administration to a subject. The solutions used for reconstitution or infusion/stabilization may contain any of the components mentioned for the pharmaceutical composition or similar components.

In embodiments, the pharmaceutical composition comprises at least one further targeting module or at least one further nucleic acid, vector or cell encoding a further targeting module, wherein the at least one further targeting module comprises at least one target cell-binding domain and a tag-binding domain or a tag, wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269, CD273, CD274, CD276, CD279, CD319, CD366 and CD371, interleukin receptors, especially preferred IL-8Rα, IL-8Rβ, IL-11Rα, IL-11Rβ, IL13Ra1, CXCR4, c-Met, mesothelin, members of the epidermal growth factor receptor family and mutants thereof, especially preferred ErbB1, ErbB2, ErbB3, ErbB4 or mutants thereof, members of the tumor necrosis factor receptor superfamily, ephrins, ephrin receptors, especially preferred EphA1-10, EphA5 or EphB1-6; prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands, wherein the targeting module and the at least one further targeting module comprise identical tag-binding domains or tags.

Another aspect of the invention is a kit comprising
a) a targeting module according to the invention, the nucleic acid, vector or cell according to the invention and
b) a vector or a cell comprising a nucleotide sequence encoding a switchable chimeric antigen receptor,
   wherein the switchable chimeric antigen receptor comprises three domains, wherein
      - the first domain is a tag-binding domain or tag,
      - the second domain is an extracellular hinge and a transmembrane domain and
      - the third domain is a signal transduction domain,
   wherein the tag-binding domain of the targeting module binds to the tag of the switchable chimeric antigen receptor or the tag of the targeting module binds to the tag-binding domain of the switchable chimeric antigen receptor.

In embodiments, the tag is a peptide epitope tag. In further embodiments, the tag comprises 10 to 20 amino acids.

In embodiments, the peptide epitope tag is a myc-tag, a His-tag, a short linear peptide sequence from yeast transcription factor GCN4, preferably according to SEQ ID No. 39, SEQ ID No. 40 or mutants thereof; a leucine zipper sequence, preferably SYNZIP 1 to SYNZIP 48, BATF, FOS, ATF4, ATF3, BACH1, JUND, NFE2L3, HEPTAD (Reinke *et al.* 2010), a sequence according to SEQ ID No. 41 or SEQ ID No. 42 or mutants thereof; or a short linear peptide sequence from a human nuclear protein, preferably from the human La protein.

In preferred embodiments, the peptide epitope tag is a myc-tag, a His-tag, a short linear peptide sequence from yeast transcription factor GCN4, preferably according to SEQ ID No. 39, SEQ ID No. 40; a leucine zipper sequence, preferably SYNZIP 1 to SYNZIP 48, BATF, FOS, ATF4, ATF3, BACH1, JUND, NFE2L3, HEPTAD (Reinke *et al.* 2010), a sequence according to SEQ ID No. 41 or SEQ ID No. 42; or a short linear peptide sequence from a human nuclear protein, preferably from the human La protein.

Preferably, the peptide epitope tag from the human La protein is the human La epitope E5B9 according to SEQ ID No. 43 or E7B6 according to SEQ ID No. 44 or SEQ ID No. 45, most preferably the human La epitope E5B9 according to SEQ ID No. 43 or E7B6 according to SEQ ID No. 45.

In embodiments, the His-tag is an amino acid sequence consisting of histidine residues, preferably in the range of six to fourteen histidine residues.

In embodiments, the tag-binding domain is an antibody or an antibody fragment.

In embodiments, the tag-binding domain is an antibody or an antibody fragment binding to a myc-tag, a His-tag, a short linear peptide sequence from yeast transcription factor GCN4, a leucine zipper sequence or a short linear peptide sequence from a human nuclear protein, preferably from a human La protein.

In embodiments, the tag-binding domain binds to a tag from the human nuclear La protein; preferably, the tag-binding domain is an antibody or an antigen-binding fragment, comprising a V_{L} according to the following sequence:
DIVMTQSPDSLAVSLGERATINCX₂₄SSQSLLNSRTX₃₅KNYLAWYQQKPGQPPKLLIYWASTR X₆₁ SGVPDRFSGSGSGTDFTLTISSLQAEDVAVYYCKQSYNLX₁₀₁TFGGGTKVEIK (SEQ ID No. 46), wherein X₂₄, X₃₅, X₆₁ and X₁₀₁ are independently from each other selected from a proteinogenic alpha-amino acid residue; or a sequence having at least 85 % sequence identity, preferably at least 95 % sequence identity; to one of the sequences SEQ ID No. 48 or SEQ ID No. 50.

In embodiments, the tag-binding domain constitutes an anti-La epitope scFv.

In some embodiments, X₂₄ to X₁₀₁ are selected as follows:
X₂₄ is selected from polar and/or positive charged residues, such as Serine, Threonine, Asparagine, Glutamine, Histidine, Lysine and Arginine; preferably Lysine or Arginine;
X₃₅ is preferably selected from Lysine and Proline;
X₆₁ is selected from polar and charged residues, such as Asparagine, Aspartic Acid, Glutamine, Glutamic acid, Histidine, Lysine and Arginine, preferably Glutamic acid and Lysine;
X₁₀₁ is selected from hydrophobic residues, such as Isoleucine, Leucine, Valine, Alanine, Methionine, Phenylalanine, Proline and Tryptophan; preferably Leucine or Proline.

In further embodiments, the tag-binding domain is an antibody or an antigen-binding fragment comprising a sequence having at least 85 % sequence identity, preferably at least 95 %sequence identity; to one of the sequences SEQ ID No. 47 or SEQ ID No. 49.

Preferably, the tag-binding domain comprises a sequence having each at least 85 % sequence identity, preferably at least 95 % sequence identity; to the sequences according to SEQ ID No. 47 (V_{H}) and SEQ ID No. 48 (V_{L}) or the sequences according to SEQ ID No. 49 (V_{H}) and SEQ ID No. 50 (V_{L}).

Especially preferred, the tag-binding domain constitutes the anti-La 5B9 scFv according to SEQ ID No. 47 (V_{H}) and SEQ ID No. 48 (V_{L}) or the anti-La 7B6 scFv according to SEQ ID No. 49 (V_{H}) and SEQ ID No. 50 (V_{L}).

As used herein, the term "extracellular hinge and a transmembrane domain" refers to a flexible peptide sequence connected to the tag-binding domain or tag, which anchors the switchable CAR into the cell membrane of the cell and protrudes from the surface of the cell for optimal binding to its particular targeting module.

In embodiments, the extracellular hinge and transmembrane domain are selected from hinge and transmembrane domains of human CD28 molecule, CD8a chain, NK cell receptors, preferably natural killer group NKG2D; or parts of the constant region of an antibody and combinations thereof. As used herein, the term "combinations thereof" refers to combinations of the different hinge and transmembrane domains.

Pinthus *et al.* and Cartellieri *et al.* describe the use of hinge and transmembrane domains of the human CD28 molecule in CARs (Pinthus *et al.* 2003, Cartellieri *et al.* 2016).

Milone *et al.* and Zhao *et al.* describe the use of hinge and transmembrane domains of human CD8a molecule in CARs (Milone *et al.* 2009, Zhao *et al.* 2009).

Zhang *et al.* describe the use of hinge and transmembrane domains of NKG2D in CARs (Zhang *et al.* 2005).

Frigault *et al.* and Wang *et al.* describe the use of hinge and transmembrane domains of parts of the constant region of immunoglobulin G1 (IgG) (Frigault *et al.* 2015, Wang *et al.* 2007). Frigault *et al.* describes the use of hinge domains of the constant region of IgG4.

Examples of combinations of the extracellular hinge and transmembrane domain are, but are not limited to, CD28 extracellular hinge and transmembrane domain, CD8alpha extracellular hinge and transmembrane domain, IgG1 or IgG4 constant regions combined with CD28 or CD137 transmembrane domain.

As used herein, the term "signal transduction domain" refers to a peptide sequence which transmits a signal into the cell by cross-linkage of the cell expressing the switchable CAR (effector cell) to a human cell surface protein or protein complex (target cell). Cross-linkage between effector and target cell is mediated by the targeting module according to the invention.

In embodiments, the signal transduction domain is selected from cytoplasmic regions of CD28, CD137 (4-1BB), CD134 (OX40), CD278 (ICOS), DAP10 and CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor α), CD360 (interleukin-21 receptor) activating Fc receptors and mutants thereof.

As used herein, the term "mutants" refers to proteins having at least 85 % sequence identity to the signal transduction domains, preferably at least 95 % sequence identity. Advantageously, the mutant transmits a signal into the cell by cross-linkage of the cell expressing the switchable CAR (effector cell) to a human cell surface protein or protein complex (target cell) in the same way as the named signal transduction domains.

In embodiments, mutants are truncated versions. As used herein, the term "truncated versions" refers to shortened proteins having at least 85 % sequence identity to the signal transduction domains, preferably at least 95 % sequence identity, more preferably having a chain length of at least 85 % and a sequence identity of 100 %, most preferably a chain length of at least 95 % and a sequence identity of 100 %. Advantageously, the truncated version has an activity of at least 80 %, preferably of at least 90 %, more preferably of at least 95 %; of the named signal transduction domains.

Hombach *et al.* and Cartellieri *et al.* describe the use of cytoplasmic regions of CD28 as signal transduction domain in CARs (Hombach *et al.* 2001, Cartellieri *et al.* 2016). Guedan *et al.* describes the use of a mutant of cytoplasmic regions of CD28 as signal transduction domain (Guedan *et al.* 2020).

Milone *et al.* and Finney *et al.* describe the use of cytoplasmic regions of CD137 (4-1 BB) as signal transduction domain (Finney *et al.* 2004, Milone *et al.* 2009).

Finney *et al.* and Hombach and Abken describe the use of cytoplasmic regions of CD134 (OX40) as signal transduction domain in CARs (Finney *et al.* 2004, Hombach and Abken 2011).

Guedan *et al.* describes the use of cytoplasmic regions of CD278 (ICOS) as signal transduction domain (Guedan *et al.* 2018).

Zhang *et al.* describes the use of DAP10 as signal transduction domain (Zhang *et al.* 2005).

Fedorov *et al.* describes the use of programmed cell death 1 (PD-1) and of cytotoxic T-lymphocyte antigen 4 (CTLA-4) as signal transduction domain in CARs (Fedorov *et al.* 2013).

Gong *et al.* and Gade *et al.* describe the use of cytoplasmic regions of CD3 chains, in particular the CD3ζ chain, as signal transduction domain in CARs (Gong *et al.* 1999, Gade *et al.* 2005).

Töpfer *et al.* describes the use of DAP12 as signal transduction domain in CARs (Töpfer *et al.* 2015).

Kagoya *et al.* describes the use of signaling chains or motifs derived from interleukin receptors as signal transduction domain in CARs (Kagoya *et al.* 2018).

Lamers *et al.* and Kershaw *et al.* describe the use of activating Fc receptors, in particular the Fc epsilon receptor γ chain, as signal transduction domain (Lamers *et al.* 2004, Kershaw *et al.* 2006).

In preferred embodiments, the signal transduction domain is selected from cytoplasmic regions of CD28, CD137 (4-1BB), CD134 (OX40), CD278 (ICOS), DAP10 and CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor α) and CD360 (interleukin-21 receptor) activating Fc receptors.

In embodiments, the switchable chimeric antigen receptor comprises at least one signal transduction domain, preferably two, three, four or more signal transduction domains, especially preferably selected from cytoplasmic regions of CD28, CD137, CD134, CD278, DAP10 and CD27, PD-1, CTLA-4, cytoplasmic regions of CD3 chains, DAP12, CD122, CD132, CD127, CD360, activating Fc receptors and mutants thereof.

In preferred embodiments, the switchable chimeric antigen receptor comprises at least one signal transduction domain, preferably two, three, four or more signal transduction domains, especially preferably selected from cytoplasmic regions of CD28, CD137, CD134, CD278, DAP10 and CD27, PD-1, CTLA-4, cytoplasmic regions of CD3 chains, DAP12, CD122, CD132, CD127, CD360 and activating Fc receptors.

In further embodiments, the switchable chimeric antigen receptor comprises a fourth domain, wherein the fourth domain is a short peptide linker in the extracellular portion of the receptor that may serve to detect the chimeric antigen receptor on the cell surface or stimulate the chimeric antigen receptor T cell.

In further embodiments, the fourth domain forms a linear epitope for a monoclonal antibody (mab) specifically binding to the fourth domain. In some embodiments, the fourth domain comprises at least one linear epitope, preferably E7B6 according to SEQ ID No. 44 or SEQ ID No. 45.

In some embodiments, the fourth domain is located in between the tag-binding domain or the tag and the extracellular hinge domain or an integral part of the extracellular hinge domain.

Advantageously, the switchable CAR engrafted cells with the fourth domain can be specifically stimulated to proliferate preferentially and persist longer compared to non-engrafted cells either *in vitro* or *in vivo.* Further advantageously, the fourth domain may be also used to purify switchable CAR engrafted cells from mixed cell populations or to dampen switchable CAR engrafted cell-mediated immune response and to eliminate switchable CAR engrafted cells *in vivo.*

In further embodiments, the switchable CAR comprises a signal peptide. Advantageously, the signal peptide allows for expression on the cell surface of an effector cell. In embodiments, the signal peptide is located at the N-terminus of the switchable CAR nucleotide sequence in front of the tag-binding domain or the tag. In some embodiments, the signal peptide targets proteins to the secretory pathway either co-translationally or post-translationally and is selected from leader peptides from proteins like CD28, CD8alpha, IL-2, lysozyme C or the heavy or light chains of antibodies of human origin to avoid immunogenic reactions.

In embodiments, the nucleic acid is a cDNA.

In embodiments, the tag-binding domain or tag is present at the amino-terminal end of the polypeptide that comprises the switchable CAR. Advantageously, locating the tag-binding domain or tag at the amino terminus permits the tag-binding domain or tag unhampered access to the targeting module that is bound to the target cell.

In embodiments, the nucleic acid encodes a switchable CAR according to one of the sequences SEQ ID No. 70 to SEQ ID No. 77. Preferably, the nucleic acid is one of the sequences SEQ ID No. 78 to SEQ ID No. 85.

In some embodiments, the cell comprising a nucleotide sequence encoding a switchable chimeric antigen receptor is selected from immune cells, preferably with cytolytic, phagocytic or immunosuppressive activity, such as T cells, Natural Killer (NK) cells and macrophages. In preferred embodiments, the cell is selected from T cells, including alpha/beta and gamma/delta T cells or subpopulations of T cells like stem-cell memory T cells or central memory T cells, cytotoxic T cells; or NK cells.

In embodiments, the kit further comprises at least one further targeting module or at least one further nucleic acid, vector or cell encoding a further targeting module, wherein the at least one further targeting module comprises at least one target cell-binding domain and a tag-binding domain or a tag, wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269, CD273, CD274, CD276, CD279, CD319, CD366 and CD371, interleukin receptors, especially preferred IL-8Rα, IL-8Rβ, IL-11Rα, IL-11Rβ, IL13Ra1, CXCR4, c-Met, mesothelin, members of the epidermal growth factor receptor family and mutants thereof, especially preferred ErbB1, ErbB2, ErbB3, ErbB4 or mutants thereof, members of the tumor necrosis factor receptor superfamily, ephrins, ephrin receptors, especially preferred EphA1-10, EphA5 or EphB1-6, prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands, wherein the targeting module and the at least one further targeting module comprise identical tag-binding domains or tags.

In embodiments, the kit comprises
- a nucleic acid, vector or cell comprising a nucleotide sequence encoding a targeting module according to the invention and
- a vector or a cell comprising a nucleotide sequence encoding a switchable chimeric antigen receptor,
   wherein the switchable chimeric antigen receptor comprises three domains, wherein
   - the first domain is a tag-binding domain or tag,
   - the second domain is an extracellular hinge and a transmembrane domain and
   - the third domain is a signal transduction domain,
- at least one further targeting module,
wherein the tag-binding domain of the targeting module binds to the tag of the switchable chimeric antigen receptor or the tag of the targeting module binds to the tag-binding domain of the switchable chimeric antigen receptor,
wherein the targeting module according to the invention and the at least one further targeting module comprise different target cell-binding domains, and identical tag-binding domains or tags.

In preferred embodiments, the at least one further targeting module comprises at least one target cell-binding domain and a tag-binding domain or a tag, wherein the at least one target cell-binding domain is an antibody, antibody fragment, a protein, a peptide or a low molecular weight organic ligand that binds to HER2, PDL1, NKG2D, EGFR or EGFRvIII, wherein the targeting module according to the invention and the at least one further targeting module comprise identical tag-binding domains or tags.

In embodiments, the kit comprises one to three targeting modules, preferably one targeting module according to the invention and one or two further targeting modules.

In embodiments, the kit further comprises an inducer, preferably tetracycline or a derivative of tetracycline, e.g. doxycycline.

In embodiments of the kit, the targeting module, the vector and/or the cell comprising a nucleotide sequence encoding a switchable chimeric antigen receptor are in the form of a pharmaceutical composition.

Another aspect of the invention is the kit according to the invention for use in a method for stimulating a chimeric antigen receptor-mediated immune response in a mammal, preferably for use in the treatment of cancer, infectious disease or autoimmune disease.

In embodiments, the targeting module according to the invention, the nucleic acid, vector or cell according to the invention or the kit according to the invention is used for preparing a medication for therapeutic and/or diagnostic use in case of cancer, an infection or an autoimmune disease.

In embodiments, the targeting module according to the invention, the nucleic acid, vector or cell according to the invention or the kit according to the invention is used for treating IL13RA2- and/or HER2-expressing tumors, preferably glioblastoma, breast cancer and/or melanoma.

A further aspect of the invention is a method for stimulating a chimeric antigen receptor-mediated immune response in a mammal, preferably a human, having cancer, an infectious or an autoimmune disease by administration of a targeting module, a cell or a vector, a pharmaceutical composition or a kit according to the invention. For therapeutic applications, a sterile pharmaceutical composition according to the invention or a sterile kit according to the invention, comprising a pharmacologically effective quantity of targeting module according to the invention and a vector or a cell comprising a nucleotide sequence encoding a switchable chimeric antigen receptor, is administered to a subject in order to treat the aforementioned illnesses.

In embodiments, the method for stimulating a chimeric antigen receptor-mediated immune response in a mammal; preferably, the method for treatment of cancer, infectious or autoimmune disease, further comprises the administration of an inducer, preferably tetracycline or a derivative of tetracycline, e.g. doxycycline.

In embodiments, an inducer, preferably tetracycline or a derivative of tetracycline, e.g. doxycycline; is administered orally or intravenously following administration of the nucleic acid, vector or cell according to the invention.

In some embodiments, the method for stimulating a chimeric antigen receptor-mediated immune response in a mammal; preferably, the method for treatment of cancer, infectious or autoimmune disease, comprises the following steps:
a) administering to a mammal an effective amount of a targeting module according to the invention and
b) administering to the mammal an effective amount of a vector or a cell comprising a nucleotide sequence encoding a switchable chimeric antigen receptor,
   wherein the switchable chimeric antigen receptor comprises three domains, wherein
      - the first domain is a tag-binding domain or tag,
      - the second domain is an extracellular hinge and a transmembrane domain and
      - the third domain is a signal transduction domain,
   wherein the tag-binding domain of the targeting module binds to the tag of the switchable chimeric antigen receptor or the tag of the targeting module binds to the
   tag-binding domain of the switchable chimeric antigen receptor,
wherein the targeting module is administered to a mammal prior to, concurrent with or after the administration of the vector or cell.

In further embodiments, the method for stimulating a chimeric antigen receptor-mediated immune response in a mammal; preferably, the method for treatment of cancer, infectious or autoimmune disease, comprises the following steps:
a) administering to a mammal an effective amount of a targeting module comprising at least one target cell-binding domain and a tag-binding domain or a tag, wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269 (BCMA), CD273, CD274, CD276, CD279, CD319, CD366 and CD371, interleukin receptors, especially preferred IL-8Rα, IL-8Rβ, IL-11Rα, IL-11Rβ, IL13Ra1, CXCR4, c-Met, mesothelin, members of the epidermal growth factor receptor family and mutants thereof, especially preferred ErbB1, ErbB2, ErbB3, ErbB4 or mutants thereof, members of the tumor necrosis factor receptor superfamily, ephrins, ephrin receptors, especially preferred EphA1-10, EphA5 or EphB1-6, prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands, and
b) administering to the mammal an effective amount of a vector or a cell comprising a nucleotide sequence encoding a switchable chimeric antigen receptor,
   wherein the switchable chimeric antigen receptor comprises three domains, wherein
      - the first domain is a tag-binding domain or tag,
      - the second domain is an extracellular hinge and a transmembrane domain and
      - the third domain is a signal transduction domain,
   wherein the tag-binding domain of the targeting module binds to the tag of the switchable chimeric antigen receptor or the tag of the targeting module binds to the tag-binding domain of the switchable chimeric antigen receptor, and
c) administering to a mammal an effective amount of a targeting module according to the invention,
wherein the targeting module according to the invention and the at least one further targeting module comprise different target cell-binding domains, and identical tag-binding domains or tags, wherein the method is carried out in the order of the steps a), b) and c).

In preferred embodiments, the further targeting module is administered on its own, preferably one hour to 2 days, more preferably 4 to 24 hours, prior to the administration of the vector or cell comprising a nucleotide sequence encoding a switchable chimeric antigen receptor and the targeting module according to the invention is administered until, preferably in the range of 3 days to 30 days, after the administration of the vector or cell comprising a nucleotide sequence encoding a switchable chimeric antigen receptor. Furthermore, additional such doses of the targeting module may be administered following resting periods to reactivate the switchable chimeric antigen receptor-carrying effector cells.

In further embodiments, the method for stimulating a chimeric antigen receptor-mediated immune response in a mammal, preferably the method for the treatment of cancer, infectious or autoimmune disease, comprises the following steps:
a) administering to a mammal an effective amount of a vector or a cell comprising an inducible expression system, preferably selected from Tet-On, Tet-On Advanced transactivator, Tet-On 3G or a promoter responsive to NFAT; and a nucleotide sequence encoding a switchable chimeric antigen receptor, wherein the switchable chimeric antigen receptor comprises three domains, wherein
   - the first domain is a tag-binding domain or tag,
   - the second domain is an extracellular hinge and a transmembrane domain and
   - the third domain is a signal transduction domain, and
   a nucleotide sequence encoding a targeting module according to the invention, wherein the tag-binding domain of the targeting module binds to the tag of the switchable chimeric antigen receptor or the tag of the targeting module binds to the tag-binding domain of the switchable chimeric antigen receptor, and
b) administering to a mammal an effective amount of an inducer, preferably tetracycline or a derivative of tetracycline, e.g. doxycycline.

In embodiments, the inducible expression system induces the transcription of the nucleotide sequence encoding a switchable chimeric antigen receptor and/or a nucleotide sequence encoding a targeting module according to the invention, preferably the inducible expression system induces the transcription of the nucleotide sequence encoding a targeting module according to the invention.

In embodiments, the method for stimulating a chimeric antigen receptor-mediated immune response in a mammal, preferably the method for treatment of cancer, infectious or autoimmune disease, comprises the following steps:
a) administering to a mammal an effective amount of a targeting module comprising at least one target cell-binding domain and a tag-binding domain or a tag,
   wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6CD52, CD90, CD99, CD123, CD133, CD150 CD181, CD182, CD184, CD223, CD229, CD269 (BCMA), CD273, CD274, CD276, CD279, CD319, CD366 and CD371, interleukin receptors, especially preferred IL-8Rα, IL-8Rβ, IL-11Rα, IL-11Rβ, IL13Ra1, CXCR4, c-Met, mesothelin, members of the epidermal growth factor receptor family, especially preferred ErbB1, ErbB2, ErbB3 or ErbB4; members of the tumor necrosis factor receptor superfamily, ephrins, ephrin receptors, especially preferred EphA1-10, EphA5 or EphB1-6; prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, preferably the at least one target cell-binding domain is an antibody or an antibody fragment that binds to surface antigens selected from the group comprising HER2, PD-L1, NKG2D, EGFR and EGFRvIII, especially preferred binding to HER2, and
b) administering to a mammal an effective amount of a vector or a cell comprising an inducible expression system, preferably selected from Tet-On, Tet-On Advanced transactivator, Tet-On 3G or a promoter responsive to NFAT; and a nucleotide sequence encoding a switchable chimeric antigen receptor, wherein the switchable chimeric antigen receptor comprises three domains, wherein
   - the first domain is a tag-binding domain or tag,
   - the second domain is an extracellular hinge and a transmembrane domain and
   - the third domain is a signal transduction domain, and
   a nucleotide sequence encoding a targeting module according to the invention, wherein the tag-binding domain of the targeting module binds to the tag of the switchable chimeric antigen receptor or the tag of the targeting module binds to the tag-binding domain of the switchable chimeric antigen receptor and,
c) administering to a mammal an effective amount of an inducer, preferably tetracycline or a derivative of tetracycline, e.g. doxycycline,
wherein the targeting module according to the invention and the at least one further targeting module comprise different target cell-binding domains, and identical tag-binding domains or tags, and wherein the method is carried out preferably in the order of the steps a), b) and c).

Another aspect of the invention is an IL13Ra2 binding IL13 mutein according to SEQ ID No. 2 (IL13-E11Y-G30Y) or according to SEQ ID No. 3 (IL13-E11Y-M32A).

In embodiments, the mutein according to the invention is used for imaging and/or diagnosis of cancer, in particular by conjugation of the mutein with a fluorescent dye, enzyme or radioactive label.

In embodiments, the mutein according to the invention is used in the treatment of cancer, infectious disease or autoimmune disease.

In embodiments, the mutein is used as mono-, bi- or multispecific binding moiety in a CAR construct, as binding domain in targeting module for a switchable CAR construct or mutein-drug conjugate.

In embodiments, the mutein according to the invention is used in a method for stimulating a chimeric antigen receptor-mediated immune response in a mammal.

Another aspect of the invention is a nucleic acid, a vector or a cell comprising a nucleotide sequence encoding an IL13Ra2-binding IL13 mutein according to SEQ ID No. 2 or according to SEQ ID No. 3.

A further aspect of the invention is a pharmaceutical composition comprising an IL13Rα2-binding IL13 mutein according to SEQ ID No. 2 or according to SEQ ID No. 3 and a pharmaceutically acceptable thinner or carrier.

Another aspect of the invention is an IL13Rα2-binding antibody or antibody fragment, wherein the V_{L} comprises the amino acid CDR sequences SEQ ID No. 11, SEQ ID No. 12 and SEQ ID No. 13, and the V_{H} comprises the amino acid CDR sequences SEQ ID No. 14, SEQ ID No. 15 and SEQ ID No. 16.

In embodiments, the variable regions of the antibody or antibody fragment comprise a humanized amino acid sequence.

In embodiments, the antibody or antibody fragment comprises a V_{L} according to SEQ ID No. 17, wherein X₁, X₂, X₄, X₁₀, X₁₃, X₁₄, X₁₈, X₁₉, X₂₁, X₂₂, X₂₄, X₄₁, X₆₀, X₇₃, X₇₇, X₇₈, X₈₀, X₈₃, X₈₄, X₈₅ and X₈₇ are independently from each other selected from a proteinogenic alpha-amino acid residue, and/or a V_{H} according to SEQ ID No. 18, wherein X₁₂, X₂₀, X₃₈, X₄₈, X₆₁, X₆₂, X₆₅, X₆₆, X₆₈, X₇₀, X₇₂, X₇₄, X₇₅, X₇₆, X₇₉, X₈₃, X₈₅, X₈₉, X₁₁₀ and X₁₁₄ are independently from each other selected from a proteinogenic alpha-amino acid residue.

In embodiments, the antibody or antibody fragment comprising a V_{L} according to one of the sequences SEQ ID No. 19 to SEQ ID No. 24 and/or a V_{H} according to one of the sequences SEQ ID No. 25 to SEQ ID No. 30.

In embodiments, X₁ is D or Q, X₂ is I or V, X₄ is L or M, X₁₀ is F or T, X₁₃ is V or L, X₁₄ is T or S, X₁₈ is K or R, X₁₉ is V or A, X₂₁ is L or I, X₂₂ is T or S, X₂₄ is R or Q, X₄₁ is D or G, X₆₀ is S or D, X₇₃ is L or F, X₇₇ is S or R, X₇₈ is V or L, X₆₀ is A or P, X₈₃ is F or A, X₈₄ is G or A, X₈₅ is T or V and/or X₈₇ is Y or F.

In embodiments, X₁₂ is V or K, X₂₀ is L or V, X₃₈ is K or R, X₄₈ is I or M, X₆₁ is A or N, X₆₂ is Q or E, X₆₅ is Q or K, X₆₆ is G or T, X₆₈ is A or V, X₇₀ is L or M, X₇₂ is V or R, X₇₄ is T or K, X₇₅ is S or P, X₇₆ is I or S or T, X₇₉ is A or V, X₈₃ is I or R, X₆₈ is R or S, X₈₉ is D or E, X₁₁₀ is Q or A, X₁₁₄ is L or V.

In preferred embodiments, the antibody or antibody fragment comprises one of the sequences according to SEQ ID No. 5 to SEQ ID No. 10. Preferably, the antibody or antigen-binding fragment is one of the sequences according to SEQ ID No. 5 to SEQ ID No. 10.

In embodiments, the antibody or antibody fragment according to the invention is used for imaging and/or diagnosis of cancer, in particular by conjugation of the antibody or antibody fragment with a fluorescent dye, enzyme or radioactive label.

In embodiments, the antibody or antibody fragment is used in the treatment of cancer, infectious disease or autoimmune disease.

In embodiments, the antibody or antibody fragment is used as an antibody, bi- or multispecific antibody, as binding domain in a CAR construct, as binding domain in targeting module for a switchable CAR construct or antibody-drug conjugate.

In embodiments, the antibody or antibody fragment is used in a method for stimulating a chimeric antigen receptor-mediated immune response in a mammal.

A further aspect of the invention is a nucleic acid, a vector or a cell comprising a nucleotide sequence encoding an antibody or antibody fragment according to the invention.

A further aspect of the invention is a pharmaceutical composition comprising an antibody or antibody fragment according to the invention and a pharmaceutically acceptable thinner or carrier.

A further aspect of the invention is a targeting module comprising
i) at least one HER2-binding domain,
ii) a tag-binding domain or a tag,
for use in a method for stimulating a chimeric antigen receptor-mediated immune response in a mammal, wherein the targeting module is administered in combination with a vector or a cell comprising a nucleotide sequence encoding a switchable chimeric antigen receptor, wherein the switchable chimeric antigen receptor comprises three domains, wherein
- the first domain is a tag-binding domain or a tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

In embodiments, the at least one HER2-binding domain is Trastuzumab (hu4D5), a Trastuzumab mutant or a fragment thereof, preferably comprising SEQ ID No. 37 and SEQ ID No. 38.

In embodiments, the HER2-binding domain is a Trastuzumab mutant, wherein the one amino acid in the light chain is mutated, in particular Y55E, and/or one amino acid in the heavy chain is mutated, in particular V109Y.

In embodiments, the tag is selected from organic molecules including fluorescence labels, e.g. FITC (Fluorescein isothiocyanate), and biotin.

In embodiments, the tag is a peptide epitope tag. In further embodiments, the tag comprises 10 to 20 amino acids.

In embodiments, the peptide epitope tag is a myc-tag, a His-tag, a short linear peptide sequence from yeast transcription factor GCN4, preferably according to SEQ ID No. 39, SEQ ID No. 40 or mutants thereof; a leucine zipper sequence, preferably SYNZIP 1 to SYNZIP 48, BATF, FOS, ATF4, ATF3, BACH1, JUND, NFE2L3, HEPTAD (Reinke *et al.* 2010), a sequence according to SEQ ID No. 41 or SEQ ID No. 42 or mutants thereof; or a short linear peptide sequence from a human nuclear protein, preferably from the human La protein.

In preferred embodiments, the peptide epitope tag is a myc-tag, a His-tag, a short linear peptide sequence from yeast transcription factor GCN4, preferably according to SEQ ID No. 39, SEQ ID No. 40; a leucine zipper sequence, preferably SYNZIP 1 to SYNZIP 48, BATF, FOS, ATF4, ATF3, BACH1, JUND, NFE2L3, HEPTAD (Reinke *et al.* 2010), a sequence according to SEQ ID No. 41 or SEQ ID No. 42; or a short linear peptide sequence from a human nuclear protein, preferably from the human La protein.

Preferably, the peptide epitope tag from the human La protein is the human La epitope E5B9 according to SEQ ID No. 43 or E7B6 according to SEQ ID No. 44 or SEQ ID No. 45, most preferably the human La epitope E5B9 according to SEQ ID No. 43 or E7B6 according to SEQ ID No. 45.

In embodiments, the His-tag is an amino acid sequence consisting of histidine residues, preferably in the range of six to fourteen histidine residues.

In embodiments, the tag-binding domain is an antibody or an antibody fragment.

In embodiments, the tag-binding domain is an antibody or an antibody fragment binding to a myc-tag, a His-tag, a short linear peptide sequence from yeast transcription factor GCN4, a leucine zipper sequence or a short linear peptide sequence from a human nuclear protein.

In embodiments, the tag-binding domain binds to a tag from the human nuclear La protein; preferably, the tag-binding domain is an antibody or an antigen-binding fragment, comprising a V_{L} according to the following sequence:
DIVMTQSPDSLAVSLGERATINCX₂₄SSQSLLNSRTX₃₅KNYLAWYQQKPGQPPKLLIYWASTR X₆₁SGVPDRFSGSGSGTDFTLTISSLQAEDVAVYYCKQSYNLX₁₀₁TFGGGTKVEIK (SEQ ID No. 46), wherein X₂₄, X₃₅, X₆₁ and X₁₀₁ are independently from each other selected from a proteinogenic alpha-amino acid residue;
or a sequence having at least 85 % sequence identity, preferably at least 95 % sequence identity; to one of the sequences SEQ ID No. 48 or SEQ ID No. 50.

In embodiments, the tag-binding domain constitutes an anti-La epitope scFv.

In some embodiments, X₂₄ to X₁₀₁ are selected as follows:
X₂₄ is selected from polar and/or positive charged residues, such as Serine, Threonine, Asparagine, Glutamine, Histidine, Lysine and Arginine; preferably Lysine or Arginine;
X₃₅ is preferably selected from Lysine and Proline;
X₆₁ is selected from polar and charged residues, such as Asparagine, Aspartic Acid, Glutamine, Glutamic acid, Histidine, Lysine and Arginine, preferably Glutamic acid and Lysine;
X₁₀₁ is selected from hydrophobic residues, such as Isoleucine, Leucine, Valine, Alanine, Methionine, Phenylalanine, Proline and Tryptophan; preferably Leucine or Proline.

In further embodiments, the tag-binding domain is an antibody or an antigen-binding fragment comprising a sequence having at least 85 % sequence identity, preferably at least 95 % sequence identity; to one of the sequences SEQ ID No. 47 or SEQ ID No. 49.

Preferably, the tag-binding domain comprises a sequence having each at least 85 % sequence identity, preferably at least 95 % sequence identity; to the sequences according to SEQ ID NO. 47 (V_{H}) and SEQ ID NO. 48 (V_{L}) or the sequences according to SEQ ID NO. 49 (V_{H}) and SEQ ID NO. 50 (V_{L}).

Most preferably, the tag-binding domain constitutes the anti-La 5B9 scFv according to SEQ ID NO. 47 (V_{H}) and SEQ ID NO. 48 (V_{L}) or the anti-La 7B6 scFv according to SEQ ID NO. 49 (V_{H}) and SEQ ID NO. 50 (V_{L}).

In embodiments, the targeting module comprising at least one HER2-binding domain for use in a method for stimulating a chimeric antigen receptor-mediated immune response in a mammal further comprises an effector cell-binding domain, wherein the effector cell-binding domain specifically binds an epitope on a human CD3 chain, a human T cell receptor (TCR) or human CD16. Preferably, the effector cell-binding domain is an antibody or antibody fragment.

In embodiments, the effector cell-binding domain specifically binds an epitope on human CD3 with a dissociation constant K_{d} up to 10⁻⁸ M. Commonly, the affinity of the effector cell-binding domain, in particular the dissociation constant K_{d}, is determined with surface plasmon resonance measurements or flow-cytometry-based cellular binding assay.

Preferably, the effector cell-binding domain specifically binds an epitope on human CD3 with a low affinity, preferably with a dissociation constant K_{d} in the range of 10⁻⁴ M to 10⁻⁸ M.

In embodiments, the effector cell-binding domain comprises at least one of the following sequences:
i) a V_{L} according to SEQ ID No. 51 or 53 and/or
ii) a V_{H} according to SEQ ID No. 52 or 54.

In embodiments, the effector cell-binding domain comprises at least one sequence with a sequence identity of at least 90 %, preferably at least 95 %, more preferably at least 98 % to SEQ ID No. 51 or 53 and/or SEQ ID No. 52 or 54, wherein the at least one sequence binds the antigen CD3.

Preferably, the humanized anti-human CD3 effector cell-binding domain comprises a variable region of the heavy chain (V_{H}) according to SEQ ID No. 54 and a variable region of the light chain (V_{L}) according to SEQ ID No. 53, e.g. in the form of an scFv. Preferably, V_{H} and V_{L} are connected via glycine serine linker.

In some embodiments, the effector cell-binding domain is a humanized anti-TCR domain and preferably comprises at least one of the following sequences:
i) a V_{L} according to SEQ ID No. 55 and/or
ii) a V_{H} according to SEQ ID No. 56.

In embodiments, the effector cell-binding domain comprises at least one sequence with a sequence identity of at least 90 %, preferably at least 95 %, more preferably at least 98 % to the sequences according to SEQ ID No. 55 or SEQ ID No. 56, wherein the at least one sequence binds the antigen TCR.

In some embodiments, the effector cell-binding domain is a humanized anti-CD16A domain and preferably comprises at least one of the following sequences:
i) a V_{L} according to SEQ ID No. 57 and/or
ii) a V_{H} according to SEQ ID No. 58,
more preferably, a V_{L} according to SEQ ID No. 57 and a V_{H} according to SEQ ID No. 58.

In an embodiment, the effector cell-binding domain comprises at least one sequence with a sequence identity of at least 90 %, preferably at least 95 %, more preferably at least 98 % to the sequences according to SEQ ID No. 57 or SEQ ID No. 58, wherein the at least one sequence binds the antigen CD16A.

In embodiments, the different domains of the targeting module according to the invention comprising at least one HER2-binding domain are linked with each other by a linker. The linker comprises a short sequence of preferably 10 to 20 amino acid residues. In embodiments, the targeting module comprises a flexible peptide sequence that is selected such that the domains have a three-dimensional folding that allows them to exhibit the specificity for effector cell and target cell binding. Preferred linkers are glycine-serine linkers with the structure (GₓS_{y}) with x and y selected from 1 to 10, preferably 1 to 5. Mostly preferred are 1 to 10 repeats of the sequence G₄S₁ (SEQ ID No. 31). Moreover, linkers are preferred that are constituted of a peptide sequence that can increase the protease resistance of the antibody derivatives. In embodiments, the linker is SEQ ID No. 32 or SEQ ID No. 33.

Preferably, the targeting module according to the invention comprising at least one HER2-binding domain is expressed as a fusion protein.

In embodiments, the targeting module according to the invention comprising at least one HER2-binding domain further comprises an effector cell-binding domain, wherein the effector cell-binding domain specifically binds an epitope on a human CD3 chain, a human T cell receptor (TCR) or human CD16.

In embodiments, the targeting module according to the invention comprising at least one HER2-binding domain comprises SEQ ID No. 34, SEQ ID No. 35 or SEQ ID No. 36. Preferably, the targeting module has SEQ ID No. 34, SEQ ID No. 35 or SEQ ID No. 36.

In embodiments, the targeting module according to the invention comprising at least one HER2-binding domain comprises a further domain selected from the group comprising co-stimulatory ligands, radionuclides, cell death-inducing chemical compounds and half-life increasing domains, preferably IgG1 Fc, IgG2 Fc, IgG3 Fc, IgG4 Fc, HSA, FcRn-binding peptides or mutants thereof. As used herein, the term "mutants" refers to proteins having at least 85 % sequence identity to the half-life increasing domain, preferably at least 95 % sequence identity. Advantageously, the mutant is capable of having one or more activities of the named peptides or proteins; in particular, the mutant increases the half-life like the half-life increasing domain.

In preferred embodiments, the targeting module according to the invention comprising at least one HER2-binding domain comprises a further domain selected from the group comprising co-stimulatory ligands, radionuclides, cell death-inducing chemical compounds and half-life increasing domains, preferably IgG1 Fc, IgG2 Fc, IgG3 Fc, IgG4 Fc, HSA or FcRn-binding peptides.

In embodiments, the length of the targeting module comprising at least one HER2-binding domain is in the range of 20 to 1600 amino acids.

In embodiments, the targeting module according to the invention comprising at least one HER2-binding domain is used in a method for stimulating a chimeric antigen receptor-mediated immune response in a mammal, wherein the targeting module is administered in combination with a vector or a cell comprising a nucleotide sequence encoding a switchable chimeric antigen receptor and at least one further targeting module, wherein the at least one further targeting module comprises at least one target cell-binding domain and a tag-binding domain or a tag, wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269 (BCMA), CD273, CD274, CD276, CD279, CD319, CD366 and CD371, interleukin receptors, especially preferred IL-8Ra (CXCR1), IL-8Rβ (CXCR2), IL-11Rα, IL-11Rβ, IL13Ra1 and IL13Ra2, CXCR4, c-Met, mesothelin, members of the epidermal growth factor receptor family and mutants thereof, especially preferred ErbB1, ErbB3, ErbB4 or mutants thereof, members of the tumor necrosis factor receptor superfamily, ephrins, ephrin receptors, especially preferred EphA1-10, EphA5 or EphB1-6; prostate specific antigens, preferably PSCA and PSMA; embryonic antigens, preferably CEA and fetal acethylcholine receptor; members of the vascular endothelia growth factor family, epithelia cell adhesion molecule (EpCAM), alphafetoprotein (AFP), members of the intercellular adhesion molecule family, members of the mucin protein family, FSHR, HMW-MAA, folate binding protein (FBP), folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands; wherein the targeting module according to the invention comprising at least one HER2-binding domain and the at least one further targeting module comprise identical tag-binding domains or tags.

In preferred embodiments, the at least one further targeting module comprises at least one target cell-binding domain and a tag-binding domain or a tag, wherein the at least one target cell-binding domain is an antibody or an antibody fragment that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269, CD273, CD274, CD276, CD279, CD319, CD366 and CD371, interleukin receptors, especially preferred IL-8Rα, IL-8Rβ, IL-11Rα, IL-11Rβ, IL13Ra1 and IL13Ra2, CXCR4, c-Met, mesothelin, members of the epidermal growth factor receptor family, especially preferred ErbB1, ErbB3 or ErbB4; members of the tumor necrosis factor receptor superfamily, ephrins, ephrin receptors, especially preferred EphA1-10, EphA5 or EphB1-6; prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, especially preferred binds to IL13Ra2, wherein the targeting module according to the invention and the at least one further targeting module comprise different target cell-binding domains, and identical tag-binding domains or a tags.

In further embodiments, the at least one further targeting module comprises two target cell-binding domains binding to surface antigens selected from PSCA and PSMA, ErbB-1 and ErbB-2, PSCA and ErbB-2, PSMA and CEA, IL13Ra2 and ErbB-2, CD38 and CD269, mesothelin and mucin 16, PD-L1 and IL13Ra2.

In preferred embodiments, the targeting module according to the invention comprising at least one HER2-binding domain is used in a method for stimulating a chimeric antigen receptor-mediated immune response in a mammal, wherein the targeting module is administered until, preferably in the range of 3 days to 30 days, after the administration of the vector or cell comprising a nucleotide sequence encoding a switchable chimeric antigen receptor and the at least one further targeting module. Furthermore, additional such doses of the targeting modules may be administered following resting periods to reactivate the switchable chimeric antigen receptor-carrying effector cells.

Especially preferred, the targeting module according to the invention comprising at least one HER2-binding domain is used in a method for stimulating a chimeric antigen receptor-mediated immune response in a mammal, wherein the at least one further targeting module is administered on its own, preferably one hour to 2 days, more preferably 4 to 24 hours, prior to the administration of the vector or cell comprising a nucleotide sequence encoding a switchable chimeric antigen receptor and the targeting module according to the invention comprising at least one HER2-binding domain is administered until, preferably in the range of 3 days to 30 days, after the administration of the vector or cell comprising a nucleotide sequence encoding a switchable chimeric antigen receptor. Furthermore, additional such doses of the targeting module may be administered following resting periods to reactivate the switchable chimeric antigen receptor-carrying effector cells.

The present invention further comprises a nucleic acid, a vector or a cell comprising a nucleotide sequence encoding a targeting module according to the invention comprising at least one HER2-binding domain for use in a method for stimulating a chimeric antigen receptor-mediated immune response in a mammal, preferably for use in the treatment of cancer, infectious disease or autoimmune disease;
wherein the nucleic acid, vector or cell is administered in combination with a vector or a cell comprising a nucleotide sequence encoding a switchable chimeric antigen receptor, wherein the switchable chimeric antigen receptor comprises three domains, wherein
   - the first domain is a tag-binding domain or a tag,
   - the second domain is an extracellular hinge and a transmembrane domain and
   - the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

In embodiments, the nucleic acid is a cDNA. In embodiments, cDNA is of synthetic origin. In further embodiments, cDNA is derived from mRNA, therefore containing only exons but no introns, as opposed to genomic DNA.

The vector is preferably a plasmid, an artificial chromosome, linearized DNA or RNA, a virus particle or another vector that contains an expression cassette that is incorporated stably into the genome of a host cell or host organism.

In embodiments, the cell is selected from immune cells, preferably with cytolytic, phagocytic or immunosuppressive activity, such as T cells, Natural Killer (NK) cells and macrophages. In preferred embodiments, the cell is selected from T cells, including alpha/beta and gamma/delta T cells or subpopulations of T cells like stem-cell memory T cells or central memory T cells, cytotoxic T cells; or NK cells.

In embodiments, the nucleic acid, vector or cell further comprises an inducible expression system. In some embodiments, the inducible expression system is based on a prokaryotic operon, including, but not limited to, the lac operon, transposon Tn10 or tetracycline operon. In other embodiments, the inducible expression system is based on components of a eukaryotic signaling pathway, including, but not limited to, expression systems based on a steroid receptor, an estrogen receptor, progesterone or metallothionein.

In preferred embodiments, the inducible expression system is selected from Tet-On^{®} (also rtTA; TET Systems, Takara Bio), Tet-On^{®} Advanced (also rtTA2^{s}-M2 or rtTA2-syn1; TET Systems, Takara Bio), Tet-On^{®} 3G (also rtTA-V16; TET Systems, Takara Bio), T-REx^{®} (Life Technologies) or a promoter responsive to nuclear factor of activated T cells (NFAT).

In embodiments, the rtTA protein is a fusion protein of rTetR (reverse tetracycline repressor), a mutated version of TetR (tetracycline repressor) from Escherichia coli, and the activation domain of virion protein 16 (VP16) from Herpes Simplex Virus. In preferred embodiments, rtTA comprises SEQ ID No. 59, SEQ ID No. 60 or SEQ ID No. 61.

In embodiments, the TRE consists of 2 to 9 repeats, preferably 7 repeats, of the 19bp bacterial TetO sequence according to SEQ ID No. 62, preferably separated by spacer sequences, more preferably by spacer sequences comprising SEQ ID No. 63.

In preferred embodiments, the TRE is SEQ ID No. 64.

In embodiments, the promoter responsive to NFAT consists of 2 to 12 repeats, preferably 6 repeats, of the 30 bp human NFAT-binding sequence according to SEQ ID No. 65, optionally separated by spacer sequences, followed by a minimal promoter.

In embodiments, the minimal promoter is derived from the interleukin-2 (IL-2) promoter according to SEQ ID No. 66 or SEQ ID No. 67.

In embodiments, the promoter responsive to NFAT has SEQ ID No. 68 or SEQ ID No. 69.

In further embodiments, the nucleic acid, vector or cell according to the invention further comprises a nucleotide sequence encoding a switchable chimeric antigen receptor,
wherein the switchable chimeric antigen receptor comprises three domains,
wherein
   - the first domain is a tag-binding domain or tag,
   - the second domain is an extracellular hinge and a transmembrane domain and
   - the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

Another aspect of the invention is a nucleic acid, a vector or a cell comprising
- an inducible expression system, preferably selected from Tet-On, Tet-On Advanced, Tet-On 3G, T-REx or a promoter responsive to NFAT, and
- a nucleotide sequence encoding a switchable chimeric antigen receptor, wherein the switchable chimeric antigen receptor comprises three domains, wherein
   - the first domain is a tag-binding domain or tag,
   - the second domain is an extracellular hinge and a transmembrane domain and
   - the third domain is a signal transduction domain, and
- a nucleotide sequence encoding a targeting module according to the invention comprising at least one HER2-binding domain,
wherein the tag-binding domain of the targeting module binds to the tag of the switchable chimeric antigen receptor or the tag of the targeting module binds to the tag-binding domain of the switchable chimeric antigen receptor.

In embodiments, the inducible expression system induces the transcription of the nucleotide sequence encoding a switchable chimeric antigen receptor and/or a nucleotide sequence encoding a targeting module according to the invention, preferably the inducible expression system induces the transcription of the nucleotide sequence encoding a targeting module according to the invention.

In embodiments, the nucleic acid, vector or cell is administered in combination with a vector or a cell comprising a nucleotide sequence encoding a switchable chimeric antigen receptor and a nucleic acid, a vector or a cell comprising a nucleotide sequence encoding a further targeting module, wherein the at least one further targeting module comprises at least one target cell-binding domain and a tag-binding domain or a tag, wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269, CD273, CD274, CD276, CD279, CD319, CD366 and CD371, interleukin receptors, especially preferred IL-8Rα, IL-8Rβ, IL-11Rα, IL-11Rβ, IL13Ra1 and IL13Ra2, CXCR4, c-Met, mesothelin, members of the epidermal growth factor receptor family and mutants thereof, especially preferred ErbB1, ErbB3, ErbB4 or mutants thereof, members of the tumor necrosis factor receptor superfamily, ephrins, ephrin receptors, especially preferred EphA1-10, EphA5 or EphB1-6; prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands, wherein the targeting module according to the invention and the at least one further targeting module comprise identical tag-binding domains or tags.

The invention further comprises a pharmaceutical composition comprising the targeting module according to the invention comprising at least one HER2-binding domain, the nucleic acid, vector or cell according to the invention and a pharmaceutically acceptable thinner or carrier for use in a method for stimulating a chimeric antigen receptor-mediated immune response in a mammal, preferably for use in the treatment of cancer, infectious disease or autoimmune disease; wherein the pharmaceutical composition is administered in combination with a vector or a cell comprising a nucleotide sequence encoding a switchable chimeric antigen receptor, wherein the switchable chimeric antigen receptor comprises three domains, wherein
- the first domain is a tag-binding domain or a tag,
- the second domain is an extracellular hinge and a transmembrane domain and
- the third domain is a signal transduction domain,
   wherein the tag-binding domain of the targeting module binds to the tag of the switchable chimeric antigen receptor or the tag of the targeting module binds to the tag-binding domain of the switchable chimeric antigen receptor.

The pharmaceutical composition is preferably administered parenterally, particularly preferred intravenously. In embodiments, the pharmaceutical composition is present in a form suitable for intravenous administration. Preferably, the pharmaceutical composition is a solution, emulsion or suspension.

In embodiments, the pharmaceutical composition is an injectable buffered solution comprising a concentration in the range of 1 ng/ml to 500 mg/ml of the targeting module, the nucleic acid, vector and/or cell according to the invention, preferably in the range of 50 µg/ml to 5 mg/ml.

The pharmaceutical composition comprises a pharmaceutically acceptable thinner (dilution agent) or carrier. In embodiments, the carrier is selected from water, an aqueous buffer solution, 0.9 % saline solution, 5 % glucose, 5 % xylitol, 0.3 % glycine solution, ringer solutions or amino acid solutions. In further embodiments, the aqueous buffer solution is selected from an aqueous histidine, sodium succinate, sodium citrate, sodium phosphate or potassium phosphate-buffered solution with a pH value in the range of pH 5.0 to pH 7.0. In embodiments, the aqueous buffer solution has a buffer concentration in the range of 1 mmol/l (mM) to 500 mM, preferably in the range of 5 mM to 20 mM, especially preferred in the range of 5 mM to 10 mM.

In embodiments, the carrier comprises sodium chloride, preferably in a concentration in the range of 1 mM to 300 mM, especially preferred 150 mM.

In embodiments, the pharmaceutical composition further comprises a stabilizer, preferably with a concentration in the range of 1 mM to 900 mM, especially preferred in the range of 50 mM and 600 mM. In embodiments, the stabilizer is sucrose, trehalose or L-methionine.

In some embodiments, the pharmaceutical composition further comprises pharmaceutically acceptable excipients. In embodiments, pharmaceutically acceptable excipients are selected from sodium acetate, sodium chloride, potassium chloride, calcium chloride, sodium lactate and polysorbate-80, preferably polysorbate-80 in the range of 0.0001 % (w/v) to 1 % (w/v), especially preferred in the range of 0.001 % (w/v) to 0.1 % (w/v).

In preferred embodiments, the pharmaceutical composition comprises the targeting module according to the invention comprising at least one HER2-binding domain in a dosage quantity in the range of 25 µg/day to 100 mg/day, preferably dosage quantities in the range of 0.1 mg/day to 20 mg/day.

In further embodiments, the pharmaceutical composition is sterile. The pharmaceutical composition is sterilized by conventional well-known techniques including, but not limited to, sterile filtration.

In embodiments, the pharmaceutical composition is used for administration to a subject.

In embodiments, the pharmaceutical composition is lyophilized prior to storage or stored as a solution at ambient temperature or below, including, but not limited to, frozen storage.

In embodiments, the pharmaceutical composition is reconstituted and/or diluted in an infusion and stabilizer solution prior to administration to a subject. The solutions used for reconstitution or infusion/stabilization may contain any of the components mentioned for the pharmaceutical composition or similar components.

In embodiments, the pharmaceutical composition comprises at least one further targeting module or at least one further nucleic acid, vector or cell encoding a further targeting module, wherein the at least one further targeting module comprises at least one target cell-binding domain and a tag-binding domain or a tag, wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269, CD273, CD274, CD276, CD279, CD319, CD366 and CD371, interleukin receptors, especially preferred IL-8Rα, IL-8Rβ, IL-11Rα, IL-11Rβ, IL13Ra1 and IL13Ra2, CXCR4, c-Met, mesothelin, members of the epidermal growth factor receptor family and mutants thereof, especially preferred ErbB1, ErbB3, ErbB4 or mutants thereof, members of the tumor necrosis factor receptor superfamily, ephrins, ephrin receptors, especially preferred EphA1-10, EphA5 or EphB1-6; prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands, wherein the targeting module according to the invention comprising at least one HER2-binding domain and the at least one further targeting module comprise identical tag-binding domains or tags.

Another aspect of the invention is a kit comprising
a) a targeting module according to the invention comprising at least one HER2-binding domain, the nucleic acid, vector or cell according to the invention and
b) a vector or a cell comprising a nucleotide sequence encoding a switchable chimeric antigen receptor,
   wherein the switchable chimeric antigen receptor comprises three domains, wherein
   - the first domain is a tag-binding domain or tag,
   - the second domain is an extracellular hinge and a transmembrane domain and
   - the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

In embodiments, the extracellular hinge and transmembrane domain are selected from the hinge and transmembrane domains of human CD28 molecule, CD8a chain, NK cell receptors, preferably natural killer group NKG2D; or parts of the constant region of an antibody and combinations thereof.

In embodiments, the signal transduction domain is selected from cytoplasmic regions of CD28, CD137 (4-1BB), CD134 (OX40), CD278 (ICOS), DAP10 and CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor α), CD360 (interleukin-21 receptor) activating Fc receptors and mutants thereof.

In preferred embodiments, the signal transduction domain is selected from cytoplasmic regions of CD28, CD137 (4-1BB), CD134 (OX40), CD278 (ICOS), DAP10 and CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor α) and CD360 (interleukin-21 receptor) activating Fc receptors.

In embodiments, the switchable chimeric antigen receptor comprises at least one signal transduction domain, preferably two, three, four or more signal transduction domains, especially preferably selected from cytoplasmic regions of CD28, CD137, CD134, CD278, DAP10 and CD27, PD-1, CTLA-4, cytoplasmic regions of CD3 chains, DAP12, CD122, CD132, CD127, CD360, activating Fc receptors and mutants thereof.

In preferred embodiments, the switchable chimeric antigen receptor comprises at least one signal transduction domain, preferably two, three, four or more signal transduction domains, especially preferably selected from cytoplasmic regions of CD28, CD137, CD134, CD278, DAP10 and CD27, PD-1, CTLA-4, cytoplasmic regions of CD3 chains, DAP12, CD122, CD132, CD127, CD360 and activating Fc receptors.

In further embodiments, the switchable chimeric antigen receptor comprises a fourth domain, wherein the fourth domain is a short peptide linker in the extracellular portion of the receptor that may serve to detect the chimeric antigen receptor on the cell surface or stimulate the chimeric antigen receptor T cell.

In further embodiments, the fourth domain forms a linear epitope for a monoclonal antibody (mab) specifically binding to the fourth domain. In some embodiments, the fourth domain comprises at least one linear epitope, preferably E7B6 according to SEQ ID No. 44 or SEQ ID No. 45.

In some embodiments, the fourth domain is located in between the tag-binding domain or the tag and the extracellular hinge domain or an integral part of the extracellular hinge domain.

In further embodiments, the switchable CAR comprises a signal peptide. Advantageously, the signal peptide allows for expression on the cell surface of an effector cell. In embodiments, the signal peptide is located at the N-terminus of the switchable CAR nucleotide sequence in front of the tag-binding domain or the tag. In some embodiments, the signal peptide targets proteins to the secretory pathway either co-translationally or post-translationally and is selected from leader peptides from proteins like CD28, CD8alpha, IL-2, lysozyme C or the heavy or light chains of antibodies of human origin to avoid immunogenic reactions.

In embodiments, the nucleic acid is a cDNA.

In embodiments, the tag-binding domain or tag is present at the amino-terminal end of the polypeptide that comprises the switchable CAR. Advantageously, locating the tag-binding domain or tag at the amino terminus permits the tag-binding domain or tag unhampered access to the targeting module that is bound to the target cell.

In embodiments, the nucleic acid encodes a switchable CAR according to one of the sequences SEQ ID No. 70 to SEQ ID No. 77. Preferably, the nucleic acid is one of the sequences SEQ ID No. 78 to SEQ ID No. 85.

In some embodiments, the cell comprising a nucleotide sequence encoding a switchable chimeric antigen receptor is selected from immune cells, preferably with cytolytic, phagocytic or immunosuppressive activity, such as T cells, Natural Killer (NK) cells and macrophages. In preferred embodiments, the cell is selected from T cells, including alpha/beta and gamma/delta T cells or subpopulations of T cells like stem-cell memory T cells or central memory T cells, cytotoxic T cells; or NK cells.

In embodiments, the kit further comprises at least one further targeting module or at least one further nucleic acid, vector or cell encoding a further targeting module, wherein the at least one further targeting module comprises at least one target cell-binding domain and a tag-binding domain or a tag, wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269, CD273, CD274, CD276, CD279, CD319, CD366 and CD371, interleukin receptors, especially preferred IL-8Rα, IL-8Rβ, IL-11Rα, IL-11Rβ, IL13Ra1 and IL13Ra2, CXCR4, c-Met, mesothelin, members of the epidermal growth factor receptor family and mutants thereof, especially preferred ErbB1, ErbB3, ErbB4 or mutants thereof, members of the tumor necrosis factor receptor superfamily, ephrins, ephrin receptors, especially preferred EphA1-10, EphA5 or EphB1-6; prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands, wherein the targeting module according to the invention comprising at least one HER2-binding domain and the at least one further targeting module comprise identical tag-binding domains or tags.

In embodiments, the kit comprises
- a nucleic acid, vector or cell comprising a nucleotide sequence encoding a targeting module according to the invention comprising at least one HER2-binding domain,
- a vector or a cell comprising a nucleotide sequence encoding a switchable chimeric antigen receptor,
   wherein the switchable chimeric antigen receptor comprises three domains, wherein
   - the first domain is a tag-binding domain or tag,
   - the second domain is an extracellular hinge and a transmembrane domain and
   - the third domain is a signal transduction domain, and
- at least one further targeting module,
wherein the tag-binding domain of the targeting module binds to the tag of the switchable chimeric antigen receptor or the tag of the targeting module binds to the tag-binding domain of the switchable chimeric antigen receptor,
wherein the targeting module according to the invention comprising at least one HER2-binding domain and the at least one further targeting module comprise different target cell-binding domains, and identical tag-binding domains or tags.

In preferred embodiments, the at least one further targeting module comprises at least one target cell-binding domain and a tag-binding domain or a tag, wherein the at least one target cell-binding domain is an antibody, antibody fragment, a protein, a peptide or a low molecular weight organic ligand that binds to IL13Rα2, wherein the targeting module according to the invention and the at least one further targeting module comprise identical tag-binding domains or tags.

In embodiments, the kit comprises one to three targeting modules, preferably one targeting module according to the invention comprising at least one HER2-binding domain and one or two further targeting modules.

In embodiments, the kit further comprises an inducer, preferably tetracycline or a derivative of tetracycline, e.g. doxycycline.

In embodiments of the kit, the targeting module, the vector and/or the cell comprising a nucleotide sequence encoding a switchable chimeric antigen receptor are in the form of a pharmaceutical composition.

Another aspect of the invention is the kit according to the invention for use in a method for stimulating a chimeric antigen receptor-mediated immune response in a mammal, preferably for use in the treatment of cancer, infectious disease or autoimmune disease.

In embodiments, the targeting module according to the invention comprising at least one HER2-binding domain, the nucleic acid, vector or cell according to the invention or the kit according to the invention is used for preparing a medication for therapeutic and/or diagnostic use in case of cancer, an infection or an autoimmune disease.

A further aspect of the invention is a method for stimulating a chimeric antigen receptor-mediated immune response in a mammal, preferably a human, having cancer, an infectious or an autoimmune disease by administration of a targeting module, a cell or a vector, a pharmaceutical composition or a kit according to the invention. For therapeutic applications, a sterile pharmaceutical composition according to the invention or a sterile kit according to the invention, comprising a pharmacologically effective quantity of targeting module according to the invention comprising at least one HER2-binding domain and a vector or a cell comprising a nucleotide sequence encoding a switchable chimeric antigen receptor, is administered to a subject in order to treat the aforementioned illnesses.

In embodiments, the method for stimulating a chimeric antigen receptor-mediated immune response in a mammal; preferably, the method for treatment of cancer, infectious or autoimmune disease, further comprises the administration of an inducer, preferably tetracycline or a derivative of tetracycline, e.g. doxycycline.

In embodiments, an inducer, preferably tetracycline or a derivative of tetracycline, e.g. doxycycline; is administered orally or intravenously following administration of the nucleic acid, vector or cell according to the invention.

In some embodiments, the method for stimulating a chimeric antigen receptor-mediated immune response in a mammal; preferably, the method for treatment of cancer, infectious or autoimmune disease, comprises the following steps:
a) administering to a mammal an effective amount of a targeting module according to the invention comprising at least one HER2-binding domain and
b) administering to the mammal an effective amount of a vector or a cell comprising a nucleotide sequence encoding a switchable chimeric antigen receptor,
   wherein the switchable chimeric antigen receptor comprises three domains, wherein
      - the first domain is a tag-binding domain or tag,
      - the second domain is an extracellular hinge and a transmembrane domain and
      - the third domain is a signal transduction domain,
   wherein the tag-binding domain of the targeting module binds to the tag of the switchable chimeric antigen receptor or the tag of the targeting module binds to the tag-binding domain of the switchable chimeric antigen receptor,
wherein the targeting module is administered to a mammal prior to, concurrent with or after the administration of the vector or cell.

In further embodiments, the method for stimulating a chimeric antigen receptor-mediated immune response in a mammal; preferably, the method for treatment of cancer, infectious or autoimmune disease, comprises the following steps:
a) administering to a mammal an effective amount of a targeting module comprising at least one target cell-binding domain and a tag-binding domain or a tag,
   wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269, CD273, CD274, CD276, CD279, CD319, CD366 and CD371, interleukin receptors, especially preferred IL-8Rα, IL-8Rβ, IL-11Rα, IL-11Rβ, IL13Ra1 and IL13Ra2, CXCR4, c-Met, mesothelin, members of the epidermal growth factor receptor family and mutants thereof, especially preferred ErbB1, ErbB3, ErbB4 or mutants thereof, members of the tumor necrosis factor receptor superfamily, ephrins, ephrin receptors, especially preferred EphA1-10, EphA5 or EphB1-6; prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands, and
b) administering to the mammal an effective amount of a vector or a cell comprising a nucleotide sequence encoding a switchable chimeric antigen receptor,
   wherein the switchable chimeric antigen receptor comprises three domains, wherein
      - the first domain is a tag-binding domain or tag,
      - the second domain is an extracellular hinge and a transmembrane domain and
      - the third domain is a signal transduction domain,
   wherein the tag-binding domain of the targeting module binds to the tag of the switchable chimeric antigen receptor or the tag of the targeting module binds to the tag-binding domain of the switchable chimeric antigen receptor, and
c) administering to a mammal an effective amount of a targeting module according to the invention comprising at least one HER2-binding domain,
wherein the targeting module according to the invention comprising at least one HER2-binding domain and the at least one further targeting module comprise different target cell-binding domains, and identical tag-binding domains or tags, wherein the method is carried out in the order of the steps a), b) and c).

In preferred embodiments, the further targeting module is administered on its own, preferably one hour to 2 days, more preferably 4 to 24 hours, prior to the administration of the vector or cell comprising a nucleotide sequence encoding a switchable chimeric antigen receptor and the targeting module according to the invention comprising at least one HER2-binding domain is administered until, preferably in the range of 3 days to 30 days, after the administration of the vector or cell comprising a nucleotide sequence encoding a switchable chimeric antigen receptor. Furthermore, additional such doses of the targeting module may be administered following resting periods to reactivate the switchable chimeric antigen receptor-carrying effector cells.

In further embodiments, the method for stimulating a chimeric antigen receptor-mediated immune response in a mammal, preferably the method for the treatment of cancer, infectious or autoimmune disease, comprises the following steps:
a) administering to a mammal an effective amount of a vector or a cell comprising an inducible expression system, preferably selected from Tet-On, Tet-On Advanced transactivator, Tet-On 3G or a promoter responsive to NFAT; and a nucleotide sequence encoding a switchable chimeric antigen receptor, wherein the switchable chimeric antigen receptor comprises three domains, wherein
   - the first domain is a tag-binding domain or tag,
   - the second domain is an extracellular hinge and a transmembrane domain and
   - the third domain is a signal transduction domain, and
   a nucleotide sequence encoding a targeting module according to the invention comprising at least one HER2-binding domain, wherein the tag-binding domain of the targeting module binds to the tag of the switchable chimeric antigen receptor or the tag of the targeting module binds to the tag-binding domain of the switchable chimeric antigen receptor, and
b) administering to a mammal an effective amount of an inducer, preferably tetracycline or a derivative of tetracycline, e.g. doxycycline.

In embodiments, the inducible expression system induces the transcription of the nucleotide sequence encoding a switchable chimeric antigen receptor and/or a nucleotide sequence encoding a targeting module according to the invention comprising at least one HER2-binding domain, preferably the inducible expression system induces the transcription of the nucleotide sequence encoding a targeting module according to the invention comprising at least one HER2-binding domain.

In embodiments, the method for stimulating a chimeric antigen receptor-mediated immune response in a mammal, preferably the method for treatment of cancer, infectious or autoimmune disease, comprises the following steps:
a) administering to a mammal an effective amount of a targeting module comprising at least one target cell-binding domain and a tag-binding domain or a tag,
   wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269, CD273, CD274, CD276, CD279, CD319, CD366 and CD371, interleukin receptors, especially preferred IL-8Rα, IL-8Rβ, IL-11Rα, IL-11Rβ, IL13Ra1 and IL13Ra2, CXCR4, c-Met, mesothelin, members of the epidermal growth factor receptor family, especially preferred ErbB1, ErbB2, ErbB3 or ErbB4; members of the tumor necrosis factor receptor superfamily, ephrins, ephrin receptors, especially preferred EphA1-10, EphA5 or EphB1-6; prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, preferably the at least one target cell-binding domain is an antibody or an antibody fragment that binds to IL13Rα2, and
b) administering to a mammal an effective amount of a vector or a cell comprising an inducible expression system, preferably selected from Tet-On, Tet-On Advanced transactivator, Tet-On 3G or a promoter responsive to NFAT; and a nucleotide sequence encoding a switchable chimeric antigen receptor, wherein the switchable chimeric antigen receptor comprises three domains, wherein
   - the first domain is a tag-binding domain or tag,
   - the second domain is an extracellular hinge and a transmembrane domain and
   - the third domain is a signal transduction domain, and
   a nucleotide sequence encoding a targeting module according to the invention comprising at least one HER2-binding domain, wherein the tag-binding domain of the targeting module binds to the tag of the switchable chimeric antigen receptor or the tag of the targeting module binds to the tag-binding domain of the switchable chimeric antigen receptor and,
c) administering to a mammal an effective amount of an inducer, preferably tetracycline or a derivative of tetracycline, e.g. doxycycline,
wherein the targeting module according to the invention at least one HER2-binding domain and the at least one further targeting module comprise different target cell-binding domains, and identical tag-binding domains or tags, and wherein the method is carried out preferably in the order of the steps a), b) and c).

In further embodiments, the preceding embodiments can be combined.

### Figures and Examples

The present invention will now be further explained by the following non-limiting figures and examples.
**Fig. 1** shows a schema of a switchable chimeric antigen receptor (CAR) with three domains, wherein the first domain is a tag-binding domain or a tag (exemplified as scFv), the second domain is an extracellular hinge and a transmembrane domain and the third domain is a signal transduction domain, and the optional fourth domain is a short peptide linker in the extracellular portion of the receptor.

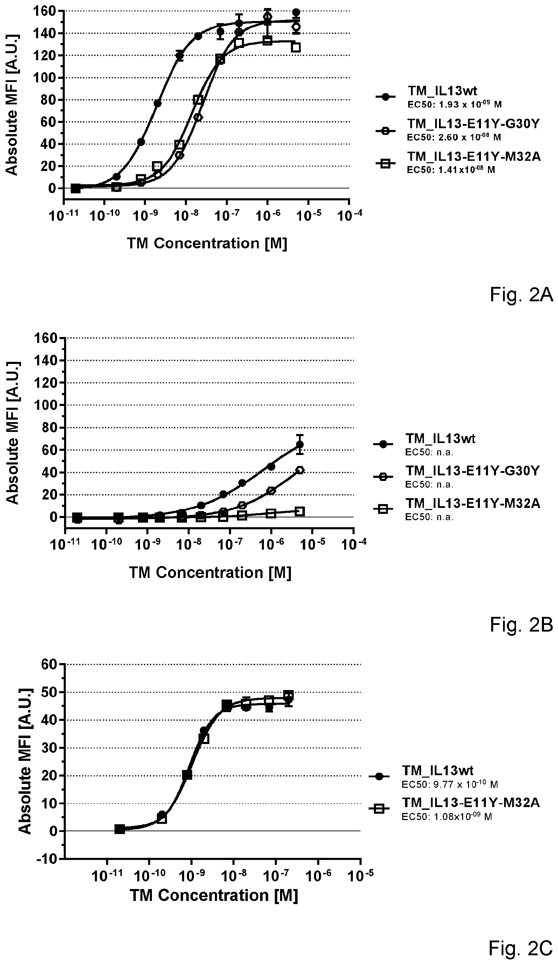

**Fig. 2** Cellular binding assay of IL13-based targeting modules: **A)** on recombinant K562-IL13Rα2 cell line, **B)** on recombinant K562-IL13Rα1 cell line and **C)** on U251-MG cell line, analyzed by FACS in a two-step immunostaining. Each data point represents the mean value of three technical replicates. Error bars represent the standard deviation.

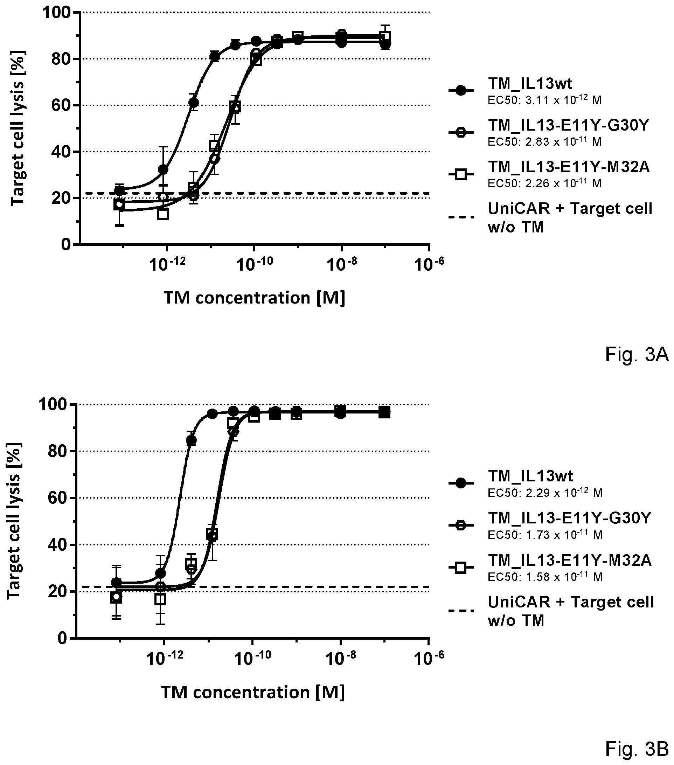

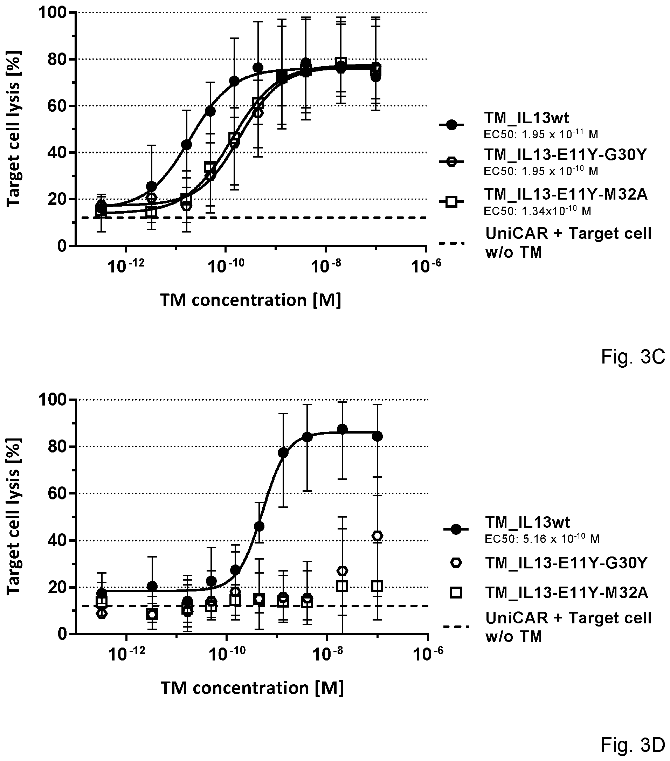

**Fig. 3** Cytotoxicity assay of IL13-based TMs targeting IL13Rα2 on **A)** U251-MG cell line, **B)** PC3 cell line, **C)** recombinant K562-IL13Rα2 cell line and **D)** recombinant K562-IL13Rα1 cell line, assessed on a high-content microscopy-based cytotoxicity assay (CTX). The dashed line shows the lysis of a control without TM (switchable CAR T and target cells). Each data point represents the mean value of three biological replicates (different T cell donors) with the corresponding standard deviation.

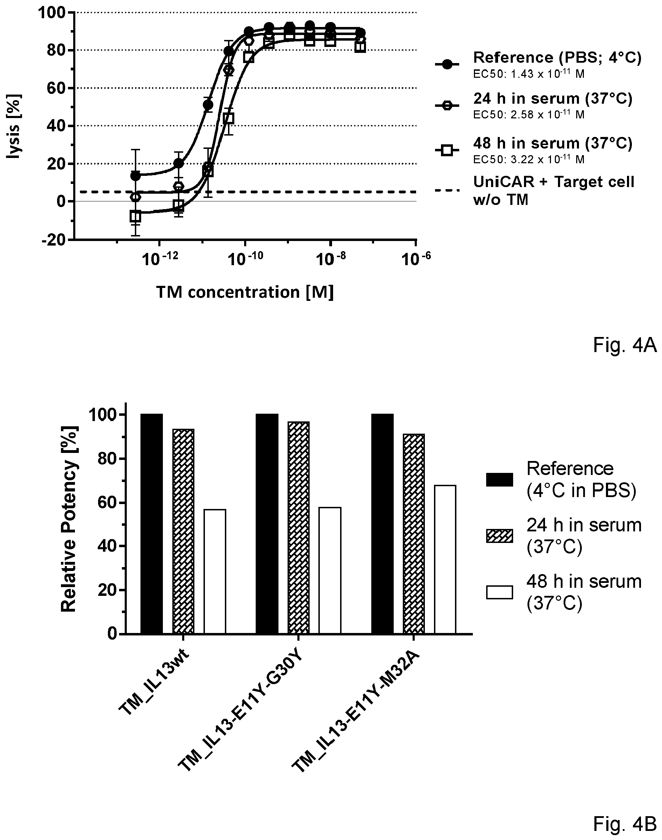

**Fig. 4 A)** TM-IL13-E11Y-M32A stability in mouse serum at 37°C for 24 h and 48h assessed via functional potency assay on a high-content microscopy-based cytotoxicity assay. Each data point represents the mean value of three technical replicates with the corresponding standard deviation. **B)** IL13-based TMs stability in human serum at 37°C for 24 h and 48h assessed via functional potency assay on a high-content microscopy-based cytotoxicity assay. EC50 values of the TM samples were relativized to the EC50 value obtained from the same TM reference stored in a phosphate-buffered saline at 4°C. Each data point represents the mean value of three technical replicates.

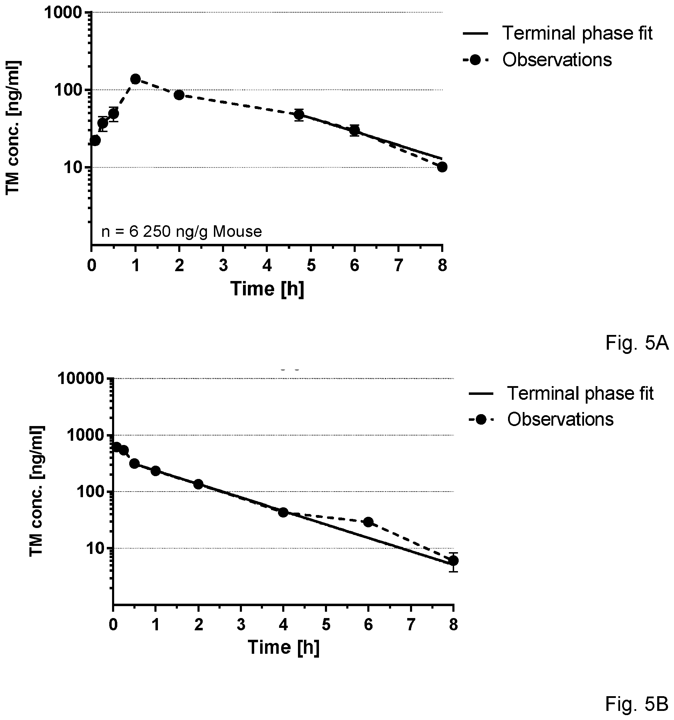

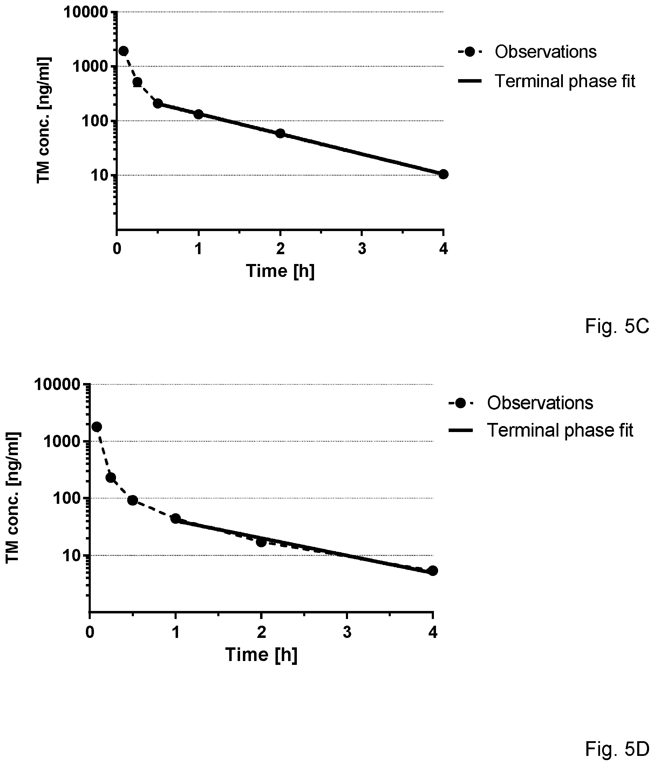

**Fig. 5** Pharmacokinetic study of diverse TMs analyzed by a sandwich ELISA: **A)** TM-IL13-E11Y-M32A after intraperitoneal administration, and **B)** TM-IL13-E11Y-M32A **C)** TM-33A1-scFv04 and **D)** TM-hu4D after intravenous administration. Each point represents the mean value of six biological replicates (different mice) with its corresponding standard deviation.

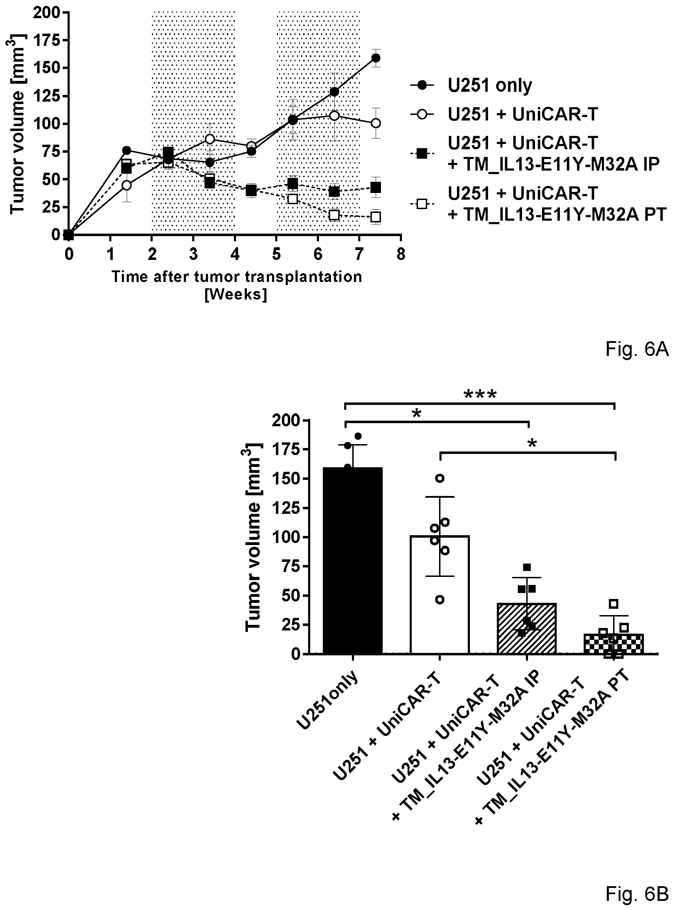

**Fig. 6** Tumor size progression in NSG mice transplanted with U251-MG treated with IL13-based TM TM-IL13-E11Y-M32A. TM therapy was divided into two cycles (dotted window in the graph) of two weeks each, excluding weekends. One therapy group was treated with TM administered intraperitoneally (IP) twice a day (1 µg/g mouse) and another group was treated with TM administered peritumorally (PT) once a day (30 µg/mouse). Each data point represents the mean value of six biological replicates (different mice) with its corresponding statistical error of the mean. **A)** Tumor size progression during immunotherapy, **B)** tumor size 7 weeks after tumor transplant. Statistical significance of tumor size differences after two therapeutic cycles (week 7) was analyzed using a non-parametric unpaired Kruskal-Wallis test with Dunnett multiple comparison test (p < 0.05 = *, p < 0.01 = **, p < 0.005 = ***).

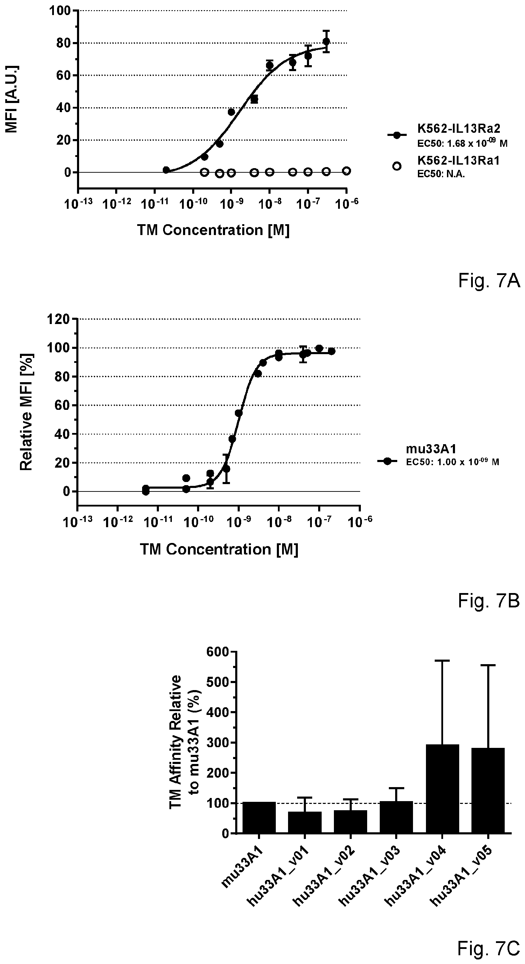

**Fig. 7** Cellular binding assay of Ab-based TM: **A)** TM-mu33A1-scFv on recombinant K562-IL13Rα2 and K562-IL13Rα1 cell line, **B)** TM-mu33A1-scFv on PC3 cell line, analyzed by FACS in a two-step immunostaining. Each data point represents the mean of values obtained from two independent experiments with three technical replicates each. Error bars represent the standard deviation. **C)** Relative affinity of TMs based on different humanized version of mu33A1-scFv (TM-hu33A1v01 to TM-hu33A1v05) on U251-MG cell line. Binding EC50 values of each humanized version was relativized to the EC50 value of the parental TM (TM-mu33A1-scFv).

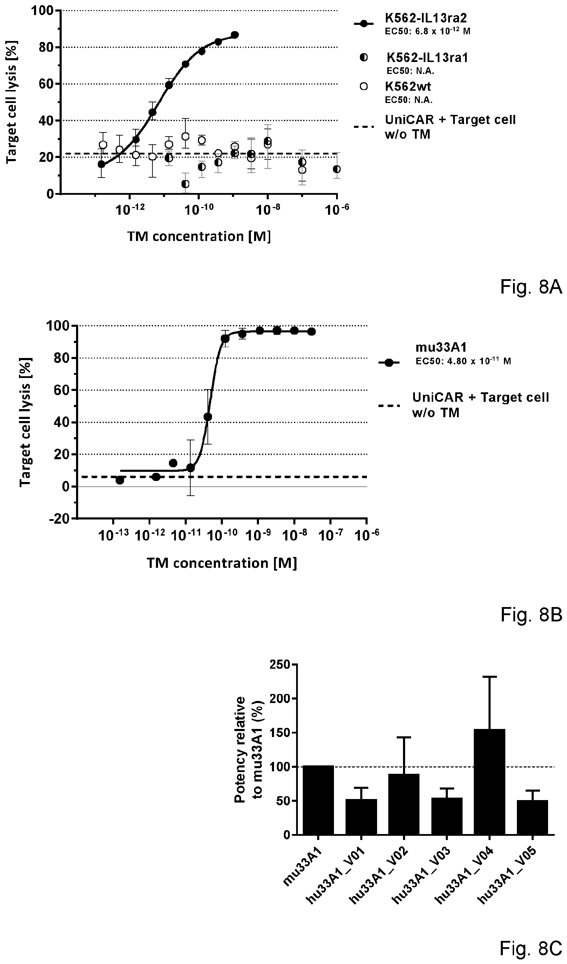

**Fig. 8** Cytotoxicity assay of Ab-based TM: **A)** TM-mu33A1-scFv on K562wt, recombinant K562-IL13Rα2 and K562-IL13Rα1 cell line, **B)** TM-mu33A1-scFv on PC3 cell line, assessed on a high-content microscopy-based cytotoxicity assay. The dashed line shows the lysis of a control without TM (switchable CAR T cell and target cells). Each data point represents the mean value of three biological replicates (different T cell donors) with the corresponding standard deviation. C) Relative potency of TMs based on different humanized version of mu33A1-scFv (TM-hu33A1v01 to TM-hu33A1v05) to induce the lysis of U251-MG cell line by UniCAR T cells. EC50 values of each humanized version was relativized to the EC50 value of the parental TM (TM-mu33A1-scFv).

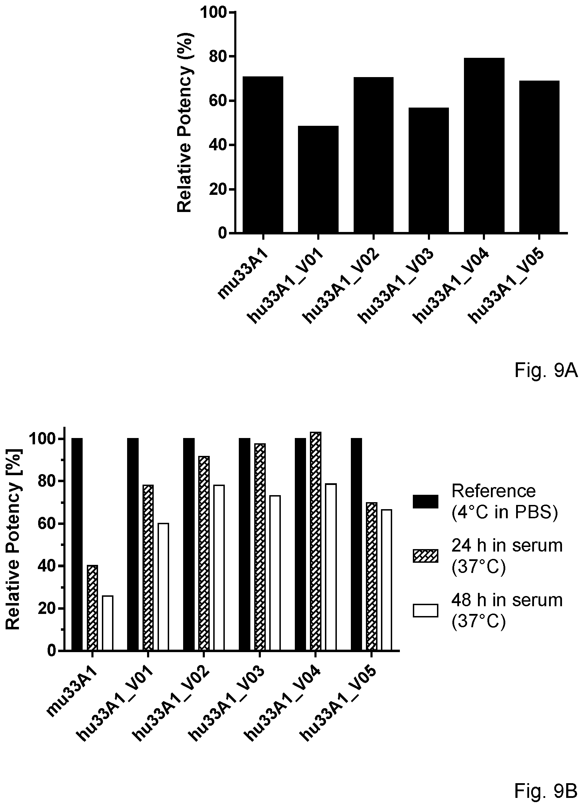

**Fig. 9** Ab-based TM stability in different conditions was assessed via functional potency assay on a high-content microscopy-based cytotoxicity assay. EC50 values of the TM samples were relativized to the EC50 value obtained from the same TM reference. **A)** Stability study of TM samples stored in a phosphate-buffered saline at 25°C for seven days. The reference sample for relativization was thawed directly before the experiment setup. Each data point represents the mean value of two biological replicates (different T cell donors) with three technical replicates each with the corresponding standard deviation. **B)** Stability study of TM incubated in human serum at 37°C for 24 h and 48 h. The reference sample was stored in a phosphate-buffered saline at 4°C. Each data point represents the mean value of three technical replicates.

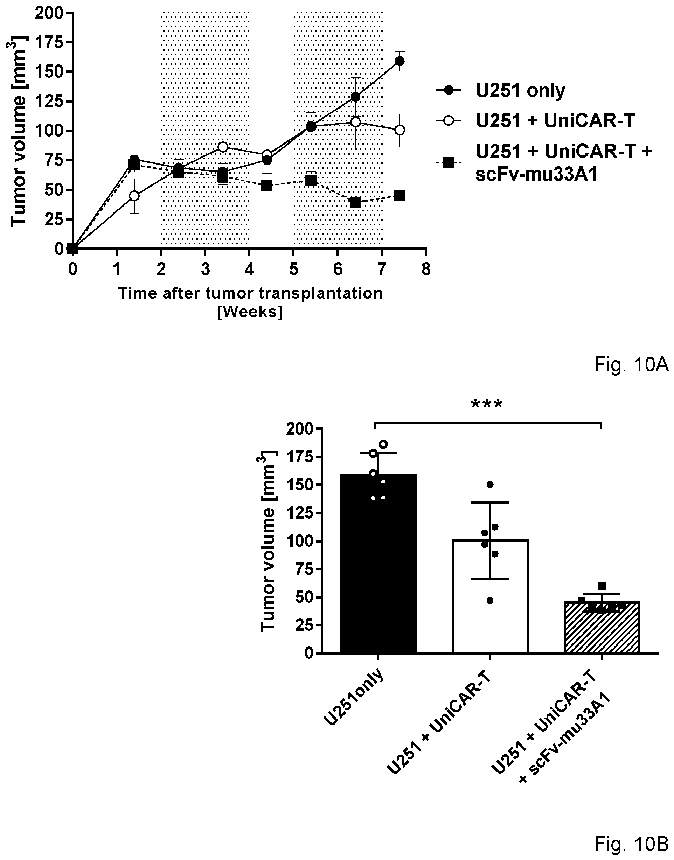

**Fig. 10** Tumor size progression in NSG mice transplanted with U251-MG treated with Ab-based TM TM-mu33A1-scFv. TM therapy was divided into two cycles (dotted window in the graph) of two weeks each, excluding weekends. The therapy group was treated with TM administered intraperitoneally (IP) twice a day (1 µg/g mouse). Each data point represents the mean value of six biological replicates (different mice) with its corresponding statistical error of the mean. **A)** Tumor size progression during immunotherapy, **B)** tumor size 7 weeks after tumor transplant.
Statistical significance of tumor size differences after two therapeutic cycles (week 7) was analyzed using a non-parametric unpaired Kruskal-Wallis test with Dunnett multiple comparison test (p < 0.05 = *, p < 0.01 = **, p < 0.005 = ***).

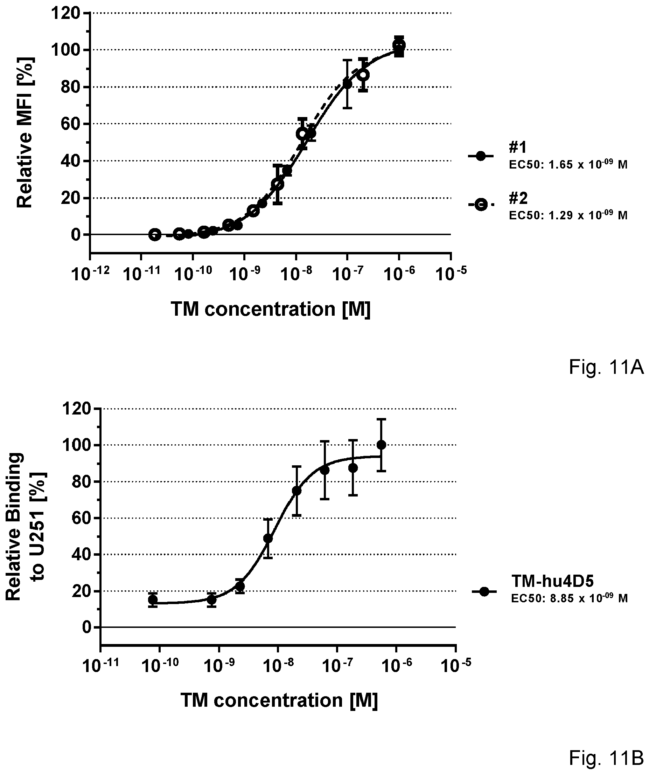

**Fig. 11** Cellular binding assay of HER2-binding targeting module TM-hu4D5: **A)** on recombinant K562-HER2 cell line (each curve corresponds to independent experiments), **B)** on U251-MG cell line, analyzed by FACS in a two-step immunostaining. Each data point represents the mean value of three technical replicates. Error bars represent the standard deviation.

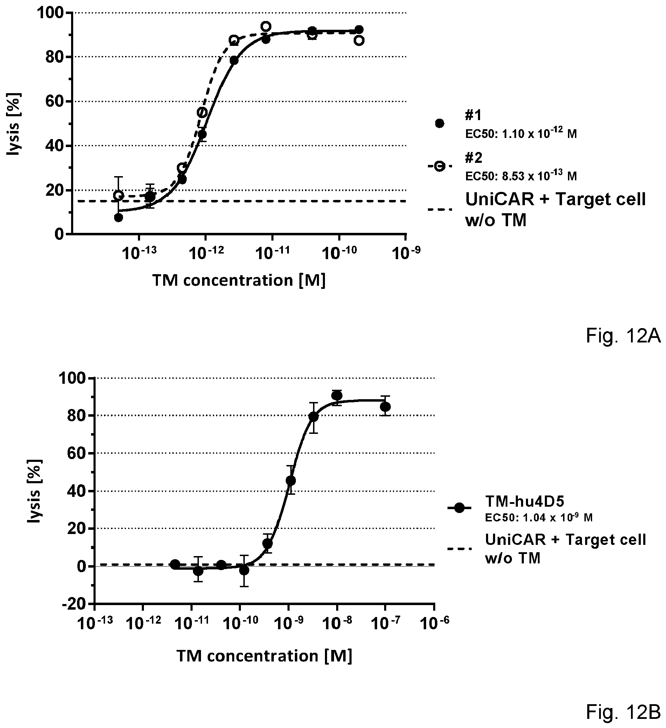

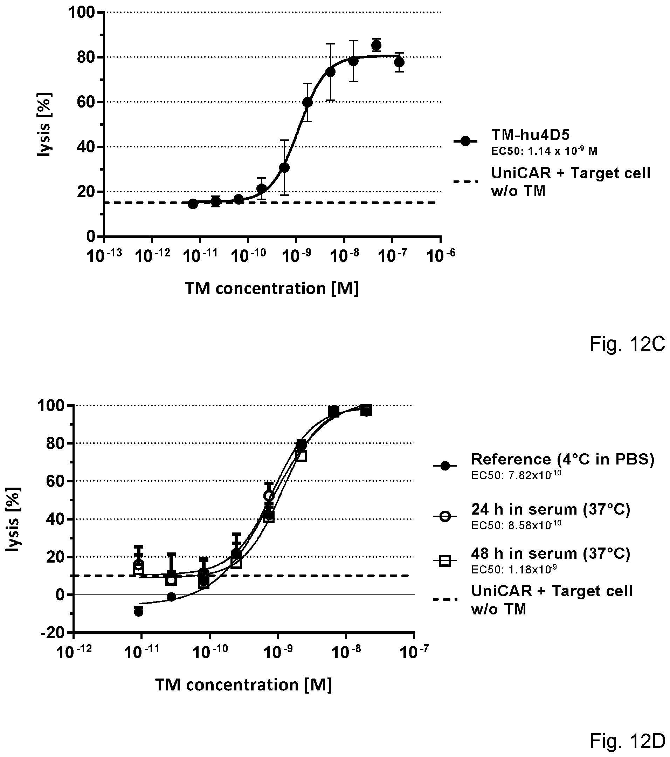

**Fig. 12** Cytotoxicity assay of HER2-binding targeting module TM-hu4D5: **A)** on recombinant K562-HER2 (each curve corresponds to independent experiments performed with different T cell donors), **B)** on U251-MG cell line and **C)** on PC3 cell line, assessed on a flow-cytometry-based cytotoxicity assay (A) or on a high-content microscopy-based cytotoxicity assay (CTX) (B and C). The dashed line shows the lysis of a control without TM (switchable CAR T cell and target cells). Each data point represents the mean value of three technical replicates (different T cell donors) (A), three biological replicates (different T cell donors) with three technical replicates each (B) or two biological replicates (different T cell donors) with three technical replicates each (C) with the corresponding standard deviation. **D)** Stability study of TM-hu4D5 incubated in human serum at 37°C for 24 h and 48 h was assessed via functional potency assay on a high-content microscopy-based cytotoxicity assay. EC50 values of the TM samples were relativized to the EC50 value obtained from the same TM reference stored in a phosphate-buffered saline at 4°C. Each data point represents the mean value of three technical replicates.

### Production of the switchable CAR cell

The immune cells can be genetically engineered to express switchable CARs. A polynucleotide vector encoding the switchable CAR and all necessary elements to ensure its expression in the genetically engineered immune cell is transferred into the immune cell. The transfer of the vector can be performed by electroporation or transfection of nucleic acids or the help of viral vector systems like adeno-, adeno-associated, retro-, foamy- or lentiviral viral gene transfer.

The lentiviral gene transfer is applied for stable expression of switchable CARs in immune cells by first constructing a lentiviral vector encoding for a selected switchable CAR. The lentiviral vector is pLVX-EF1alpha UniCAR 28/ζ (Clontech, Takara Bio Group), in which the lentiviral parts of the vector are derived from the human immunodeficiency virus (HIV) and the MSC/IRES/ZxGreenl portion was replaced by the switchable CAR construct.

The lentiviral particles are produced by transient transfection of human embryonal kidney (HEK) 293T (ACC 635) cells with the switchable CAR encoding lentiviral vector plasmid and cotransfection with a group specific antigen (gag) and Polymerase (pol) encoding plasmid (psPAX2) plus a plasmid encoding for an envelope (pMD2.G). After transfection, the packaging plasmid expresses Gag and Pol protein of HIV-1. The plasmid MD2.G encodes the glycoprotein of the vesicular stomatitis virus (VSV-G). VSV-G protein is used to lentiviral vectors to transduce a broad range of mammalian cells. Various envelopes from different virus species can be utilized for this purpose. Lentiviral vectors can successfully pseudotype with the envelope glycoproteins (Env) of amphotropic murine leukemia virus (MLV) or the G protein of vesicular stomatitis virus (VSV-G), a modified envelope of the prototypic foamy virus (PFV) or chimeric envelope glycoprotein variants derived from gibbon ape leukemia virus (GaLV) and MLV.

Supernatants from transfected HEK293T cells are harvested 24 h to 96 h after transfection and virus particles are concentrated from the supernatant by ultracentrifugation or other methods. For lentiviral transduction of immune cells, *peripheral blood mononuclear cells* (PBMC) or isolated T cells are activated with mab specific for the CD3 complex, e.g. clone OKT3 or UCHT1, either given in solution or coated to plastic cell culture dishes or magnetic beads or a biodegradable polymer matrix. Activation of PBMC or isolated T cells is further enhanced by stimulating costimulatory pathways with mabs or ligands specific for CD27, CD28, CD134 or CD137 either alone or in combinations coated to plastic cell culture dishes or magnetic beads or a biodegradable polymer matrix and the supply with exogenous recombinant cytokines like interleukin (IL)-2, IL-7, IL-12, IL-15 and IL-21. Concentrated or non-concentrated virus particles are added to PBMC or T cell cultures 24 h to 96 h after initial administration of activating CD3 specific antibodies and/or antibodies specific for costimulatory receptors CD27, CD28, CD134 or CD137 and/or recombinant cytokines as single or multiple doses. T cell electroporation, transduction and expansion may be performed in open cell culture systems by manual handling or in closed partially or fully automated systems.

Stable transduction of T cells may be determined by flow cytometry after staining with tag-containing molecules for surface expression of switchable CARs or mabs directed against the fourth domain of switchable CARs from day 3 onwards after the final administration of virus supernatant. Switchable CAR transduced T cells can be propagated *in vitro* by culturing them under the supply of recombinant cytokines and activating anti-CD3 mabs.

In case the switchable CAR harbors the optional fourth domain, a peptide sequence forming a linear epitope for a mab, immune cells genetically modified to express switchable CARs can be specifically propagated *in vitro* by coating a mab or antibody fragments thereof binding to the fourth switchable CAR domain to the surface of culture dishes or to beads of any kind or a biodegradable polymer matrix, which are added to the cell culture at a defined ratio. The binding of surface-coated mabs to the switchable CAR peptide domain induces cross-linkage of cell-surface expressed switchable CARs and formation of an immune synapse, which leads to the activation of signal pathways specifically triggered by the signal domain of the switchable CAR. Depending on the signal pathways induced, this may lead to enhance proliferation and sustained resistance against activation-induced cell death of the switchable CAR-carrying immune cells and therefore enrichment of switchable CAR genetically modified immune cells in a mixed population.

The optional fourth domain, a peptide sequence forming a linear epitope for a mab, can be further utilized to enrich and purify switchable CAR-expressing immune cells from mixed populations. Enrichment and purification are performed with the help of a mab or antibody fragment thereof binding to the fourth switchable CAR domain to either mark switchable CAR-expressing cells for cell sorting or to transiently link the switchable CAR expressing immune cell to small particles, which can be utilized for cell isolation. In one aspect, switchable CAR-engrafted immune cells are incubated with the mab recognizing the fourth domain. Next, magnetic beads are added, which are conjugated with antibodies or fragments thereof directed against the species- and isotype-specific heavy and light chains of the mab binding to the optional fourth domain. Thus, switchable CAR-expressing immune cells and magnetic beads are linked and are trapped and separated from other immune cells in a magnetic field.

### Design of targeting modules according to the invention

The targeting modules comprise the epitope E5B9 from the human La protein recognized by a switchable CAR and an 8x-histidine tag for detection and purification purposes at the C-terminus. The antigen-binding domain of these targeting modules consist of either a mutein of the human IL13 optimized to target the tumor-associated IL13Rα2 without presenting cross-reactivity against the ubiquitously expressed IL13Rα1, or an antibody fragment targeting IL13Rα2 or HER2.

### Characterization of the functionality of the targeting modules according to the invention

The functionality of the targeting modules described in this invention was assessed regarding the ability of these molecules to bind their corresponding targeted antigens, both in soluble form (surface plasmon resonance) and attached to the membrane of tumor cells (flow cytometry-based cellular binding assay), as well as their potential to induce the elimination of IL13Ra2- and HER2-expressing tumor cells by switchable CAR T cells both *in vivo* and *in vitro.* Other parameters described to support the functionality of these targeting modules include the stability of these molecules in mouse and/or human serum at 37°C for two days or in a saline buffer at 25°C for 7 days.

### Cellular binding assay:

Cellular binding of IL13-based targeting modules (TM) was tested on recombinant K562-IL13Rα2 cell line (**Fig. 2A**), recombinant K562-IL13Rα1 cell line (**Fig. 2B**) and U251-MG cells (**Fig. 2C**) (derived from a malignant glioblastoma tumor by explant technique). The affinity of TMs was analyzed by FACS in a two-step immunostaining. The TMs were titrated on an AML cell line with recombinant overexpression of IL13Rα2 or IL13Rα1 or U251-MG cells, respectively, and then detected by a mouse anti-His-tag antibody conjugated to phycoerythrin. A dose-response curve was obtained when the geometric mean fluorescence (MFI) was plotted against the TM concentration.

The data for the cellular binding of IL13-based TMs on the IL13Rα2-expressing AML cell line and U251-MG cells were fitted using a four-parameter model with a variable slope for sigmoidal curves. The 50% effective concentration (EC₅₀) obtained from this model can be interpreted as a representative value of the TM affinity for the cells overexpressing the target receptor (**Fig. 2A** and **2C**). The EC50 values of IL13-based TMs were in a range between 1.9 nM and 14.1 nM for K562-IL13Rα2 cells and between 0.98 nM and 1.1 nM for the U251-MG cell line.

Since no saturation of the binding sites of IL13-based TMs on the IL13Rα1 cell line was reached in the tested concentration range, the observed data could not be fitted to a dose-response curve (**Fig. 2B**), confirming the absence of the undesired crossreactivity with IL13Rα1.

Furthermore, the cellular binding of Ab-based targeting modules, in particular an IL13Rα2-binding antibody fragment with CDRs according to SEQ ID No. 11 to 16 (TM-mu33A1-scFv (**Fig. 7A-C**) and TM-hu33A1-scFv01 to TM-hu33A1-scFv05 (**Fig. 7C**)), was tested on recombinant K562-IL13Rα2 cell line, recombinant K562-IL13Rα1 cell line (**Fig. 7A**), the prostatic adenocarcinoma PC3 cell line (**Fig. 7B**) and the glioblastoma U251-MG cell line (**Tab. 1** and **Fig. 7C**). The data were fitted using a four-parameter model with a variable slope for sigmoidal curves. The EC₅₀ value obtained from this model can be interpreted as a representative value of the TM affinity for the cells overexpressing the target receptor. The TM based on the parental murine clone 33A1 yielded an EC50 value of 1.68 nM for K562-IL13Rα2 cells and 1 nM for PC3 cells. No specific binding was found titrating this TM on K562-IL13Rα1, confirming the absence of undesired crossreactivity of this clone family for IL13Rα1, which is expressed on normal tissues. TM-mu33A1-scFv, as well as TM-hu33A1-scFv01 to TM-hu33A1-scFv05, binding to the glioblastoma cell line, U251-MG, yielded EC50 value in a range between 1.0 nM and 3.6 nM. TM-hu33A1-scFv04 showed the highest affinity for U251-MG cells with an EC50 value of 1.0 nM.

The relative affinity for U251-MG cells of the targeting modules containing different versions of the humanized antibody fragment 33A1 (TM-hu33A1-scFv01 to TM-hu33A1-scFv05) was calculated by relativizing the EC₅₀ value of each humanized targeting module to the EC50 value of the targeting module containing the parental murine antibody fragment (TM-mu33A1-scFv). The mean values were calculated from the results obtained from two independent experiments with three technical replicates each (see **Tab. 1**).

**Tab. 1 EC₅₀ values obtained from the cellular binding assay of TM-mu33A1-scFv and TM-hu33A1-scFv01 to TM-hu33A1-scFv05 on U251-MG cells.**

| | **EC₅₀ (M)** | | **Relative Affinity to mu33A1 (%)** | |
|---|---|---|---|---|
| **TM** | **Mean** | **SD** | **Mean** | **SD** |
| **mu33A1** | 2.5E-09 | 1.4E-09 | 100.0 | 0.0 |
| **hu33A1_V01** | 3.6E-09 | 1.5E-10 | 68.3 | 35.2 |
| **hu33A1_V02** | 3.2E-09 | 6.4E-10 | 72.8 | 28.3 |
| **hu33A1_V03** | 2.2E-09 | 5.9E-10 | 102.4 | 33.7 |
| **hu33A1_V04** | 1.0E-09 | 2.3E-10 | 289.8 | 199.0 |
| **hu33A1_V05** | 1.1E-09 | 3.0E-10 | 277.5 | 196.9 |

The results of the cellular binding assay of a targeting module binding to HER2 (TM-hu4D5) are shown in **Fig. 11****.** The cellular binding of the HER2-TM was tested on a K562 cell line with recombinant overexpression of HER2 (K562-HER2) (**Fig. 11A**) and on U251-MG cell line (**Fig. 11B**). The procedure was the same as described above. The data were fitted using a four-parameter model with a variable slope for sigmoidal curves. The EC₅₀ value obtained from this model can be interpreted as a representative value of the TM affinity for the cells overexpressing the target receptor. TM-hu4D5 binding to K562-HER2 cells yielded an EC50 value in a range between 1.3 nM and 1.7 nM, while the binding to U251-MG cells produced an EC50 value of 8.9 nM.

### Surface Plasmon Resonance Measurements:

The functionality of the IL13Rα2- and/or HER2-binding TMs can be further confirmed in binding assays to soluble recombinant IL13Rα2 (**Tab. 2** and **3**) or soluble recombinant HER2 (**Tab. 4**) using surface plasmon resonance as well as in cell-based cytotoxicity assays.

IL13-based TM interaction with soluble recombinant IL13Rα2 ectodomain yielded a dissociation constant at equilibrium (K_{D}) in a range between 14 nM und 30 nM, while the scFv-based TMs K_{D} ranged between 0.5 nM und 2.56 nM. TM-hu4D binding to soluble recombinant HER2 ectodomain produced a K_{D} of 0.75 nM.

**Tab. 2** Binding kinetics parameters of TMs comprising IL13wt, IL13-E11Y-G30Y and IL13-E11Y-M32A to soluble recombinant IL13Rα2. Data obtained by surface plasmon resonance were fitted with a "1:1 binding" model using the Biacore X100 Evaluation Software (V2.0.1). The following parameters were calculated from this model: association rate (kₐ), dissociation rate (k_{d}) and the dissociation constant at equilibrium (K_{D}).

| **Binding Kinetics Parameters** | **IL13wt** | **IL13-E11Y-G30Y** | **IL13-E11Y-M32A** |
|---|---|---|---|
| **kₐ (1/Ms)** | 5.4 x 10⁴ | 2.6 x 10⁴ | 3.5 x 10⁴ |
| **k_{d} (1/s)** | 7.4 x 10⁻⁴ | 4.9 x 10⁻⁴ | 7.3 x 10⁻⁴ |
| **K_{D} (M)** | 1.4 x 10⁻⁸ | 3.0 x 10⁻⁸ | 2.1 x 10⁻⁸ |

**Tab. 3** Binding kinetics parameters of scFv based on mu33A1 and different versions of hu33A1 (hu33A1-scFv01 to hu33A1-scFv05) to soluble recombinant IL13Rα2. Data obtained by surface plasmon resonance were fitted with a "two-state reaction" model using the Biacore X100 Evaluation Software (V2.0.1). The following parameters were calculated from this model: association rate (kₐ), dissociation rate (k_{d}) and the dissociation constant at equilibrium (K_{D}).

| **Binding Kinetics Parameters** | **mu33A1** | **hu33A1-scFv01** | **hu33A1-scFv02** | **hu33A1-scFv03** | **hu33A1-scFv04** | **hu33A1-scFv05** |
|---|---|---|---|---|---|---|
| **kₐ1 (1/Ms)** | 8.71 x 10⁵ | 8.05 x 10⁵ | 8.97 x 10⁵ | 1.32 x 10⁶ | 1.47 x 10⁶ | 1.14 x 10⁶ |
| **kₐ2 (1/Ms)** | 7.08 x 10⁻⁴ | 8.64 x 10⁻⁴ | 1.14 x 10⁻³ | 9.11 x 10⁻⁴ | 5.79 x 10⁻⁴ | 8.46 x 10⁻⁴ |
| **K_{d}1 (1/s)** | 2.40 x 10⁻³ | 5.20 x 10⁻³ | 4.33 x 10⁻³ | 4.26 x 10⁻³ | 1.45 x 10⁻³ | 3.51 x 10⁻³ |
| **K_{d}2 (1/s)** | 7.21 x 10⁻⁴ | 5.69 x 10⁻⁴ | 8.29 x 10⁻⁴ | 4.52 x 10⁻⁴ | 5.41 x 10⁻⁴ | 4.62 x 10⁻⁴ |
| **K_{D} (M)** | 1.39 x 10⁻¹ | 2.56 x 10⁻⁹ | 2.03 x 10⁻⁹ | 1.08 x 10⁻¹ | 4.77 x 10⁻¹⁰ | 1.09 x 10⁻⁹ |

**Tab. 4** Binding kinetics parameters of TM-4D5 to soluble recombinant HER2. Data obtained by surface plasmon resonance were fitted with a "1:1 binding" model using the Biacore X100 Evaluation Software (V2.0.1). The following parameters were calculated from this model: association rate (ka), dissociation rate (kd) and the dissociation constant at equilibrium (KD)

| **Binding Kinetics Parameters** | **TM_hu4D5** |
|---|---|
| **kₐ (1/Ms)** | 4.2 x 10⁵ |
| **k_{d} (1/s)** | 3.2 x 10⁻⁴ |
| **K_{D} (M)** | 7.5 x 10⁻¹⁰ |

### Cytotoxicity assay:

The potency of IL13-based TMs targeting IL13Rα2 to induce a tumor cell elimination by switchable CAR-T cells was tested on a high-content microscopy-based cytotoxicity assay with U251-MG cell line (**Fig. 3A**), PC3 cells (**Fig. 3B**), recombinant K562-IL13Rα2 cell line (**Fig. 3C**) and recombinant K562-IL13Rα1 cell line (**Fig. 3D**). TMs potency was assessed on a high-content microscopy-based cytotoxicity assay. Switchable CAR-T cells were incubated with the target cells at a E:T ratio of 2:1 in the presence of various TM concentrations for 48 h in the case of the flow cytometry-based assay and for 72 h in the case of the microscopy-based assay. Target cells were quantified and lysis was calculated normalizing the cell count of each sample to a control sample where only tumor cells were plated. Data were fitted with a four-parameter model with a variable slope for sigmoidal curves. The calculated EC₅₀ value can be interpreted as a representative value for the TM potency against these tumor cells.

IL13-based TMs presented the potential to induce the lysis of U251-MG by switchable CAR T cells with an EC50 ranging between 3.11 pM and 28.3 pM. The EC50 values of these TMs in the cytotoxicity assay with PC3 cells ranged between 2.29 pM and 15.8 pM, while the EC50 value range with K562-IL13Rα2 comprised values between 19.5 pM and 134.0 pM. The TM based on IL13wt induced the switchable CAR T cell-dependent lysis of K562-IL13Rα1 with a EC50 of 516 pM and maximal lysis of 85% at saturation levels, while the maximal lysis with TMs based on IL13 muteins only reached 20% (TM-IL13-E11Y-M32A) and 40% (TM-IL13-E11Y-G30Y) at the highest concentration tested in this assay (100 nM). No lysis saturation of K562-IL13Ra1 was reached with TMs based on IL13 muteins, which confirmed the success of the introduced amino acid changes in avoiding cross-reactivity with IL13Rα1.

The stability of targeting module was tested in mouse serum (**Fig. 4A**) or human serum (**Fig. 4B**) at 37°C and assessed via functional potency assay. TM was diluted in PBS for the reference sample and in mouse or human serum for the test samples at a concentration of 0.01 mg/ml. Reference sample was kept at 4°C for 48 h and test sample was kept at 37°C for either 24 h or 48 h. TMs potency was assessed on a high-content microscopy-based cytotoxicity assay. Switchable CAR-T cells were incubated with U251-MG cells as target cells in the presence of various TM concentrations for 48 h. Target cells were quantified and lysis was calculated normalizing the cell count of each sample to a control sample where only tumor cells were plated. The dashed line shows the lysis of a control without TM (switchable CAR-T cell and U251-MG cells). Data were fitted with a four-parameter model with a variable slope for sigmoidal curves. The calculated EC₅₀ value can be interpreted as a representative value for the TM potency against these tumor cells. TM-IL13-E11Y-M32A potency in mouse serum at 37°C for 24 h decreased as indicated by the increment of the EC₅₀ value from 14.3 pM to 25.8 pM and further to 32.2 pM after 48 h. IL13-based TMs showed high stability in human serum at 37°C with a decay in potency of only 3.2% to 8.8% after 24h and 32.4% to 43.3% after 48 h.

Furthermore, the cytotoxicity of the Ab-based targeting modules, in particular an IL13Rα2-binding antibody fragment with CDRs according to SEQ ID No. 11 to 16 (TM-mu33A1-scFv (**Fig. 8A-C**) and TM-hu33A1-scFv1 to TM-hu33A1-scFv5 (**Tab. 5** and **Fig. 8C**)), was tested on K562wt, recombinant K562-IL13Rα2 and K562-IL13Rα1 cell line (**Fig. 8A**), PC3 cell line (**Fig. 8B**) and U251-MG cell line (**Tab. 5** and **Fig. 8C**).

The TM based on the parental murine clone 33A1 yielded an EC₅₀ value of 6.8 pM for K562-IL13Rα2 cells and 48.0 pM for PC3 cells. No specific cytotoxicity was found titrating this TM on K562-IL13Rα1 in combination with switchable CAR T cells, confirming the absence of undesired cross-reactivity of this clone family for IL13Rα1, which is expressed on normal tissues. TM-mu33A1-scFv, as well as TM-hu33A1-scFv01 to TM-hu33A1-scFv05, showed potency to induce the switchable CAR T cells-dependent lysis of the glioblastoma cell line U251-MG, yielding EC₅₀ values in a range of 23 pM and 59 pM. TM-hu33A1-scFv04 showed the highest potency to induce the lysis of U251-MG cells with an EC₅₀ value of 23 pM.

**Tab. 5** EC₅₀ values obtained from the cytotoxicity assay of TM-mu33A1-scFv and TM-hu33A1-scFv1 to TM-hu33A1-scFv5 on U251-MG cells. Relative potency of TMs based on different humanized versions of mu33A1-scFv (TM-hu33A1v01 to TM-hu33A1v05) to induce the lysis of U251-MG cell line by UniCAR T cells. EC50 values of each humanized version were relativized to the EC₅₀ value of the parental TM (TM-mu33A1-scFv).

| | **EC₅₀ (M)** | | **Relative Potency to mu33A1 (%)** | |
|---|---|---|---|---|
| **TM** | **Mean** | **SD** | **Mean** | **SD** |
| **mu33A1** | 2.8E-11 | 8.0E-12 | 100.0 | 0.0 |
| **hu33A1_V01** | 5.8E-11 | 1.5E-11 | 50.4 | 15.2 |
| **hu33A1_V02** | 4.5E-11 | 2.4E-11 | 87.4 | 45.3 |
| **hu33A1_V03** | 5.3E-11 | 2.6E-12 | 52.5 | 12.7 |
| **hu33A1_V04** | 2.3E-11 | 1.1E-11 | 153.3 | 64.1 |

| | | | | |
|---|---|---|---|---|
| **hu33A1_V05** | 5.9E-11 | 1.4E-11 | 49.1 | 13.0 |

Additionally, the stability of the Ab-based targeting modules (TM-mu33A1-scfv and TM-hu33A1-scFv1 to TM-hu33A1-scFv5) was assessed via functional potency assay. Switchable CAR-T cells were incubated with U251-MG cells as target cells in the presence of various TM concentrations for 48 h at an E:T ratio of 2:1. Target cells were quantified and lysis was calculated normalizing the cell count of each sample to a control sample where only tumor cells were plated. Data were fitted with a four-parameter model with a variable slope for sigmoidal curves. The calculated EC₅₀ value of each sample was relativized to the EC₅₀ obtained with the corresponding reference. For the stability study at 25°C, the TMs were incubated in a phosphate-buffered saline for seven days and the reference was kept at 4°C (**Tab. 6** and **Fig. 9A**). The stability of TMs in human serum was performed at 37°C for 24 h or 48 h and the reference was stored in a phosphate-buffered saline at 4°C (**Tab. 7** and **Fig. 9B**).

The relative potency of scFv-based TM stored in a phosphate-saline buffer at 25°C for seven days ranged between 47.8% and 78.5%. The potency of the TM based on the parental murine clone 33A1 decreased by 60% after 24 h incubation in human serum at 37°C and further by 74.2% after 48 h. The TMs based on different humanized versions of the clone 33A1 presented higher stability in human serum at 37°C with relative potencies of 69.7% to 102.9% after 24 h and 60.0% to 78.5% after 48 h. TM-hu33A1-scFv04 showed the highest stability in both stabilities studies with a relative potency of 78.5% after storage in a phosphate-saline at 25°C for seven days and also 78.5% after incubation in human serum at 37°C for 48 h.

**Tab. 6** EC₅₀ values obtained from the cytotoxicity assay of TM-mu33A1-scFv and TM-hu33A1-scFv1 to TM-hu33A1-scFv5 on U251-MG cells with samples freshly thawed as reference and samples that were incubated in a saline buffer for 7 days at 25°C. Relative potency of TMs based on different humanized versions of mu33A1-scFv (TM-hu33A1v01 to TM-hu33A1v05) to induce the lysis of U251-MG cell line by UniCAR T cells. EC₅₀ values of samples incubated at 25°C for 7 days were relativized to the EC₅₀ value of the reference sample that was directly thawed for the assay setup.

| | **EC₅₀ (M)** | | |
|---|---|---|---|
| **TM** | **Reference (Freshly thawed)** | **7 days at 25°C** | **Relative Potency** |
| **mu33A1** | 2.51E-11 | 3.58E-11 | 70.1 |
| **hu33A1_V01** | 5.73E-11 | 1.20E-10 | 47.8 |
| **hu33A1_V02** | 4.95E-11 | 7.10E-11 | 69.7 |
| **hu33A1_V03** | 5.67E-11 | 1.01E-10 | 56.1 |
| **hu33A1_V04** | 2.26E-11 | 2.88E-11 | 78.5 |
| **hu33A1_V05** | 6.20E-11 | 9.10E-11 | 68.1 |

**Tab. 7** EC₅₀ values obtained from the cytotoxicity assay of TM-mu33A1-scFv and TM-hu33A1-scFv1 to TM-hu33A1-scFv5 on U251-MG cells with samples incubated in a phosphate-buffered saline at 4°C as reference and samples that were incubated in human serum at 37°C for 24 h and 48 h. Relative potency of TMs based on different humanized versions of mu33A1-scFv (TM-hu33A1v01 to TM-hu33A1v05) to induce the lysis of U251-MG cell line by UniCAR T cells. EC₅₀ values of samples incubated at 25°C for 7 days were relativized to the EC₅₀ value of the reference sample.

| | **EC₅₀** | | | **Relative Potency** | | |
|---|---|---|---|---|---|---|
| **TM** | **Reference (4°C in PBS)** | **24 h in serum (37°C)** | **48 h in serum (37°C)** | **Reference (4°C in PBS)** | **24 h in serum (37°C)** | **48 h in serum (37°C)** |
| **mu33A1** | 2.3E-11 | 5.9E-11 | 9.1E-11 | 100 | 40.1 | 25.8 |
| **hu33A1_V01** | 4.5E-11 | 7.5E-11 | 5.7E-11 | 100.0 | 78.1 | 60.0 |
| **hu33A1_V02** | 4.1E-11 | 4.5E-11 | 5.3E-11 | 100.0 | 91.4 | 78.1 |
| **hu33A1_V03** | 3.9E-11 | 4.1E-11 | 5.4E-11 | 100.0 | 97.4 | 73.1 |
| **hu33A1_V04** | 1.5E-11 | 1.9E-11 | 1.5E-11 | 100.0 | 102.9 | 78.5 |
| **hu33A1_V05** | 3.1E-11 | 4.5E-11 | 4.7E-11 | 100.0 | 69.7 | 66.4 |

The cytotoxicity of HER2-binding TMs, in particular TM-hu4D5, was tested on recombinant K562 target cells (**Fig. 12A**), the glioblastoma cell line U251 (**Fig. 12B**) and on prostate cancer PC3 cell line (**Fig. 12C**). TMs potency was assessed on a flow-cytometry-based cytotoxicity assay (**Fig. 12A**) or on a high-content microscopy-based cytotoxicity assay (**Fig. 12** **B** and **C**). Switchable CART cells were incubated with K562 cells with recombinant overexpression of HER2, U251-MG cells or PC3 cells as target cells in the presence of various TM concentrations for 48 h at an E:T ratio of 2:1 (**Fig. 12A** and **B**) or 1:1 (**Fig. 12C**), respectively. Target cells were quantified and lysis was calculated normalizing the cell count of each sample to a control sample where only tumor cells were plated. Data were fitted with a four-parameter model with a variable slope for sigmoidal curves. The calculated EC50 value can be interpreted as a representative value for the TM potency against these tumor cells. Additionally, the stability of TM-hu4D5 in human serum at 37°C for 24 h and 48 h was assessed via a similar cytotoxicity assay. The EC₅₀ value of the TM samples were relativized to the reference sample that was stored in a phosphate-buffered saline at 4°C (**Fig. 12D**).

TM-hu4D5 potency to induce the lysis of HER2-expressing tumor cells was represented by the EC₅₀ values obtained in the cytotoxicity assays with K562-HER2 (1.0 pM), with PC3 cells (1.04 nM) and with U251-MG cells (1.14 nM). The stability of this TM in human serum at 37°C was also confirmed with an increment in the EC₅₀ from 0.78 nM to 0.86 nM in 24 h and further to 1.18 nM after 48 h.

### Pharmacokinetic studies:

For the pharmacokinetic studies of TMs in NSG mice, this molecule was administered as a single intraperitoneal (i.p) or intravenous (i.v.) bolus of 250 ng TM/g mouse. TM concentration in blood plasma samples taken at different time points after TM administration was determined by a sandwich ELISA. In **Fig. 5****,** representative data of five to six mice per time point analyzed is shown. Indicated pharmacokinetic parameters were defined utilizing PK solver2.0 (Zhang *et al.* 2010) applying a non-compartmental analysis. The slope of the terminal phase was calculated from the last three or six time points (t_{1/2}). The concentration decay of TM-IL13-E11Y-M32A after i.p. administration is shown in **Fig. 5A** and after i.v. administration is shown in **Fig. 5B****.** The concentration decay after i.v. administration of TM-33A1-scFv04 is shown in **Fig. 5C** and after i.v. administration of TM-hu4D5 is shown in **Fig. 5D****. Tab. 8** shows the calculated pharmacokinetic (PK) parameters. The short terminal half-life of these TMs (0.81 h to 1.52 h) presents the potential for a tight control of the immune response induced by the switchable CAR T cells.

**Tab. 8** Pharmacokinetics parameters of a targeting module comprising IL13-E11Y-M32A after intravenous and intraperitoneal administration in NSG mice. The following parameters were calculated using the non-compartmental analysis of PK Solver (v2.0): K (slope of the terminal phase), t_{1/2} (terminal plasma half-life), Tmax (time at which the maximal concentration in plasma was observed), Cmax (observed maximal concentration), C₀ (extrapolated initial concentration), AUC₀₋ₜ (area under the curve from beginning to last observed time point), AUC_{0-∞} (area under the curve extrapolated from beginning to infinite), Cl (clearance), V_{c} (initial central volume of distribution), Vₐᵣₑₐ (large volume of distribution), and F (bioavailability).

| | ***intraperitonal (i.p.)*** | ***intravenous (i.v.)*** | | |
|---|---|---|---|---|
| **Pharmacokinetics parameters** | **TM-IL13-E11Y-M32A** | | **TM-33A1-scFv04** | **TM-hu4D5** |
| **k [1/h]** | 0.47 | 0.46 | 0.85 | 0.68 |
| **t_{1/2} [h]** | 1.46 | 1.52 | 0.81 | 1.00 |
| **Tₘₐₓ [h]** | 1 | 0.08 | 0.08 | 0.08 |
| **Cₘₐₓ [ng/ml]** | 137.60 | 589.35 | 1909.25 | 1794.99 |
| **C₀ [ng/ml]** | - | 615.38 | 3675.54 | 5027.27 |
| **AUC₀₋ₜ** [ng/ml*h] | 480.14 | 859.11 | 773.80 | 579.98 |
| **AUC_{0-∞} [ng/ml*h]** | 501.48 | 878.90 | 786.03 | 587.79 |
| **Cl [ml/h/kg]** | 284.45 | 284.45 | 318.05 | 425.32 |
| **Vc [ml/kg]** | - | 406.25 | 68.01 | 49.72 |
| **Varea [ml/kg]** | 1050.78 | 624.83 | 373.64 | 618.83 |
| **F** | 0.57 | 1.00 | 1.00 | 1.00 |

### Therapeutic proof of concept in vivo

Tumor size progression in NSG mice transplanted with U251-MG and treated with switchable CAR-T cells and the IL13-based TM TM-IL13-E11Y-M32A is shown in **Fig. 6****.** 2x10⁶ U251-IL13Rα2 cells were injected subcutaneously on the right flank of NSG mice. Two weeks after tumor transplant a single dose of 2x10⁶ switchable CAR-T cells was administered intravenously. TM therapy began directly after T cell transplant and was divided into two cycles (**Fig. 6A****,** dotted window in the graph) of two weeks each, excluding weekends. One therapy group was treated with TM administered intraperitoneally (IP) twice a day (1 µg/g mouse) and another group was treated with TM administered peritumorally (PT) once a day (30 µg/mouse). Each data point represents the mean value of six biological replicates (different mice) with its corresponding statistical error of the mean. **Fig. 6B** shows the statistical significance of tumor size differences after two therapeutic cycles (week 7) analyzed using a non-parametric unpaired Kruskal-Wallis test with Dunnett multiple comparison test (p < 0.05 = *, p < 0.01 = **, p < 0.005 = ***).

Furthermore, the tumor size progression in NSG mice transplanted with U251-MG treated with switchable CAR-T cells and the mu33A1-based TM is shown in **Fig. 10****.** The test was carried out as described above, except that there was only one therapy group, which was treated with TM administered intraperitoneally (IP) twice a day (1 µg/g mouse).

The therapy with intraperitoneal administration of TM-mu33A1-scFv or TM-IL13-E11Y-M32A achieved an anti-tumor response after two therapy cycles in comparison with the control group that was transplanted with only U251 cells. A statistically significant tumor size reduction in comparison with the control group that received switchable CAR-T cell treatment without TM therapy was achieved when the TM was administered peritumorally.

### Cited non-patent literature

Brown CE, Aguilar B, Starr R, Yang X, Chang WC, Weng L, Chang B, Sarkissian A, Brito A, Sanchez JF, Ostberg JR, D'Apuzzo M, Badie B, Barish ME, Forman SJ (2018) Optimization of IL13Rα2-Targeted Chimeric Antigen Receptor T Cells for Improved Anti-tumor Efficacy against Glioblastoma. Mol Ther. 26 (1), 31-44.
Brudno JN, Kochenderfer JN (2016) Toxicities of chimeric antigen receptor T cells: recognition and management. Blood 127, 3321-3330.
Cartellieri M, Bachmann M, Feldmann A, Bippes C, Stamova S, Wehner R, Temme A, Schmitz M (2010) Chimeric Antigen Receptor-Engineered T Cells for Immunotherapy of Cancer J. Biomed. Biotechnol. Article ID 956304, doi: 10.1155/2010/956304.
Cartellieri M, Feldmann A, Koristka S, Arndt C, Loff S, Ehninger A, von Bonin M, Bejestani EP, Ehninger G, Bachmann MP (2016) Switching CAR T cells on and off: a novel modular platform for retargeting of T cells to AML blasts. Blood cancer J. 6 (8), e458.
Fedorov VD, Themeli M, Sadelain M (2013) PD-1- and CTLA-4-based inhibitory chimeric antigen receptors (iCARs) divert off-target immunotherapy responses. Sci. Transl. Med. 5 (215), 215ra172.
Finney HM, Akbar AN, Lawson AD (2004) Activation of resting human primary T cells with chimeric receptors: costimulation from CD28, inducible costimulator, CD134, and CD137 in series with signals from the TCR zeta chain. J. Immunol. 172 (1), 104-113.
Frigault MJ, Lee J, Basil MC, Carpenito C, Motohashi S, Scholler J, Kawalekar OU, Guedan S, McGettigan SE, Posey AD Jr, Ang S, Cooper LJ, Platt JM, Johnson FB, Paulos CM, Zhao Y, Kalos M, Milone MC, June CH (2015) Identification of chimeric antigen receptors that mediate constitutive or inducible proliferation of T cells. Cancer Immunol. Res. 3 (4), 356-367.
Gade TP, Hassen W, Santos E, Gunset G, Saudemont A, Gong MC, Brentjens R, Zhong XS, Stephan M, Stefanski J, Lyddane C, Osborne JR, Buchanan IM, Hall SJ, Heston WD, Rivière I, Larson SM, Koutcher JA, Sadelain M (2005) Targeted elimination of prostate cancer by genetically directed human T lymphocytes. Cancer Res. 65 (19), 9080-9088.
Gong MC, Latouche JB, Krause A, Heston WDW, Bander NH, Sadelain M (1999) Cancer patient T cells genetically targeted to prostate-specific membrane antigen specifically lyse prostate cancer cells and release cytokines in response to prostate-specific membrane antigen. Neoplasia. 1 (2), 123-127.
Guedan S, Posey AD, Shaw C, Wing A, Da T, Patel PR, McGettigan SE, Casado-Medrano V, Kawalekar OU, Uribe-Herranz M, Song D, Melenhorst JJ, Lacey SF, Scholler J, Keith B, Young RM, June CH (2018) Enhancing CAR T cell persistence through ICOS and 4-1BB costimulation. JCI Insight. 3(1), 96976.
Guedan S, Madar A, Casado-Medrano V, Shaw CE, Wing A, Liu F, Young RM, June CH, Posey AD (2020) Single residue in CD28-costimulated CAR T cells limits long-term persistence and antitumor durability. J Clin Invest. 133215.
Hombach AA, Abken H (2011) Costimulation by chimeric antigen receptors revisited the T cell antitumor response benefits from combined CD28-OX40 signalling. Int. J. Cancer. 129 (12), 2935-2944.
Hombach A, Sent D, Schneider C, Heuser C, Koch D, Pohl C, Seliger B, Abken H (2001) T-cell activation by recombinant receptors: CD28 costimulation is required for interleukin 2 secretion and receptor-mediated T-cell proliferation but does not affect receptor-mediated target cell lysis. Cancer Res. 61 (5), 1976-1982.
Kagoya Y, Tanaka S, Guo T, Anczurowski M, Wang CH, Saso K, Butler MO, Minden MD, Hirano N (2018) A novel chimeric antigen receptor containing a JAK-STAT signaling domain mediates superior antitumor effects. Nat Med. 24(3), 352-359.
Kershaw MH, Westwood JA, Parker LL, Wang G, Eshhar Z, Mavroukakis SA, White DE, Wunderlich JR, Canevari S, Rogers-Freezer L, Chen CC, Yang JC, Rosenberg SA, Hwu P (2006) A phase I study on adoptive immunotherapy using gene-modified T cells for ovarian cancer. Clin. Cancer Res. 12 (20), 6106-6115.
Lamers CH, Sleijfer S, Willemsen RA, Debets R, Kruit WHJ, Gratama JW, Stoter G (2004) Adoptive immuno-gene therapy of cancer with single chain antibody [scFv(Ig)] gene modified T lymphocytes. J. Biol. Regul. Homeost. Agents. 18 (2), 134-140.
Milone MC, Fish JD, Carpenito C, Carroll RG, Binder GK, Teachey D, Samanta M, Lakhal M, Gloss B, Danet-Desnoyers G, Campana D, Riley JL, Grupp SA, June CH (2009) Chimeric receptors containing CD137 signal transduction domains mediate enhanced survival of T cells and increased antileukemic efficacy in vivo. Mol. Ther. 17 (8), 1453-1464.
Morgan RA, Yang JC, Kitano M, Dudley ME, Laurencot CM, Rosenberg SA (2010) Case Report of a Serious Adverse Event Following the Administration of T Cells Transduced With a Chimeric Antigen Receptor Recognizing ERBB2. Mol. Ther. 18, 843-851.
Pinthus JH, Waks T, Kaufman-Francis K, Schindler DG, Harmelin A, Kanety H, Ramon J, Eshhar Z (2003) Immuno-gene therapy of established prostate tumors using chimeric receptor-redirected human lymphocytes. Cancer Res. 63 (10), 2470-2476.
Reinke AW, Grant RA, Keating AE (2010) A Synthetic Coiled-Coil Interactome Provides Heterospecific Modules for Molecular Engineering. JACS 132, 6025-6031.
Sotillo E, Barrett DM, Black K., Bagashev A, Oldridge D, Wu G, Sussman R, Lanauze C, Ruella M, Gazzara MR, Martinez NM, Harrington CT, Chung EY, Perazzelli J, Hofmann TJ, Maude SL, Raman P, Barrera A, Gill S, Lacey SF, Melenhorst JJ, Allman D, Jacoby E, Fry T, Mackall C, Barash Y, Lynch KW, Maris JM, Grupp SA, Thomas-Tikhonenko A (2015) Convergence of Acquired Mutations and Alternative Splicing of CD19 Enables Resistance to CART-19 Immunotherapy. Cancer Discov. 5, 1282-1295.
Titov A, Petukhov A, Staliarova A, Motorin D, Bulatov E, Shuvalov O, Soond SM, Piacentini M, Melino G, Zaritskey A, Barlev NA (2018) The biological basis and clinical symptoms of CAR-T therapy-associated toxicites. Cell Death Dis 9, 897.
Töpfer K, Cartellieri M, Michen S, Wiedemuth R, Müller N, Lindemann D, Bachmann M, Füssel M, Schackert G, Temme A (2015) DAP12-based activating chimeric antigen receptor for NK cell tumor immunotherapy. J. Immunol. 194 (7), 3201-3212.
Wang J, Jensen M, Lin Y, Sui X, Chen E, Lindgren CG, Till B, Raubitschek A, Forman SJ, Qian X, James S, Greenberg P, Riddell S, Press OW (2007) Optimizing adoptive polyclonal T cell immunotherapy of lymphomas, using a chimeric T cell receptor possessing CD28 and CD137 costimulatory domains. Hum. Gene Ther. 18 (8), 712-725.
Wikstrand CJ, Hale LP, Batra SK, Hill ML, Humphrey PA, Kurpad SN, McLendon RE, Moscatello D, Pegram CN, Reist CJ, et al. (1995) Monoclonal antibodies against EGFRvIII are tumor specific and react with breast and lung carcinomas and malignant gliomas. Cancer Res. 55(14), 3140-3148.
Yu S, Yi M, Qin S, Wu K (2019) Next generation chimeric antigen receptor T cells: safety strategies to overcome toxicity. Molecular Cancer. 18 (1).
Zhang T, Lemoi BA, Sentman CL (2005) Chimeric NK-receptor-bearing T cells mediate antitumor immunotherapy. Blood. 106 (5), 1544-1551.
Zhang Y, Huo M, Zhou J, Xie S (2010) PKSolver: An add-in program for pharmacokinetic and pharmacodynamic data analysis in Microsoft Excel. Computer Methods and Programs in Biomedicine 99 (3), 306-314.
Zhao Y, Wang QJ, Yang S, Kochenderfer JN, Zheng Z, Zhong X, Sadelain M, Eshhar Z, Rosenberg SA, Morgan RA (2009) A herceptin-based chimeric antigen receptor with modified signaling domains leads to enhanced survival of transduced T lymphocytes and antitumor activity. J. Immunol. 183 (9), 5563-5574.

### Reference signs

- **1**: first domain, a tag-binding domain or tag.
- **2**: second domain, an extracellular hinge and a transmembrane domain.
- **3**: third domain, a signal transduction domain.
- **4**: optional fourth domain, a short peptide linker.

## Claims

1. A targeting module comprising
i) at least one IL13Rα2-binding domain,
ii) a tag-binding domain or a tag,
for use in a method for stimulating a chimeric antigen receptor-mediated immune response in a mammal,
wherein the targeting module is administered in combination with a vector or a cell comprising a nucleotide sequence encoding a switchable chimeric antigen receptor, wherein the switchable chimeric antigen receptor comprises three domains,
wherein
- the first domain is a tag-binding domain or a tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

2. The targeting module for use in a method for stimulating a chimeric antigen receptor-mediated immune response in a mammal according to claim 1, wherein the IL13Rα2-binding domain comprises a human IL13 according to SEQ ID No. 1 or an IL13 mutein with a sequence identity of at least 95 % with SEQ ID No. 1, preferably human IL13 E11Y-G30Y according to SEQ ID No. 2 or human IL13 E11Y-M32A according to SEQ ID No. 3, or an antibody or antigen-binding fragment thereof, wherein the variable region of the light chain (V_{L}) comprises the amino acid complementarity determining region (CDR) sequences SEQ ID No. 11, SEQ ID No. 12 and SEQ ID No. 13, and the variable region of the heavy chain (V_{H}) comprises the amino acid sequences SEQ ID No. 14, SEQ ID No. 15 and SEQ ID No. 16.

3. The targeting module for use in a method for stimulating a chimeric antigen receptor-mediated immune response in a mammal according to claim 1 or 2, wherein the tag is a peptide epitope tag.

4. The targeting module for use in a method for stimulating a chimeric antigen receptor-mediated immune response in a mammal according to claim 3, wherein the peptide epitope tag is a myc-tag, a His-tag, a short linear peptide sequence from yeast transcription factor GCN4, a leucine zipper sequence or a short linear peptide sequence from a human nuclear protein, preferably from the human La protein.

5. The targeting module for use in a method for stimulating a chimeric antigen receptor-mediated immune response in a mammal according to one of the claims 1 to 4, wherein the variable regions of the at least one IL13RA2-binding domain and/or the effector cell-binding domain comprise a humanized amino acid sequence.

6. The targeting module for use in a method for stimulating a chimeric antigen receptor-mediated immune response in a mammal according to one of the claims 1 to 5, wherein the length is in the range of 20 to 1600 amino acids.

7. The targeting module for use in a method for stimulating a chimeric antigen receptor-mediated immune response in a mammal according to one of the claims 1 to 6 comprising SEQ ID No. 86 to SEQ ID No. 112.

8. The targeting module for use in a method for stimulating a chimeric antigen receptor-mediated immune response in a mammal according to one of the claims 1 to 7,
wherein the targeting module is administered in combination with a vector or a cell comprising a nucleotide sequence encoding a switchable chimeric antigen receptor and at least one further targeting module,
wherein the at least one further targeting module comprises at least one target cell-binding domain and a tag-binding domain or a tag,
wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269, CD273, CD274, CD276, CD279, CD319, CD366 and CD371, interleukin receptors, especially preferred IL-8Rα, IL-8Rβ, IL-11Rα, IL-11Pβ, IL13Rα1, CXCR4, c-Met, mesothelin, members of the epidermal growth factor receptor family and mutants thereof, especially preferred ErbB1, ErbB2, ErbB3, ErbB4 or mutants thereof, members of the tumor necrosis factor receptor superfamily, ephrins, ephrin receptors, especially preferred EphA1-10, EphA5 or EphB1-6; prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands, wherein the targeting module according to one of the claims 1 to6 and the at least one further targeting module comprise identical tag-binding domains or tags.

9. A nucleic acid, a vector or a cell comprising a nucleotide sequence encoding a targeting module according to one of the claims 1 to 7 for use in a method for stimulating a chimeric antigen receptor-mediated immune response in a mammal,
wherein the nucleic acid, vector or cell is administered in combination with a vector or a cell comprising a nucleotide sequence encoding a switchable chimeric antigen receptor, wherein the switchable chimeric antigen receptor comprises three domains,
wherein
- the first domain is a tag-binding domain or a tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

10. The nucleic acid, vector or cell for use in a method for stimulating a chimeric antigen receptor-mediated immune response in a mammal according to claim 9 further comprising a nucleotide sequence encoding a switchable chimeric antigen receptor,
wherein the switchable chimeric antigen receptor comprises three domains,
wherein
- the first domain is a tag-binding domain or tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

11. The nucleic acid, vector or cell for use in a method for stimulating a chimeric antigen receptor-mediated immune response in a mammal according to claim 9 or 10,
wherein the nucleic acid, vector or cell is administered in combination with a vector or a cell comprising a nucleotide sequence encoding a switchable chimeric antigen receptor and a nucleic acid, a vector or a cell comprising a nucleotide sequence encoding a further targeting module,
wherein the at least one further targeting module comprises at least one target cell-binding domain and a tag-binding domain or a tag,
wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269, CD273, CD274, CD276, CD279, CD319, CD366 and CD371, interleukin receptors, especially preferred IL-8Rα, IL-8Rβ, IL-11Rα, IL-11Pβ, IL13Rα1, CXCR4, c-Met, mesothelin, members of the epidermal growth factor receptor family and mutants thereof, especially preferred ErbB1, ErbB2, ErbB3, ErbB4 or mutants thereof, members of the tumor necrosis factor receptor superfamily, ephrins, ephrin receptors, especially preferred EphA1-10, EphA5 or EphB1-6; prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands, wherein the targeting module and the at least one further targeting module comprise identical tag-binding domains or tags.

12. A pharmaceutical composition comprising the targeting module according to one of the claims 1 to 7, the nucleic acid, vector or cell according to claim 9 and a pharmaceutically acceptable thinner or carrier for use in a method for stimulating a chimeric antigen receptor-mediated immune response in a mammal,
wherein the pharmaceutical composition is administered in combination with a vector or a cell comprising a nucleotide sequence encoding a switchable chimeric antigen receptor, wherein the switchable chimeric antigen receptor comprises three domains,
wherein
- the first domain is a tag-binding domain or a tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

13. The pharmaceutical composition for use in a method for stimulating a chimeric antigen receptor-mediated immune response in a mammal according to claim 12 comprising at least one further targeting module or at least one further nucleic acid, vector or cell encoding a further targeting module,
wherein the at least one further targeting module comprises at least one target cell-binding domain and a tag-binding domain or a tag,
wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269, CD273, CD274, CD276, CD279, CD319, CD366 and CD371, interleukin receptors, especially preferred IL-8Rα, IL-8Rβ, IL-11Rα, IL-11Pβ, IL13Rα1, CXCR4, c-Met, mesothelin, members of the epidermal growth factor receptor family and mutants thereof, especially preferred ErbB1, ErbB2, ErbB3, ErbB4 or mutants thereof, members of the tumor necrosis factor receptor superfamily, ephrins, ephrin receptors, especially preferred EphA1-10, EphA5 or EphB1-6; prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands, wherein the targeting module and the at least one further targeting module comprise identical tag-binding domains or tags.

14. A kit comprising
a) a targeting module according to one of the claims 1 to 7 or the nucleic acid, vector or cell according to claim 9 and
b) a vector or a cell comprising a nucleotide sequence encoding a switchable chimeric antigen receptor,
wherein the switchable chimeric antigen receptor comprises three domains, wherein
- the first domain is a tag-binding domain or tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

15. The kit according to claim 14, wherein the tag is a myc-tag, a His-tag, a short linear peptide sequence from yeast transcription factor GCN4, a leucine zipper sequence or a short linear peptide sequence from a human nuclear protein, preferably from the human La protein.

16. The kit according to claim 14 or 15, wherein the tag-binding domain is an antibody or an antibody fragment binding to a myc-tag, a His-tag, a short linear peptide sequence from yeast transcription factor GCN4, a leucine zipper sequence or a short linear peptide sequence from a human nuclear protein, preferably from a human La protein.

17. The kit according to one of the claims 14 to 16, wherein the extracellular hinge and transmembrane domain is selected from hinge and transmembrane domains of human CD28 molecule, CD8a chain NK cell receptors, preferably natural killer group NKG2D; or parts of the constant region of an antibody and combinations thereof.

18. The kit according to one of the claims 14 to 17, wherein the signal transduction domain is selected from cytoplasmic regions of CD28, CD137 (4-1BB), CD134 (OX40), CD278 (ICOS), DAP10 and CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor α), CD360 (interleukin-21 receptor), activating Fc receptors and mutants thereof.

19. The kit according to one of the claims 14 to 18 further comprising at least one further targeting module or at least one further nucleic acid, vector or cell encoding a further targeting module, wherein the at least one further targeting module comprises at least one target cell-binding domain and a tag-binding domain or a tag,
wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150 CD181, CD182, CD184, CD223, CD229, CD269, CD273, CD274, CD276, CD279, CD319, CD366 and CD371, interleukin receptors, especially preferred IL-8Rα, IL-8Rβ, IL-11Rα, IL-11Pβ, IL13Rα1, CXCR4, c-Met, mesothelin, members of the epidermal growth factor receptor family and mutants thereof, especially preferred ErbB1, ErbB2, ErbB3, ErbB4 or mutants thereof, members of the tumor necrosis factor receptor superfamily, ephrins, ephrin receptors, especially preferred EphA1-10, EphA5 or EphB1-6; prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, diasialogangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands, wherein the targeting module and the at least one further targeting module comprise identical tag-binding domains or tags.

20. The kit according to claim 19, wherein the at least one target cell-binding domain of the further targeting module is Trastuzumab or a fragment thereof.

21. The kit according to one of the claims 14 to 20, wherein the targeting module and/or the vector or cell comprising a nucleotide sequence encoding a switchable chimeric antigen receptor are in the form of a pharmaceutical composition.

22. The kit according to one of the claims 14 to 21 for use in a method for stimulating a chimeric antigen receptor-mediated immune response in a mammal, preferably for use in the treatment of cancer, infectious disease or autoimmune disease.

23. An IL13Rα2-binding IL13 mutein according to SEQ ID No. 2 or according to SEQ ID No. 3.

24. The mutein according to claim 23 for use in the treatment of cancer, infectious disease or autoimmune disease.

25. The mutein according to claim 23 for use in a method for stimulating a chimeric antigen receptor-mediated immune response in a mammal.

26. A nucleic acid, a vector or a cell comprising a nucleotide sequence encoding a mutein according to claim 23.

27. A pharmaceutical composition comprising a mutein according to claim 23 and a pharmaceutically acceptable thinner or carrier.

28. An IL13Rα2-binding antibody or antibody fragment, wherein the V_{L} comprises the amino acid CDR sequences SEQ ID No. 11, SEQ ID No. 12 and SEQ ID No. 13, and the V_{H} comprises the amino acid CDR sequences SEQ ID No. 14, SEQ ID No. 15 and SEQ ID No. 16.

29. The antibody or antibody fragment according to claim 28, wherein the variable regions of the antibody comprise a humanized amino acid sequence.

30. The antibody or antibody fragment according to claim 28 or 29 comprising a V_{L} according to SEQ ID No. 17, wherein X₁, X₂, X₄, X₁₀, X₁₃, X₁₄, X₁₈, X₁₉, X₂₁, X₂₂, X₂₄, X₄₁, X₆₀, X₇₃, X₇₇, X₇₈, X₈₀, X₈₃, X₈₄, X₈₅ and X₈₇ are independently from each other selected from a proteinogenic alpha-amino acid residue, and/or a V_{H} according to SEQ ID No. 18, wherein X₁₂, X₂₀, X₃₈, X₄₈, X₆₁, X₆₂, X₆₅, X₆₆, X₆₈, X₇₀, X₇₂, X₇₄, X₇₅, X₇₆, X₇₉, X₈₃, X₈₅, X₈₉, X₁₁₀ and X₁₁₄ are independently from each other selected from a proteinogenic alpha-amino acid residue.

31. The antibody or antibody fragment according to one of the claims 28 to 30 comprising one of the sequences according to SEQ ID No. 5 to SEQ ID No. 10.

32. The antibody or antibody fragment according to one of the claims 28 to 31 for use in the treatment of cancer, infectious disease or autoimmune disease.

33. The antibody or antibody fragment according to one of the claims 28 to 31 for use in a method for stimulating a chimeric antigen receptor-mediated immune response in a mammal.

34. A nucleic acid, a vector or a cell comprising a nucleotide sequence encoding an antibody or antibody fragment according to one of the claims 28 to 31.

35. A pharmaceutical composition comprising an antibody or antibody fragment according to one of the claims 28 to 31 and a pharmaceutically acceptable thinner or carrier.

36. A targeting module comprising
i) at least one HER2-binding domain,
ii) a tag-binding domain or a tag,
for use in a method for stimulating a chimeric antigen receptor-mediated immune response in a mammal,
wherein the targeting module is administered in combination with a vector or a cell comprising a nucleotide sequence encoding a switchable chimeric antigen receptor, wherein the switchable chimeric antigen receptor comprises three domains,
wherein
- the first domain is a tag-binding domain or a tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

37. The targeting module for use in a method for stimulating a chimeric antigen receptor-mediated immune response in a mammal according to claim 36, wherein the at least one HER2-binding domain is Trastuzumab, a Trastuzumab mutant or a fragment thereof, preferably comprising SEQ ID No. 37 and SEQ ID No. 38.

38. The targeting module for use in a method for stimulating a chimeric antigen receptor-mediated immune response in a mammal according to claim 36 or 37, wherein the tag is a peptide epitope tag.

39. The targeting module for use in a method for stimulating a chimeric antigen receptor-mediated immune response in a mammal according to claim 38, wherein the peptide epitope tag is a myc-tag, a His-tag, a short linear peptide sequence from yeast transcription factor GCN4, a leucine zipper sequence or a short linear peptide sequence from a human nuclear protein, preferably from the human La protein.

40. The targeting module for use in a method for stimulating a chimeric antigen receptor-mediated immune response in a mammal according to one of the claims 36 to 39, wherein the variable regions of the at least one HER2-binding domain and/or the effector cell-binding domain comprise a humanized amino acid sequence.

41. The targeting module for use in a method for stimulating a chimeric antigen receptor-mediated immune response in a mammal according to one of the claims 36 to 40, wherein the length is in the range of 20 to 1600 amino acids.

42. The targeting module for use in a method for stimulating a chimeric antigen receptor-mediated immune response in a mammal according to one of the claims 36 to 41 comprising SEQ ID No. 34, SEQ ID No. 35 orSEQ ID No. 36.

43. The targeting module for use in a method for stimulating a chimeric antigen receptor-mediated immune response in a mammal according to one of the claims 36 to 42,
wherein the targeting module is administered in combination with a vector or a cell comprising a nucleotide sequence encoding a switchable chimeric antigen receptor and at least one further targeting module,
wherein the at least one further targeting module comprises at least one target cell-binding domain and a tag-binding domain or a tag,
wherein the at least one target cell-binding domain is an antibody, antibody fragment, a protein, a peptide or a low molecular weight organic ligand that binds to surface antigens selected from the group comprising CD2, CD3, CD4, CD8, CD10, CD19, CD20, CD22, CD23, CD25, CD30, CD33, CD38, CD44, CD44v6 CD52, CD90, CD99, CD123, CD133, CD150
CD181, CD182, CD184, CD223, CD229, CD269, CD273, CD274, CD276, CD279, CD319, CD366 and CD371, interleukin receptors, especially preferred IL-8Rα, IL-8Rβ, IL-11Rα, IL-11Pβ, IL13Rα1 and IL13Rα2, CXCR4, c-Met, mesothelin, members of the epidermal growth factor receptor family and mutants thereof, especially preferred ErbB1, ErbB3, ErbB4 or mutants thereof, members of the tumor necrosis factor receptor superfamily, ephrins, ephrin receptors, especially preferred EphA1-10, EphA5 or EphB1-6; prostate specific antigens, preferably PSCA and PSMA; embryonic antigens, preferably CEA and fetal acethylcholine receptor; members of the vascular endothelia growth factor family, EpCAM, AFP, members of the intercellular adhesion molecule family, members of the mucin protein family, FSHR, HMW-MAA, FBP, folate receptors, somatostatin receptors, ligands of the NKG2D receptor, cytokine receptors, members of the epithelia glycoprotein family, diasiabgangliosides, glypicans, G protein-coupled receptors, members of the carbonic anhydrase family, members of the carbohydrate antigen family, Notch ligands, MCSP, glycoprotein A33, guanylate cyclase 2C and tumor-specific glycans, including mutants and analogues of the named antibodies, antibody fragments, proteins, peptides or low molecular weight organic ligands, wherein the targeting module according to one of the claims 36 to 42 and the at least one further targeting module comprise different target cell-binding domains, and identical tag-binding domains or tags.

44. A nucleic acid, a vector or a cell comprising a nucleotide sequence encoding a targeting module according to one of the claims 36 to 42 for use in a method for stimulating a chimeric antigen receptor-mediated immune response in a mammal,
wherein the nucleic acid, vector or cell is administered in combination with a vector or a cell comprising a nucleotide sequence encoding a switchable chimeric antigen receptor,
wherein the switchable chimeric antigen receptor comprises three domains,
wherein
- the first domain is a tag-binding domain or a tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

45. The nucleic acid, vector or cell for use in a method for stimulating a chimeric antigen receptor-mediated immune response in a mammal according to claim 44 further comprising a nucleotide sequence encoding a switchable chimeric antigen receptor,
wherein the switchable chimeric antigen receptor comprises three domains,
wherein
- the first domain is a tag-binding domain or tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

46. A pharmaceutical composition comprising the targeting module according to one of the claims 36 to 42 or the nucleic acid, vector or cell according to claim 44 and a pharmaceutically acceptable thinner or carrier for use in a method for stimulating a chimeric antigen receptor-mediated immune response in a mammal,
wherein the pharmaceutical composition is administered in combination with a vector or a cell comprising a nucleotide sequence encoding a switchable chimeric antigen receptor,
wherein the switchable chimeric antigen receptor comprises three domains,
wherein
- the first domain is a tag-binding domain or a tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

47. A kit comprising
a) a targeting module according to one of the claims 36 to 42, the nucleic acid, vector or cell according to claim 44 and
b) a vector or a cell comprising a nucleotide sequence encoding a switchable chimeric antigen receptor,
wherein the switchable chimeric antigen receptor comprises three domains, wherein
- the first domain is a tag-binding domain or tag,
- the second domain is an extracellular hinge and a transmembrane domain and
- the third domain is a signal transduction domain,
wherein the tag-binding domain of the targeting module binds to the tag of the switchable chimeric antigen receptor or the tag of the targeting module binds to the tag-binding domain of the switchable chimeric antigen receptor.

48. The kit according to claim 47, wherein the tag is a myc-tag, a His-tag, a short linear peptide sequence from yeast transcription factor GCN4, a leucine zipper sequence or a short linear peptide sequence from a human nuclear protein, preferably from the human La protein.

49. The kit according to claim 47 or 48, wherein the tag-binding domain is an antibody or an antibody fragment binding to a myc-tag, a His-tag, a short linear peptide sequence from yeast transcription factor GCN4, a leucine zipper sequence or a short linear peptide sequence from a human nuclear protein, preferably from a human La protein.

50. The kit according to one of the claims 47 to 49, wherein the extracellular hinge and transmembrane domain is selected from hinge and transmembrane domains of human CD28 molecule, CD8a chain NK cell receptors, preferably natural killer group NKG2D; or parts of the constant region of an antibody and combinations thereof.

51. The kit according to one of the claims 47 to 50, wherein the signal transduction domain is selected from cytoplasmic regions of CD28, CD137 (4-1BB), CD134 (OX40), CD278 (ICOS), DAP10 and CD27, programmed cell death-1 (PD-1), cytotoxic T-lymphocyte antigen 4 (CTLA-4), cytoplasmic regions of CD3 chains, DAP12, CD122 (interleukin-2 receptor β), CD132 (interleukin-2 receptor γ), CD127 (interleukin-7 receptor α), CD360 (interleukin-21 receptor), activating Fc receptors and mutants thereof.

52. The kit according to one of the claims 47 to 51, wherein the targeting module and/or the vector or cell comprising a nucleotide sequence encoding a switchable chimeric antigen receptor are in the form of a pharmaceutical composition.

53. The kit according to one of the claims 47 to 52 for use in a method for stimulating a chimeric antigen receptor-mediated immune response in a mammal, preferably for use in the treatment of cancer, infectious disease or autoimmune disease.
